# EUROPEAN PATENT SPECIFICATION

(11) **EP 3 059 288 B1**
(45) Date of publication and mention of the grant of the patent: **22.12.2021**
(21) Application number: 14854518.9
(22) Date of filing: 17.10.2014
(51) Int. Cl.: C08L 101/00, C08K 5/55

(54) **RESIN COMPOSITION AND MOLDED ARTICLE**
HARZZUSAMMENSETZUNG UND FORMARTIKEL
COMPOSITION DE RÉSINE ET ARTICLE MOULÉ

(30) Priority: 17.10.2013 JP 2013216785; 26.12.2013 JP 2013270447; 19.05.2014 JP 2014103584
(43) Date of publication of application: 24.08.2016
(73) Proprietor: DIC Corporation, Tokyo 174-8520 (JP); National University Corporation Kochi University, Kochi-shi, Kochi 780-8520 (JP)
(72) Inventor: SAKURAI Naoto, Sakura-shi Chiba 285-8668 (JP); IKEDA Takeo, Sakura-shi Chiba 285-8668 (JP); SAKURAI Yoshinobu, Sakura-shi Chiba 285-8668 (JP); WATANABE Yasuyuki, Sakura-shi Chiba 285-8668 (JP); SATO Takayuki, Kochi-shi Kochi 780-8520 (JP)
(74) Representative: TBK
(86) International application number: PCT/JP2014/077696
(87) International publication number: WO 2015/056779

(56) References cited:
- WO-A1-2007/126052
- JP-A- H08 509 471
- JP-A- 2009 031 713
- JP-A- 2009 263 614
- JP-A- 2010 090 313
- JP-A- 2011 241 160
- JP-A- 2012 115 535
- US-A- 5 433 896
- US-A- 6 005 113
- US-A1- 2011 012 075
- GEORG M. FISCHER ET AL.: 'Near-Infrared Dyes and Fluorophores Based on Diketopyrrolopyrroles' ANGEWANTE CHEMIE INTERNATIONAL EDITION vol. 46, no. ISSUE, pages 3750 - 3753, XP055002052

## Description

### Technical Field

The present invention relates to a resin composition which emits near infrared fluorescent and a molded article obtained by processing the resin composition.

### Background Art

A near infrared fluorescent material has been used in various industrial applications mainly requiring product identification and anti-counterfeiting, and in recent years, has been used in medical applications such as a living body imaging probe or a test agent. As features of the near infrared wavelength regions, it is known that light in the near infrared wavelength region cannot be observed with the naked eye of a human, the influence thereof on a living body is small, and the transparency thereof with respect to the skin or the like is high. Such features can be utilized by incorporating a near infrared fluorescent material in a medical tool itself. For example, a system where a near infrared fluorescent material is incorporated in a medical tool such as a shunt tube and the position of the medical tool embedded into a living body is confirmed by irradiating with near infrared light from the outside of the living body is disclosed (for example, refer to PTL 1).

To visualize a medical implant embedded subcutaneously or the like, excitation in the near infrared light having high skin transparency is required, and the fluorescence emitted from the medical implant is also required to be in a near infrared region having high skin transparency. That is, typically, to ensure the visibility, the near infrared fluorescent material itself contained in the medical implant should strongly absorb light in the near infrared region, and, in addition, is required to emit strong fluorescence. Therefore, as the near infrared fluorescent material contained in the resin composition which is a raw material of a medical implant, it is preferable that the maximum absorption wavelength in the resin is in the near infrared region.

The near infrared fluorescent material includes an inorganic fluorescent material and an organic fluorescent material. Generally, the inorganic near infrared fluorescent material has an advantage that the light emit wavelength is easily adjusted in a predetermined range by using various metals. However, rare earths such as rare earth elements and nanoparticles having a uniform particle size, which are expensive, are required. On the other hand, the organic near infrared fluorescent material can be relatively easily synthesized and the wavelength thereof is easily adjusted, but the material capable of being stably mixed into the resin is not known.

If the near infrared fluorescent material can be mixed and dispersed in the resin, various molded articles which emit a near infrared fluorescent can be produced using the resin as a raw material. As a resin in which the near infrared fluorescent material is dispersed, for example, PTL 2 discloses a near infrared fluorescent resin in which a near infrared fluorescent material containing a reactive group, which is obtained by introducing a polyester reactive group into a phthalocyanine material, a naphthalocyanine material or a squalene material, is copolymerized in polyethylene terephthalate (PET).

On the other hand, as the organic fluorescent material having a higher emission quantum yield, a boron complex which is a **π**-conjugated compound is known, and for example, BODIPY materials having a boron dipyrromethene skeleton, in which a disubstituted boron atom and dipyrromethene (or a derivative thereof) forms a complex are known (for example, refer to NPL 1). In addition, as the BODIPY materials which emits near infrared fluorescence, in PTL 3, a BODIPY material having a heterocycle in a BODIPY skeleton is disclosed.

Furthermore, in NPL 2, a near infrared fluorescent material, which is a DPP-based boron complex having two boron complex units in the molecule, obtained by boron-complexation of a diketopyrrolopyrrole (DPP) derivative, is disclosed. These BODIPY materials and DPP-based boron complexes are mainly used as a biomarker for labeling biological molecules such as nucleic acids or proteins or tumor tissues, and there are almost no reports regarding a resin containing BODIPY materials or DPP-based boron complexes. It is disclosed in PTL 4 that, by copolymerizing, in a silicone resin, a siloxane-containing BODIPY material having an organosiloxanyl group introduced through an alkylene group, a resin which emits fluorescencein the visible light region is obtained, as the resin composition containing the BODIPY materials. In PTL 5, a composition which emits fluorescence in the visible light region obtained by mixing a BODIPY material together with a solvent in a polymer to increase the compatibility of the BODIPY material which emits the visible light is disclosed. In PTL 6, an optical filter which contains a BODIPY material having at least one electron withdrawing group and a resin and has a high absorbability of light in the visible light region is disclosed, and in PTL 7, a color conversion material which contains a BODIPY material and a resin and converts a shorter-wavelength light into a long wavelength light is disclosed.

In PTL 8, DPP boron complexes are exemplified as a compound which has absorbability in the infrared region and does not have absorbability in the visible light region, and in PTL 9, an infrared absorbing composition including the compound and a hydrophobic polymer is disclosed.

US 2011/012075 A1 relates to a fine particle containing an infrared absorptive compound represented by the following formula (1):

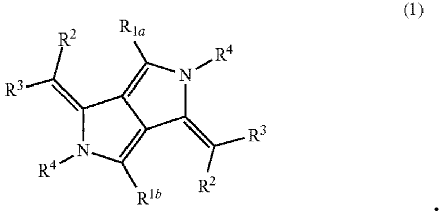

US 6005113 A relates to fluorescent dyes that are (un)substituted derivatives of 1-(isoindolyl)methylene-isoindole that are bound through both isoindole nitrogens to a boron difluoride moiety, forming a fluorescent dibenzopyrrometheneboron difluoride compound that is further substituted by bathochromic substituents that are aryl or heteroaryl moieties further substituted by an additional aryl or heteroaryl, that is itself optionally further substituted by an additional aryl or heteroaryl. JP 2009-031713 A relates to a colored photosensitive curing composition, which comprises, as its colorant, a dipyrromethene-based boron complex compound represented by formula (I),

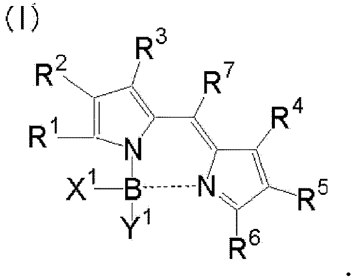

JP H08-509471 A relates to a group of fluorescent organic compounds having a variety of uses and including a tricyclic compound having the following structure (I)

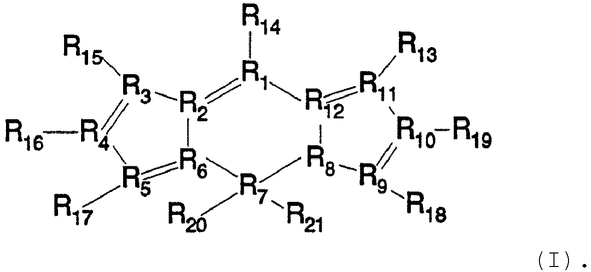

US 5433896 A relates to fluorescent dyes that are substituted or unsubstituted derivatives of 1-[isoindolyl]methylene-isoindole that are bound through both isoindole nitrogens to a boron difluoride moiety, forming a fluorescent dibenzopyrrometheneboron difluoride compound whose fluorescence properties are modified by the selection of appropriate chemical substituents.

### Citation List

### Patent Literature

[PTL 1] JP-A-2012-115535
[PTL 2] JP-A-2003-176289
[PTL 3] Japanese Patent No. 5177427
[PTL 4] JP-A-2013-060399
[PTL 5] US-A-2013/0249137
[PTL 6] US-A-2013/0252000
[PTL 7] JP-A-2011-241160
[PTL 8] Japanese Patent No. 5380019
[PTL 9] JP-A-2010-090313

### Non Patent Literature

[NPL 1] Tomimori et al., Tetrahedron, 2011, Vol. 67, pp. 3187-3193.
[NPL 2] Fischer et al., Angewandte Chemie International Edition, 2007, Vol. 46, pp. 3750-3753.

### Summary of Invention

### Technical Problem

In PTL 3, BODIPY materials which emit near infrared fluorescence are disclosed, but there is no description regarding whether these can be contained in a resin or not.

On the other hand, since a phthalocyanine-based material and the like have a low emission quantum yield of the material skeleton itself, there is a problem in that in the reactive group-containing near infrared fluorescent material which is made of these materials disclosed in PTL 2, sufficient emission intensity cannot obtained.

The siloxane-containing BODIPY material described in PTL 4 has good compatibility with a silicone monomer solution before being cured, and a silicone resin in which a material is uniformly dispersed is obtained by curing, but there is a problem that the compatibility with other resins or resin solutions is low. In the resin composition described in PTL 5, there is a possibility that the solvent remains in the resin, and thus, there is a problem in safety. In addition, in PTLs 4, 5, 6, and 7, there is no description regarding the BODIPY material which emits near infrared fluorescence, and there is also no description regarding application to medical applications. Similarly, in PTLs 8 and 9, there is no description regarding the DPP-based boron complex which emits near infrared rays, and there is also no report regarding application to medical applications.

In addition, a material which is directly covalent coupled to a polymer of a resin, such as the fluorescent material disclosed in PTL 2 or 4, is difficult to produce and has few general-purpose properties. In addition, regarding introducing of the reactive group to the material, there is a problem in that since a synthesis path is complicated, the production costs are increased. Accordingly, it is not suitable for industrial mass production. In view of the general-purpose properties, it is preferable that the near infrared fluorescent resin can be produced only by mixing and dispersing the near infrared fluorescent material into the resin. In particular, in a case where the material is dispersed into a thermoplastic resin or the like, a method of melt-kneading of a resin and a material can be considered. Even in a case where the melt-kneading is performed at a temperature lower than the decomposition point of the material, depending on the type of the resin or the material and the kneading conditions, fluorescence is not emitted due to poor dispersion or decomposition of the material, in some cases. Furthermore, whether or not the material can be dispersed in the thermoplastic resin or the like is difficult to predict from the thermal physical properties of the material.

An object of the present invention is to provide a resin composition which emits near infrared fluorescent, has a high emission quantum yield, and can be relatively easily prepared, and a molded article obtained by processing the resin composition.

### Solution to Problem

A resin composition and a molded article according to the claimed present invention are provided as the following [1] to [9].

[1] A resin composition containing a near infrared fluorescent material and a resin, in which the near infrared fluorescent material is one or more of compounds selected from the group consisting of compounds represented by the following General Formula (I₃-1), (I₃-2), (I₃-3), (I₃-4), (I₃-5), (I₃-6), (I₄-1), (I₄-2), (I₄-3), (I₄-4), (I₄-5) and (I₄-6), the content of the near infrared fluorescent material in the resin composition is within the range of 0.0001% to 0.5% by mass, and the resin composition has a maximum fluorescence wavelength of 650 nm or longer.

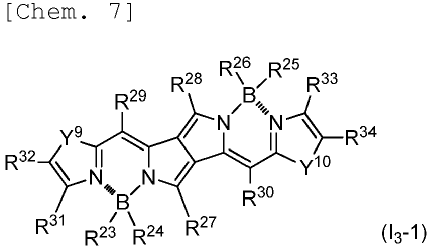

In Formula (I₃-1),
each of R²³, R²⁴, R²⁵, and R²⁶ independently represents a halogen atom, a C₁₋₂₀ alkyl group, a C₁₋₂₀ alkoxy group, an aryl group, or a heteroaryl group;
each of R²⁷ and R²⁸ independently represents a hydrogen atom, a halogen atom, a C₁₋₂₀ alkyl group, a C₁₋₂₀ alkoxy group, an aryl group, or a heteroaryl group;
each of R²⁹ and R³⁰ independently represents a hydrogen atom or an electron withdrawing group selected from a methyl halide group, a nitro group, a cyano group, an aryl group, a heteroaryl group, an alkynyl group, an alkenyl group, a substituent having a carbonyl group, a sulfoxide group, a sulfonyl group, an alkoxymethyl group, and an aminomethyl group;
each of Y⁹ and Y¹⁰ independently represents a sulfur atom, an oxygen atom, a nitrogen atom, or a phosphorus atom;
(p4) each of R³¹ and R³² independently represents a hydrogen atom, a halogen atom, a C₁₋₂₀ alkyl group, a C₁₋₂₀ alkoxy group, an aryl group, or a heteroaryl group, or
(p5) R³¹ and R³² together form an aromatic 5-membered ring which may have a substituent or an aromatic 6-membered ring which may have a substituent; and
(q4) each of R³³ and R³⁴ independently represents a hydrogen atom, a halogen atom, a C₁₋₂₀ alkyl group, a C₁₋₂₀ alkoxy group, an aryl group, or a heteroaryl group, or
(q5) R³³ and R³⁴ together form an aromatic 5-membered ring which may have a substituent or an aromatic 6-membered ring which may have a substituent.

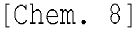

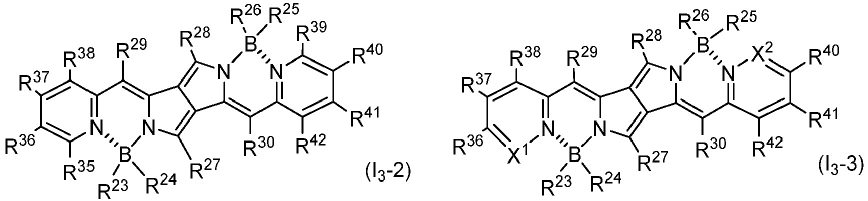

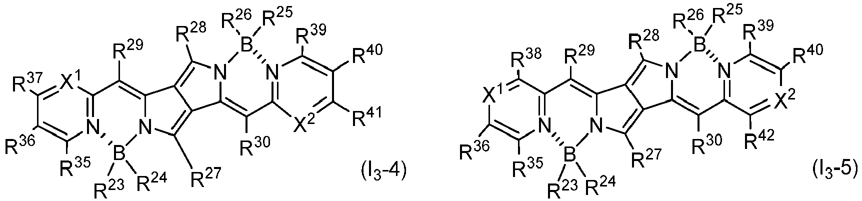

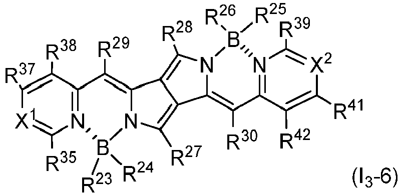

In Formulas (I₃-2) to (I₃-6),
each of R²³ to R³⁰ is the same as in Formula (I₃-1);
each of X¹ and X² independently represents a nitrogen atom or a phosphorus atom;
(p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group,
(p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group,
(p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group, or
(p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group; and
(q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group,
(q7) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group,
(q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group, or
(q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group.

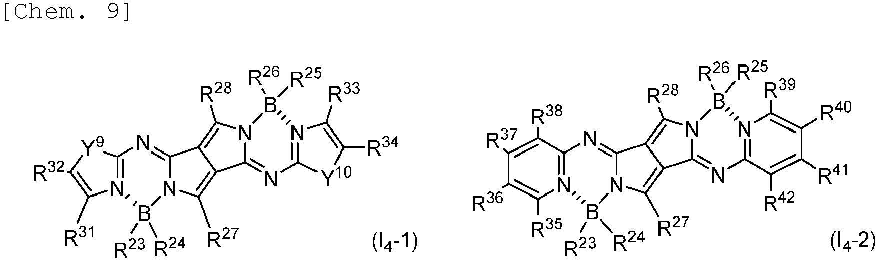

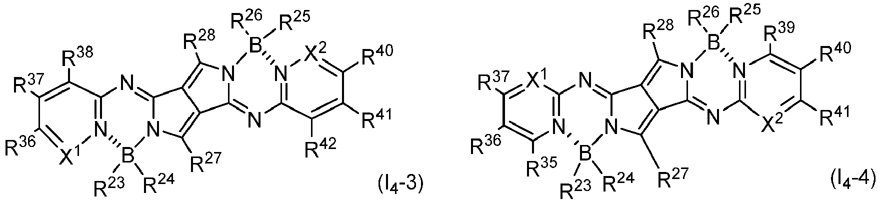

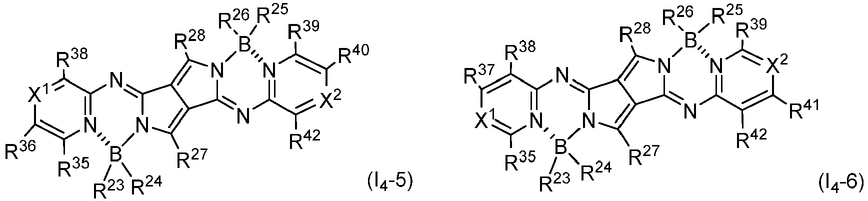

In Formulas (I₄-1) to (I₄-6), each of R²³ to R²⁸ is the same as in Formula (I₃-1), and in Formula (I₄-1), each of R³¹ to R³⁴, Y⁹, and Y¹⁰ is the same as in Formula (I₃-1), in Formulas (I₄-2) to (I₄-6), each of R³⁵ to R⁴² is the same as in Formula (I₃-2), and in Formulas (I₄-3) to (I₄-6), each of X¹ and X² is the same as in Formula (I₃-3).

[2] The resin composition according to [1], containing one or more of compounds selected from the group consisting of compounds represented by any one of the following General Formulas (I₃-7) to (I₃-9) and (I₄-7) to (I₄-9).

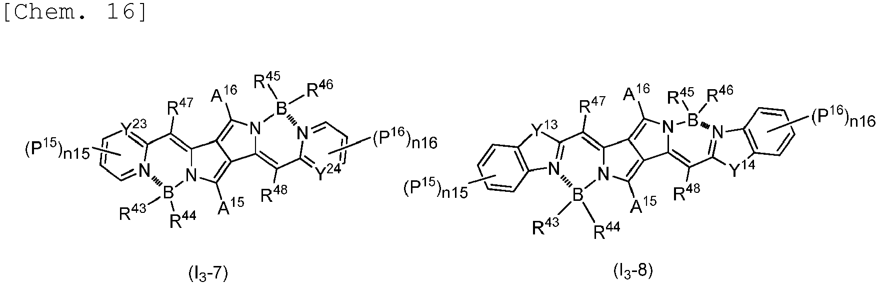

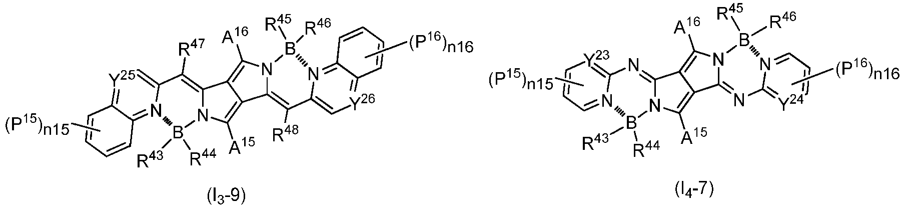

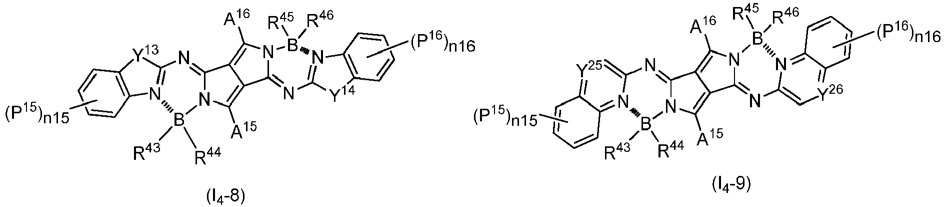

In the formulas,
each of Y²³ and Y²⁴ independently represents a carbon atom or a nitrogen atom;
each of Y¹³ and Y¹⁴ independently represents an oxygen atom or a sulfur atom;
each of Y²⁵ and Y²⁶ independently represents a carbon atom or a nitrogen atom;
each of R⁴⁷ and R⁴⁸ independently represents a hydrogen atom or an electron withdrawing group selected from a methyl halide group, a nitro group, a cyano group, an aryl group, a heteroaryl group, an alkynyl group, an alkenyl group, a substituent having a carbonyl group, a sulfoxide group, a sulfonyl group, an alkoxymethyl group, and an aminomethyl group;
each of R⁴³, R⁴⁴, R⁴⁵, and R⁴⁶ represents a halogen atom or an aryl group which may have a substituent;
each of P¹⁵ and P¹⁶ independently represents a halogen atom, a C₁₋₂₀ alkyl group, a C₁₋₂₀ alkoxy group, an amino group, a monoalkylamino group, or a dialkylamino group; each of n15 and n16 independently represents an integer of 0 to 3; and
each of A¹⁵ and A¹⁶ independently represents a phenyl group which may have one to three substituents selected from the group consisting of a halogen atom, a C₁₋₂₀ alkyl group, a C₁₋₂₀ alkoxy group, an amino group, a monoalkylamino group, or a dialkylamino group.

[3] The resin composition according to [1] or [2], containing one or more of compounds selected from the group consisting of compounds represented by any one of the following General Formulas (6-1) to (6-12) and (7-1) to (7-12): 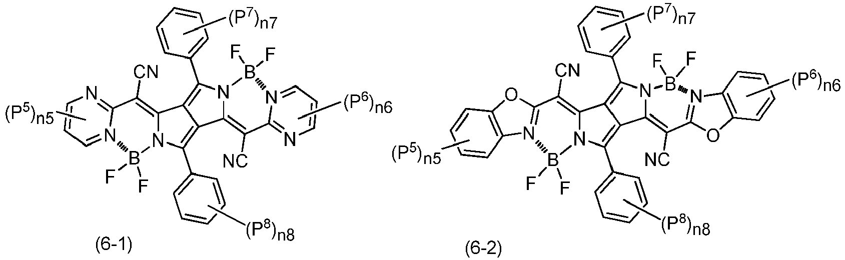 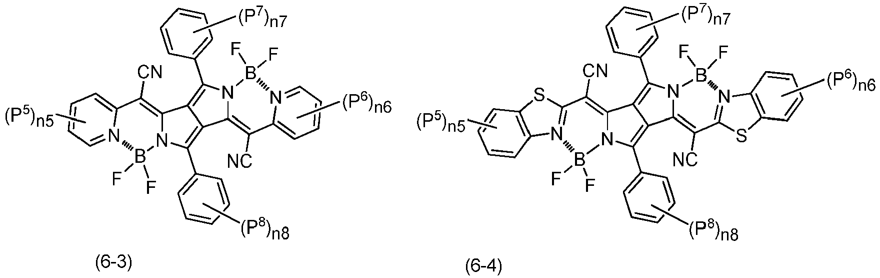 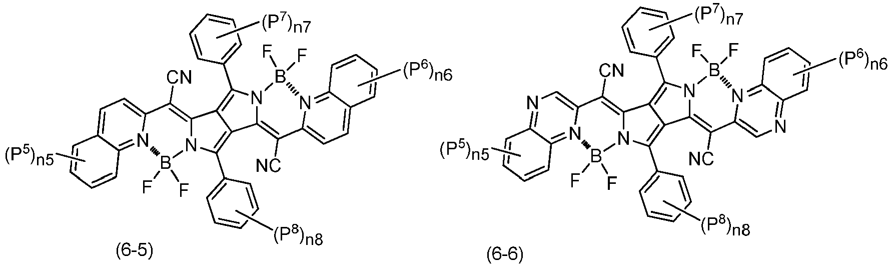 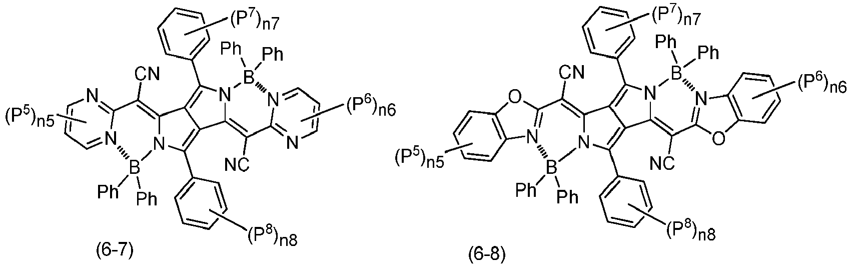 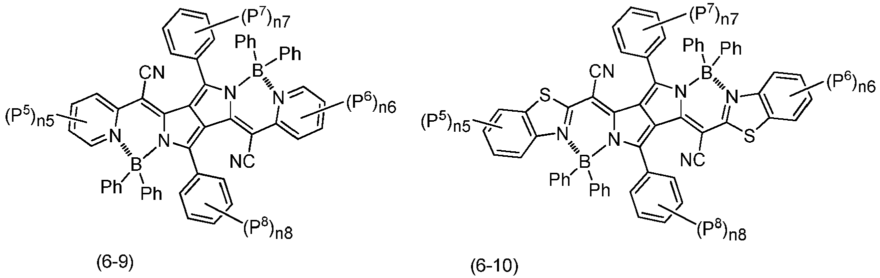 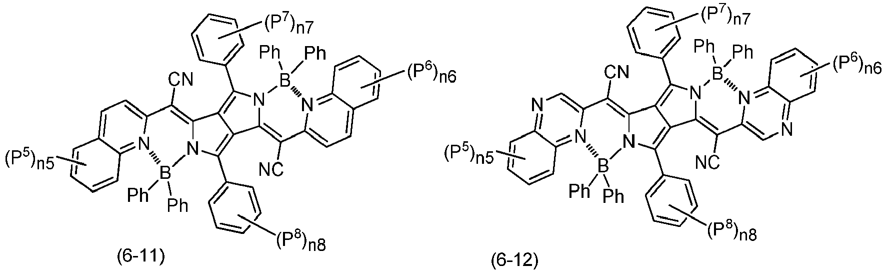 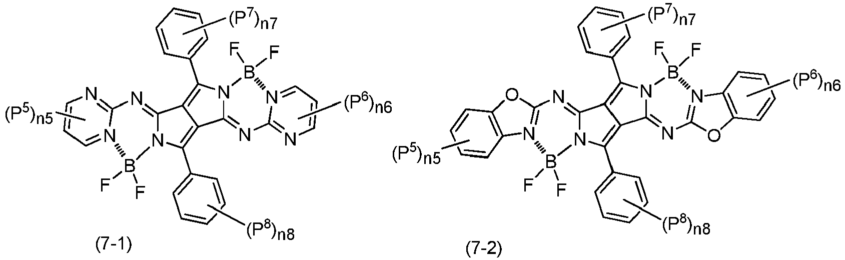 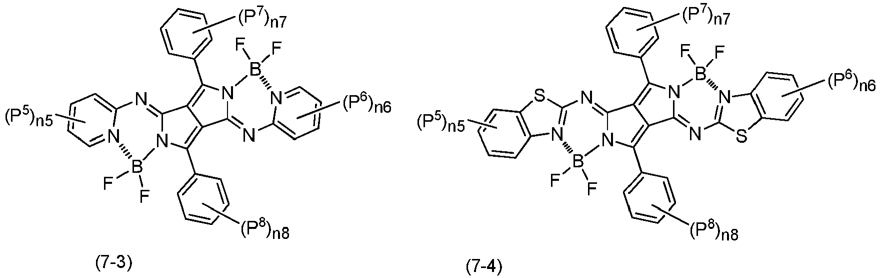 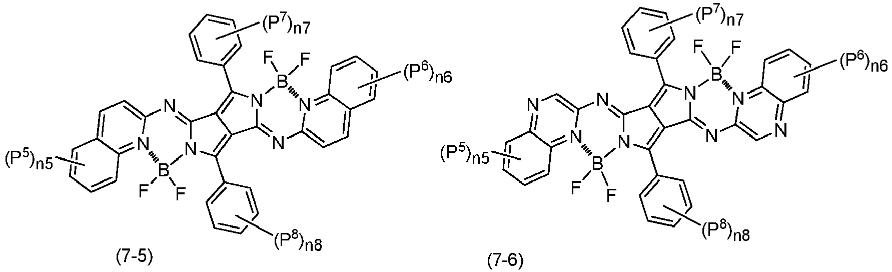 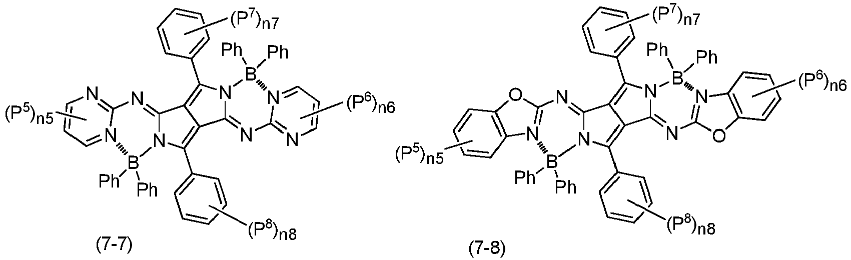 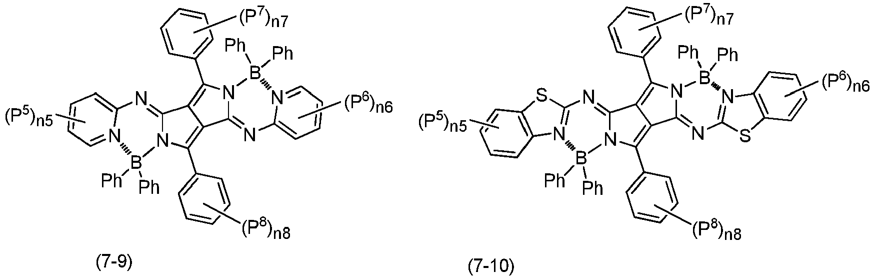 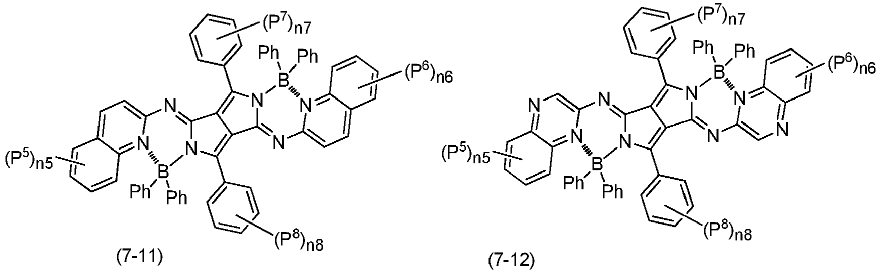 wherein each of P⁵ to P⁸ independently represents a halogen atom, a C₁₋₂₀ alkyl group, a C₁₋₂₀ alkoxy group, an amino group, a monoalkylamino group, or a dialkylamino group;
each of n5 to n8 independently represents an integer of 0 to 3.

[4] The resin composition according to any one of [1] to [3], in which the resin is a thermoplastic resin.

[5] The resin composition according to any one of [1] to [4], in which the near infrared fluorescent material and the resin are melt-kneaded.

[6] The resin composition according to any one of [1] to [5], in which the maximum fluorescence wavelength is 700 nm or longer.

[7] The resin composition according to any one of [1] to [6], which is used as a medical material.

[8] A molded article obtained by processing the resin composition according to any one of [1] to [7].

[9] The molded article according to [8], in which at least a part of the molded article is a medical tool that is used in the body of a patient.

### Advantageous Effects of Invention

In the present invention, a BODIPY material or the DPP-based boron complex having excellent heat resistance and emission quantum yield, which emits a near infrared fluorescence is used. Therefore, the near infrared fluorescent resin composition having strong emission intensity in a condition not causing a copolymerization reaction between an organic near infrared fluorescent material and the resin component, and a molded article which is obtained by processing of the composition can be obtained.

### Brief Description of Drawings

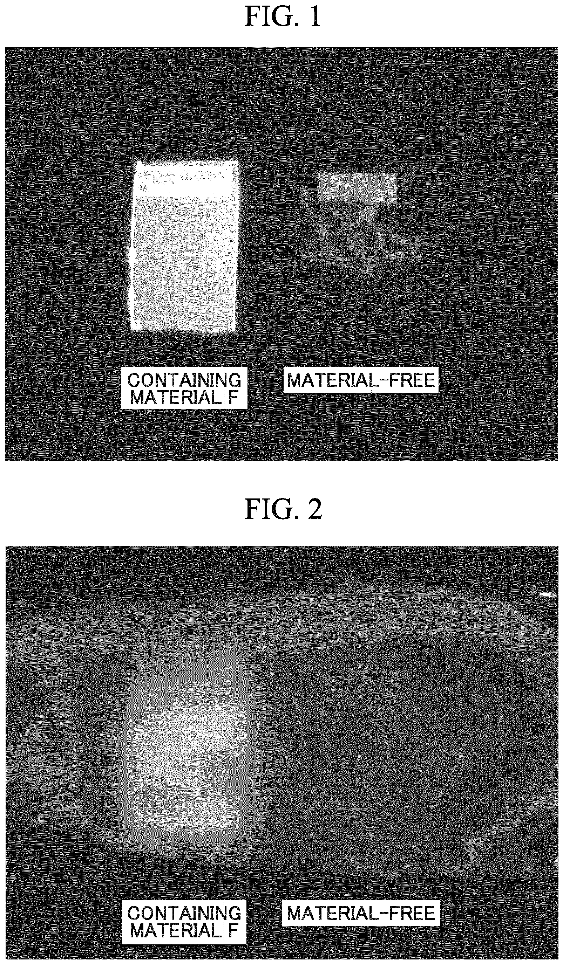

Fig. 1 is a photograph of a material-containing film of a near infrared fluorescent material F (in Fig. 1, "CONTAINING MATERIAL F") and a material-free film (in Fig. 1, "MATERIAL-FREE") taken using a near infrared fluorescent detection camera, in Example 9.
Fig. 2 is a photograph of a material-containing film of a near infrared fluorescent material F (in Fig. 2, "CONTAINING MATERIAL F ") and a material-free film (in Fig. 2, "MATERIAL-FREE") over a piece of pork having a thickness of 2 mm taken using a near infrared fluorescent detection camera, in Example 9.

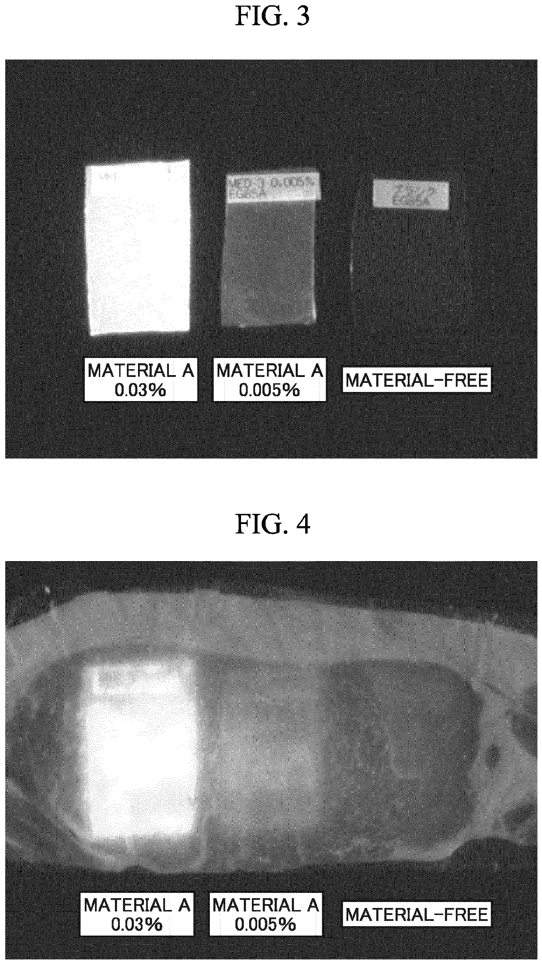

Fig. 3 is a photograph of a 0.03% by mass near infrared fluorescent material A-containing film (in Fig. 3, "MATERIAL A 0.03%"), a 0.005% by mass near infrared fluorescent material A-containing film (in Fig. 3, "MATERIAL A 0.005%"), and a material-free film (in Fig. 3, "MATERIAL-FREE") taken using a near infrared fluorescent detection camera, in Example 12.
Fig. 4 is a photograph of a 0.03% by mass near infrared fluorescent material A-containing film (in Fig. 4, "MATERIAL A 0.03%"), a 0.005% by mass near infrared fluorescent material A-containing film (in Fig. 4, "MATERIAL A 0.005%"), and a material-free film (in Fig. 4, "MATERIAL-FREE") over a piece of pork having a thickness of 2 mm taken using a near infrared fluorescent detection camera, in Example 12.

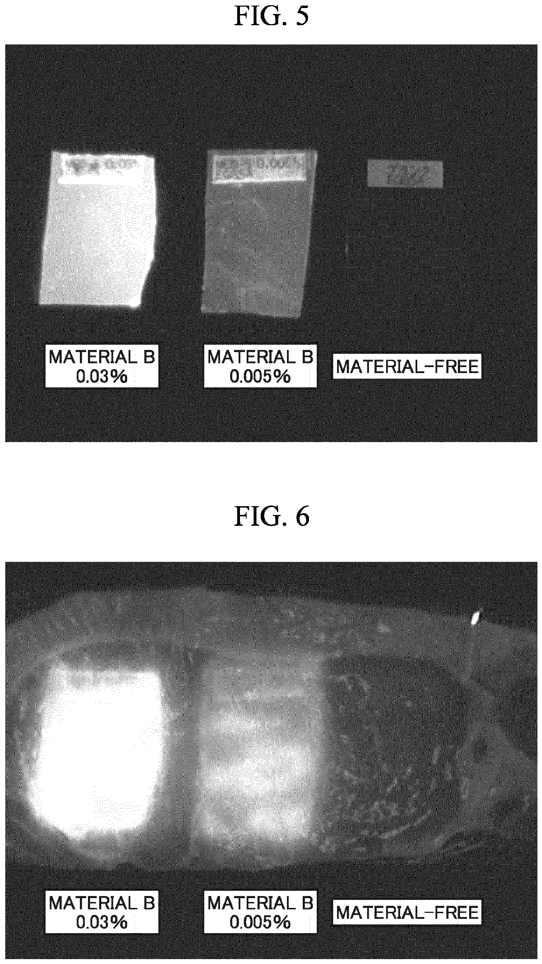

Fig. 5 is a photograph of a 0.03% by mass near infrared fluorescent material B-containing film (in Fig. 5, "MATERIAL B 0.03%"), a 0.005% by mass near infrared fluorescent material B-containing film (in Fig. 5, "MATERIAL B 0.005%"), and a material-free film (in Fig. 5, "MATERIAL-FREE") taken using a near infrared fluorescent detection camera, in Example 13.
Fig. 6 is a photograph of a 0.03% by mass near infrared fluorescent material B-containing film (in Fig. 6, "MATERIAL B 0.03%"), a 0.005% by mass near infrared fluorescent material B-containing film (in Fig. 6, "MATERIAL B 0.005%"), and a material-free film (in Fig. 6, "MATERIAL-FREE") over a piece of pork having a thickness of 2 mm taken using a near infrared fluorescent detection camera, in Example 13.

### Description of Embodiments

A resin composition according to the present invention includes a near infrared fluorescent material and a resin, and has a maximum fluorescence wavelength of 650 nm or longer. Since the resin composition according to the present invention emits a near infrared fluorescent, and is easily molded, the resin composition can be suitably used as a medical material such as a raw material for a medical tool which is used in a living body or the like.

### <Near Infrared Fluorescent Material>

A near infrared fluorescent material contained in the resin composition according to the claimed present invention is one or more of compounds selected from the group consisting of compounds represented by the following General Formula (I₃-1), (I₃-2), (I₃-3), (I₃-4), (I₃-5), (I₃-6), (I₄-1), (I₄-2), (I₄-3), (I₄-4), (I₄-5) and (I₄-6). Hereinafter, the compound is referred to as a "near infrared fluorescent material according to the present invention" sometimes. The present specification further includes information about compounds represented by the following General Formula (I₁), (I₂), (I₃), or (I₄), which are useful to understand the invention, and the compounds are referred to as a "near infrared fluorescent material according to the present specification" sometimes.

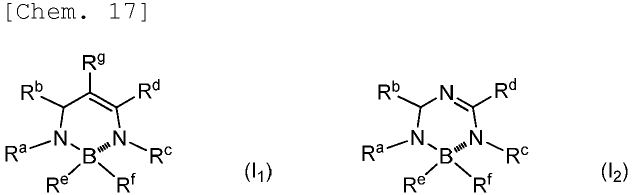

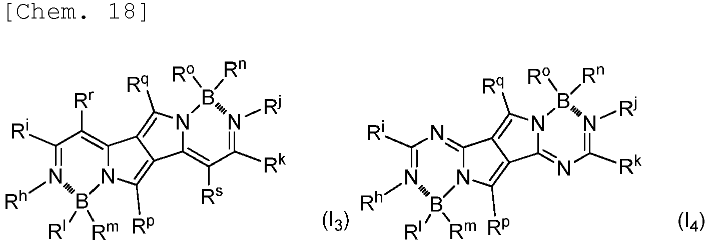

In General Formula (I₁) or (I₂), R^{a} and R^{b} form an aromatic ring consisting of one to three rings together with the nitrogen atom to which R^{a} is bonded and the carbon atom to which R^{b} is bonded. Similarly, in General Formula (I₁) or (I₂), R^{c} and R^{d} form an aromatic ring consisting of one to three rings together with the nitrogen atom to which R^{c} is bonded and the carbon atom to which R^{d} is bonded. Each ring of the aromatic ring which R^{a} and R^{b} form and the aromatic ring which R^{c} and R^{d} form is a 5-membered ring or a 6-membered ring. The compound represented by General Formula (I₁) or (I₂) has a ring structure formed by condensation of the aromatic ring which R^{a} and R^{b} form and the aromatic ring which R^{c} and R^{d} form by a ring including the boron atom bonded to two nitrogen atoms. That is, the compound represented by General Formula (I₁) or (I₂) has a rigid condensed ring structure configured of a wide conjugate plane.

In General Formula (I₃) or (I₄), R^{h} and Rⁱ form an aromatic ring consisting of one to three rings together with the nitrogen atom to which R^{h} is bonded and the carbon atom to which Rⁱ is bonded. Similarly, in General Formula (I₃) or (I₄), R^{j} and R^{k} form an aromatic ring consisting of one to three rings together with the nitrogen atom to which R^{j} is bonded and the carbon atom to which R^{k} is bonded. Each ring of the aromatic ring which R^{h} and Rⁱ form and the aromatic ring which R^{j} and R^{k} form is a 5-membered ring or a 6-membered ring. The compound represented by General Formula (I₃) or (I₄) has a ring structure formed by condensation between the 5-membered hetero rings in three rings formed by condensation of the aromatic ring which R^{h} and Rⁱ form, the ring including the boron atom bonded to two nitrogen atoms, and a 5-membered hetero ring including one nitrogen atom and three rings formed by condensation of the aromatic ring which R^{j} and R^{k} form, the ring including the boron atom bonded to two nitrogen atoms, and a 5-membered hetero ring including one nitrogen atom, that is, a ring structure formed by condensation of at least 6 rings. In this manner, the compound represented by General Formula (I₃) or (I₄) has a rigid condensed ring structure configured of a very wide conjugate plane.

Each of the aromatic ring which R^{a} and R^{b} form, the aromatic ring which R^{c} and R^{d} form, the aromatic ring which R^{h} and Rⁱ form, and the aromatic ring which R^{j} and R^{k} form is not particularly limited as long as it has aromaticity. Examples of the aromatic ring include a pyrrole ring, an imidazole ring, a pyrazole ring, an oxazole ring, a thiazole ring, a pyridine ring, a pyrimidine ring, a pyridazine ring, an isoindole ring, an indole ring, an indazole ring, a purine ring, a perimidine ring, a thienopyrrole ring, a furopyrrole ring, a pyrrolothiazole ring, and a pyrrolooxazole ring. Since the maximum fluorescence wavelength becomes a longer wavelength to the near infrared region, in particular, in the case of General Formula (I₁) or (I₃), the number of condensed rings of the aromatic ring is preferably 2 or 3, and more preferably 2 from the viewpoint of complexity of synthesis. Here, even in a case where the number of condensed rings of the aromatic ring is 1, it is also possible to make wavelengths be longer by devising the substituent on the ring or boron. In addition, in particular, in the case of General Formula (I₂) or (I₄), it is possible to make wavelengths be longer to the near infrared region by simply bonding a substituted aryl group or a heteroaryl group thereto.

Each of the aromatic ring which R^{a} and R^{b} form, the aromatic ring which R^{c} and R^{d} form, the aromatic ring which R^{h} and Rⁱ form, and the aromatic ring which R^{j} and R^{k} form may not have a substituent or may have one or plural substituents. The substituent which the aromatic ring may be "any group which does not inhibit fluorescence of a compound".

In a case where the resin composition according to the present invention is used as a medical material (raw material for medical tools), the near infrared fluorescent material according to the present invention is preferably a near infrared fluorescent material of which mutagenicity, cytotoxicity, sensitization, skin irritation, and the like are not contained in the required biological safety testing. In addition, from the viewpoint of safety, the near infrared fluorescent material according to the present invention is preferably not eluted from a molded article obtained by processing the resin composition of the present invention by body fluid such as blood or tissue fluid. Thus, the near infrared fluorescent material according to the present invention preferably has a low solubility in biological components such as blood. Here, even when the near infrared fluorescent material according to the present invention is water-soluble, the resin component itself in the resin composition according to the present invention is hardly eluted into the body fluid or the like in some cases, and in a case where the content of the near infrared fluorescent material itself is a very small amount, the molded article of the resin composition according to the present invention can be used while avoiding elution of the near-infrared fluorescent material even in vivo. Considering these, as the substituent, a substituent which is less likely to express mutagenicity or the like or decreases water solubility is preferably selected.

Examples of the substituent include a halogen atom, a nitro group, a cyano group, a hydroxy group, a carboxyl group, an aldehyde group, a sulfonic acid group, an alkylsulfonyl group, a halogenosulfonyl group, a thiol group, an alkylthio group, an isocyanate group, a thioisocyanate group, an alkyl group, an alkenyl group, an alkynyl group, an alkoxy group, an alkoxycarbonyl group, an alkyl amide carbonyl group, an alkyl carbonyl amide group, an acyl group, an amino group, a monoalkylamino group, a dialkylamino group, a silyl group, a monoalkylsilyl group, a dialkylsilyl group, a trialkylsilyl group, a monoalkoxysilyl group, a dialkoxysilyl group, a trialkoxysilyl group, an aryl group, and a heteroaryl group. The substituents which the aromatic ring which R^{a} and R^{b} form, the aromatic ring which R^{c} and R^{d} form has, the aromatic ring which R^{h} and Rⁱ form, or the aromatic ring which R^{j} and R^{k} form are preferably a cyano group, a hydroxy group, a carboxyl group, an alkylthio group, an alkyl group, an alkoxy group, an alkoxycarbonyl group, an amide group, an alkylsulfonyl group, fluorine, chlorine, an aryl group, or a heteroaryl group, from the viewpoint of safety with respect to a living body, and these substituents may further have a substituent. Here, since, even in the case of a substituent other than these substituents, it is possible to improve safety by further introducing a suitable substituent, the present specification is not limited to these substituents.

Examples of the halogen atom include a fluorine atom, a chlorine atom, a bromine atom, and an iodine atom, and a fluorine atom, a chlorine atom, or a bromine atom is preferable, and a fluorine atom is more preferable.

The alkyl group, the alkenyl group, and the alkynyl group may be linear, branched, or cyclic (aliphatic cyclic group) . Each of these groups preferably has 1 to 20 carbon atoms, more preferably 1 to 12 carbon atoms, still more preferably 1 to 6 carbon atoms. Examples of the alkyl group include a methyl group, an ethyl group, a propyl group, an isopropyl group, a n-butyl group, an isobutyl group, a t-butyl group (tert-butyl group), a pentyl group, an isoamyl group, a hexyl group, a heptyl group, an octyl group, a nonyl group, a decyl group, a undecyl group, and a dodecyl group. Examples of the alkenyl group include a vinyl group, an allyl group, a 1-propenyl group, an isopropenyl group, a 2-butenyl group, a 1, 3-butadienyl group, a 2-pentenyl group, and a 2-hexenyl group. Examples of the alkynyl group include an ethynyl group, a 1-propynyl group, a 2-propynyl group, an isopropynyl group, a 1-butynyl group, and an isobutynyl group.

Examples of the alkyl group portion in an alkylsulfonyl group, an alkylthio group, an alkoxy group, an alkoxycarbonyl group, an alkyl amide carbonyl group, an alkyl carbonyl amide group, a monoalkylamino group, a dialkylamino group, a monoalkylsilyl group, a dialkylsilyl group, a trialkylsilyl group, a monoalkoxysilyl group, a dialkoxysilyl group, and a trialkoxysilyl group include the same as the alkyl groups described above. Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group. In addition, examples of the monoalkylamino group include a methylamino group, an ethylamino group, a propylamino group, an isopropylamino group, a butylamino group, an isobutyl amino group, a t-butylamino group, a pentylamino group, and a hexylamino group, and examples of the dialkylamino group include a dimethylamino group, a diethylamino group, a dipropylamino group, a diisopropylamino group, a dibutylamino group, a diisobutylamino group, a dipentylamino group, a dihexylamino group, an ethylmethylamino group, a methylpropylamino group, a butylmethylamino group, an ethylpropylamino group, and a butylethylamino group.

Examples of the aryl group include a phenyl group, a naphthyl group, an indenyl group, and a biphenyl group. The aryl group is preferably a phenyl group.

Examples of the "heteroaryl group" include 5-membered ring heteroaryl groups such as a pyrrolyl group, an imidazolyl group, a pyrazolyl group, a thienyl group, a furanyl group, an oxazolyl group, an isoxazolyl group, a thiazolyl group, an isothiazolyl group, and a thiadiazole group; 6-membered ring heteroaryl groups such as a pyridinyl group, a pyrazinyl group, a pyrimidinyl group, and a pyridazinyl group; and condensed heteroaryl groups such as an indolyl group, an isoindolyl group, an indazolyl group, a quinolizinyl group, a quinolinyl group, an isoquinolinyl group, a benzofuranyl group, an isobenzofuranyl group, a chromenyl group, a benzoxazolyl group, a benzisoxazolyl group, a benzothiazolyl group, and a benzisothiazolyl group.

Each of the alkyl group, the alkenyl group, the alkynyl group, the aryl group, and the heteroaryl group may be an unsubstituted group, or may be a group in which one or more hydrogen atoms are substituted with substituents. Examples of the substituent include a halogen atom, an alkyl group, an alkoxy group, a nitro group, a cyano group, a hydroxy group, an amino group, a thiol group, a carboxyl group, an aldehyde group, a sulfonic acid group, an isocyanate group, a thioisocyanate group, an aryl group, and a heteroaryl group.

The absorption wavelength and the fluorescence wavelength of the fluorescent material are dependent on the surrounding environment. Therefore, the absorption wavelength of the fluorescent material in the resin becomes shorter in some cases and becomes longer in some cases, than that in a solution. In a case where the absorption wavelength of the near infrared fluorescent material according to the present invention becomes a longer wavelength, the maximum absorption wavelength becomes so as to be in the near infrared region even in various resins, and thus, this is preferable. The maximum absorption wavelength of the fluorescent material can become a longer wavelength by narrowing the band gap between the highest occupied molecular orbital (HOMO) and the lowest unoccupied molecular orbital (LUMO) by introducing an electron donating group and an electron withdrawing group into a suitable position in the molecule.

For example, in the compound represented by General Formula (I₁), the maximum absorption wavelength and the maximum fluorescence wavelength of the compound can become longer wavelengths by introducing electron donating groups into the aromatic ring which R^{a} and R^{b} form and the aromatic ring which R^{c} and R^{d} form and introducing an electron withdrawing group into R^{g}. Similarly, in the compound represented by General Formula (I₃), the maximum absorption wavelength and the maximum fluorescence wavelength of the compound can become longer wavelengths by introducing electron donating groups into the aromatic ring which R^{h} and Rⁱ form and the aromatic ring which R^{j} and R^{k} form, introducing, in a case where each of RP and R^{q} has an aromatic ring, an electron donating group into the aromatic ring, or introducing an electron withdrawing group into R^{r} and R^{s}. By suitably combining these designs, it is possible to adjust to a target wavelength.

The compound represented by General Formula (I₂) having an aza BODIPY skeleton has a skeleton having absorption at a relatively long wavelength even in a case where the aromatic ring which R^{a} and R^{b} form and the aromatic ring which R^{c} and R^{d} form are unsubstituted. For example, in the skeleton, the crosslinking portion of the pyrrole is a nitrogen atom, and thus, it is not possible to introduce a substituent on the nitrogen, unlike the compound represented by General Formula (I₁), but by introducing electron donating groups into the pyrrole portions (the aromatic ring which R^{a} and R^{b} form and the aromatic ring which R^{c} and R^{d} form), the maximum absorption wavelength and the maximum fluorescence wavelength of the compound can become longer wavelengths. Similarly, in the case of the compound represented by General Formula (I₄), the maximum absorption wavelength and the maximum fluorescence wavelength of the compound can become longer wavelengths by introducing electron donating groups into the pyrrole portions (the aromatic ring which R^{h} and Rⁱ form and the aromatic ring which R^{j} and R^{k} form), or in a case where each of R^{p} and R^{q} has an aromatic ring, introducing an electron donating group into the aromatic ring.

Therefore, as a substituent of the aromatic ring which R^{a} and R^{b} form, the aromatic ring which R^{c} and R^{d} form, the aromatic ring which R^{h} and Rⁱ form, and the aromatic ring which R^{j} and R^{k} form, a group which functions as an electron donating group with respect to the aromatic rings, among "any groups which does not inhibit fluorescence of a compound", is preferable. By introducing an electron donating group into the aromatic ring, fluorescence of the compound represented by General Formula (I₁), (I₂), (I₃), or (I₄) becomes a longer wavelength side. Examples of the group which functions as an electron donating group include an alkyl group; an alkoxy group such as a methoxy group; an aryl group (aromatic ring group) such as a phenyl group, a p-alkoxyphenyl group, a p-dialkylaminophenyl group, or a dialkoxyphenyl group; and a heteroaryl group (heteroaromatic ring) such as a 2-thienyl group or a 2-furanyl group. As the alkyl group, the alkyl group in a substituent of the phenyl group, and the alkyl group portion in the alkoxy group, a linear or branched alkyl group having 1 to 10 carbon atoms is preferable. Moreover, the number of carbon atoms in the alkyl portion or the presence or absence of a branch may be suitably selected in view of the physical properties of the material. From the viewpoint of solubility, compatibility, or the like, it is preferable in some cases that the alkyl portion has 6 or more carbon atoms or it is preferable in some cases that the alkyl portion is branched. As a substituent having the aromatic ring which R^{a} and R^{b} form, the aromatic ring which R^{c} and R^{d} form, the aromatic ring which R^{h} and Rⁱ form, and the aromatic ring which R^{j} and R^{k} form, a C₁₋₆ alkyl group, a C₁₋₆ alkoxy group, an aryl group, or a heteroaryl group is preferable, a methyl group, an ethyl group, a methoxy group, a phenyl group, a p-methoxyphenyl group, a p-ethoxyphenyl group, a p-dimethylaminophenyl group, a dimethoxyphenyl group, a thienyl group, or a furanyl group is more preferable, and a methyl group, an ethyl group, a methoxy group, a phenyl group, or a p-methoxyphenyl group is still more preferable. Since the BODIPY skeleton have high planarity, the molecules thereof are likely to be aggregated to each other by **π-π** stacking. By introducing an aryl group or a heteroaryl group having a bulky substituent into the BODIPY skeleton, it is possible to suppress aggregation of the molecules, and it is possible to increase the emission quantum yield of the resin composition according to the present invention.

In General Formula (I₁) or (I₂), the aromatic ring which R^{a} and R^{b} form and the aromatic ring which R^{c} and R^{d} form may be different from each other or the same type. In General Formula (I₃) or (I₄), the aromatic ring which R^{h} and Rⁱ form and the aromatic ring which R^{j} and R^{k} form may be different from each other or the same type. Since the near infrared fluorescent material according to the present specification can be easily synthesized and tends to have a higher emission quantum yield, the aromatic ring which R^{a} and R^{b} form, the aromatic ring which R^{c} and R^{d} form, the aromatic ring which R^{h} and Rⁱ form, and the aromatic ring which R^{j} and R^{k} form are preferably the same type.

In General Formula (I₁) or (I₂), each of R^{e} and R^{f} independently represents a halogen atom or an oxygen atom. In a case where each of R^{e} and R^{f} is a halogen atom, a fluorine atom, a chlorine atom, a bromine atom, or an iodine atom is preferable, a fluorine atom or a chlorine atom is more preferable, and a fluorine atom is particularly preferable since it has a strong bond to the boron atom. Since a compound in which each of R^{e} and R^{f} is a fluorine atom has high heat resistance, the compound is advantageous in the case of being melt-kneaded together with a resin at a high temperature. Moreover, even in a case where the compound represented by General Formula (I₁) or (I₂) has a substituent in which each of R^{e} and R^{f} includes an atom which can bond to a boron atom rather than a halogen atom or an oxygen atom, the compound can be contained in a resin in the same manner as the near infrared fluorescent material according to the present invention. As the substituent, any substituent is acceptable as long as it does not inhibit fluorescence.

In General Formula (I₁) or (I₂), in a case where R^{e} and R^{f} are oxygen atoms, R^{e}, the boron atom bonded to R^{e}, R^{a}, and the nitrogen atom bonded to R^{a} may together form a ring, and R^{f}, the boron atom bonded to R^{f}, R^{c}, and the nitrogen atom bonded to R^{c} may together form a ring. That is, in the case of forming a ring structure, the ring which R^{e}, the boron atom bonded to R^{e}, R^{a}, and the nitrogen atom bonded to R^{a} form is condensed with the aromatic ring which R^{a} and R^{b} form, and the ring which R^{f}, the boron atom bonded to R^{f}, R^{c}, and the nitrogen atom bonded to R^{c} form is condensed with the aromatic ring which R^{c} and R^{d} form. The ring which R^{e} and the like forms and the ring which R^{f} and the like forms are preferably 6-membered rings.

In General Formula (I₁) or (I₂), in a case where R^{e} is an oxygen atom and does not form a ring, R^{e} is an oxygen atom having a substituent (an oxygen atom bonded to a substituent) . Examples of the substituent include a C₁₋₂₀ alkyl group, an aryl group, a heteroaryl group, an alkylcarbonyl group, an arylcarbonyl group, or a heteroarylcarbonyl group. Similarly, in General Formula (I₁) or (I₂), in a case where R^{f} is an oxygen atom and does not form a ring, R^{f} is an oxygen atom having a substituent (an oxygen atom bonded to a substituent). Examples of the substituent include a C₁₋₂₀ alkyl group, an aryl group, a heteroaryl group, an alkylcarbonyl group, an arylcarbonyl group, or a heteroarylcarbonyl group. Moreover, in a case where both of R^{e} and R^{f} are oxygen atoms having a substituent, the substituent which R^{e} has and the substituent which R^{f} has may be the same as or different from each other.

In General Formula (I₁) or (I₂), in a case where each of R^{e} and R^{f} is an oxygen atom, R^{e}, R^{f}, and the boron atom bonded to R^{e}, R^{f} may together form a ring. Examples of the ring structure include a structure in which R^{e} and R^{f} are connected to the same aryl ring or heteroaryl ring and a structure in which R^{e} and R^{f} are connected by an alkylene group.

In General Formula (I₃) or (I₄), each of R^{l}, R^{m}, Rⁿ, and R^{o} independently represents a halogen atom, a C₁₋₂₀ alkyl group, a C₁₋₂₀ alkoxy group, an aryl group, or a heteroaryl group. In a case where each of R^{l}, R^{m}, Rⁿ, and R^{o} is a halogen atom, a fluorine atom, a chlorine atom, a bromine atom, or an iodine atom is preferable, a fluorine atom or a chlorine atom is more preferable, and a fluorine atom is particularly preferable since it has a strong bond to the boron atom. Since a compound in which each of R^{l}, R^{m}, Rⁿ, and R^{o} is a fluorine atom has high heat resistance, the compound is advantageous in the case of being melt-kneaded together with a resin at a high temperature.

Moreover, in the present invention and the present specification, the "C₁₋₂₀ alkyl group" means an alkyl group having 1 to 20 carbon atoms, and the "C₁₋₂₀ alkoxy group" means an alkoxy group having 1 to 20 carbon atoms.

In a case where R^{l}, R^{m}, Rⁿ, or R^{o} is a C₁₋₂₀ alkyl group, the alkyl group may be linear, branched, or cyclic (aliphatic cyclic group). Examples of the alkyl group include a methyl group, an ethyl group, a propyl group, an isopropyl group, a n-butyl group, an isobutyl group, a t-butyl group, a pentyl group, an isoamyl group, a hexyl group, a heptyl group, an octyl group, a nonyl group, a decyl group, a undecyl group, and a dodecyl group.

In a case where R^{l}, R^{m}, Rⁿ, or R^{o} is a C₁₋₂₀ alkoxy group, the alkyl group portion of the alkoxy group may be linear, branched, or cyclic (aliphatic cyclic group) . Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group.

In a case where R^{l}, R^{m}, Rⁿ, or R^{o} is an aryl group, examples of the aryl group include a phenyl group, a naphthyl group, an indenyl group, and a biphenyl group.

In a case where R^{l}, R^{m}, Rⁿ, or R^{o} is a heteroaryl group, examples of the heteroaryl group include 5-membered ring heteroaryl groups such as a pyrrolyl group, an imidazolyl group, a pyrazolyl group, a thienyl group, a furanyl group, an oxazolyl group, an isoxazolyl group, a thiazolyl group, an isothiazolyl group, and a thiadiazole group; 6-membered ring heteroaryl groups such as a pyridinyl group, a pyrazinyl group, a pyrimidinyl group, and a pyridazinyl group; and condensed heteroaryl groups such as an indolyl group, an isoindolyl group, an indazolyl group, a quinolizinyl group, a quinolinyl group, an isoquinolinyl group, a benzofuranyl group, an isobenzofuranyl group, a chromenyl group, a benzoxazolyl group, a benzisoxazolyl group, a benzothiazolyl group, and a benzisothiazolyl group.

Each of the C₁₋₂₀ alkyl group, the C₁₋₂₀ alkoxy group, the aryl group, and the heteroaryl group represented by R^{l}, R^{m}, Rⁿ, or R^{o} may be an unsubstituted group, or may be a group in which one or more hydrogen atoms are substituted with substituents. Examples of the substituent include a halogen atom, an alkyl group, an alkoxy group, a nitro group, a cyano group, a hydroxy group, an amino group, a thiol group, a carboxyl group, an aldehyde group, a sulfonic acid group, an isocyanate group, a thioisocyanate group, an aryl group, and a heteroaryl group.

As the compound represented by General Formula (I₃) or (I₄), a compound in which each of R^{l}, R^{m}, Rⁿ, and R^{o} is a halogen atom, an unsubstituted aryl group, or an aryl group having a substituent is preferable, a compound in which each of R^{l}, R^{m}, Rⁿ, and R^{o} is a fluorine atom, a chlorine atom, a bromine atom, an unsubstituted phenyl group, or a phenyl group substituted with a C₁₋₂₀ alkyl group or a C₁₋₂₀ alkoxy group is preferable, a compound in which each of R^{l}, R^{m}, Rⁿ, and R^{o} is a fluorine atom, a chlorine atom, an unsubstituted phenyl group, or a phenyl group substituted with a C₁₋₁₀ alkyl or a C₁₋₁₀ alkoxy group is more preferable, and a compound in which each of R^{l}, R^{m}, Rⁿ, and R^{o} is a fluorine atom or an unsubstituted phenyl group is particularly preferable.

In General Formula (I₃) or (I₄), each of R^{p} and R^{q} independently represents a hydrogen atom, a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by RP or R^{q} include the same as those represented by R^{l}, R^{m}, Rⁿ, or R^{o} in General Formula (I₃).

As the compound represented by General Formula (I₃) or (I₄), a compound in which each of R^{p} and R^{q} is a hydrogen atom or an aryl group is preferable, a compound in which each of R^{p} and R^{q} is an unsubstituted phenyl group, or a phenyl group substituted with a C₁₋₂₀ alkyl group or a C₁₋₂₀ alkoxy group is preferable, a compound in which each of R^{p} and R^{q} is an unsubstituted phenyl group, or a phenyl group substituted with a C₁₋₂₀ alkoxy group is more preferable, and a compound in which each of R^{p} and R^{q} is an unsubstituted phenyl group, or a phenyl group substituted with a C₁₋₁₀ alkoxy group is particularly preferable.

In General Formula (I₁), R^{g} represents a hydrogen atom or an electron withdrawing group. In addition, in General Formula (I₃), each of R^{r} and R^{s} independently represents a hydrogen atom or an electron withdrawing group. Examples of the electron withdrawing group include a methyl halide group such as a trifluoromethyl group; a nitro group; a cyano group; an aryl group; a heteroaryl group; an alkynyl group; an alkenyl group; a substituent having a carbonyl group such as a carboxyl group, an acyl group, a carbonyloxy group, an amide group, and an aldehyde group; a sulfoxide group; a sulfonyl group; an alkoxymethyl group; and an aminomethyl group, and an aryl group or a heteroaryl group having the electron withdrawing group as a substituent can also be used. Among these electron withdrawing groups, from the viewpoint of making the maximum fluorescence wavelength to be longer, a trifluoromethyl group, a nitro group, a cyano group, a phenyl group, or a sulfonyl group which can function as a strong electron withdrawing group is preferable.

As the near infrared fluorescent material according to the present specification, a compound represented by the following General Formula (I₁-0) or (I₂-0) is preferable. A compound having a boron dipyrromethene skeleton is preferably since the maximum fluorescence wavelength becomes a longer wavelength, and, in particular, a compound satisfying the following (p2), (p3), (q2), and (q3), in which the pyrrole ring is condensed with an aromatic ring or a heteroaromatic ring is preferable as the near infrared fluorescent material since the maximum wavelength becomes a longer wavelength.

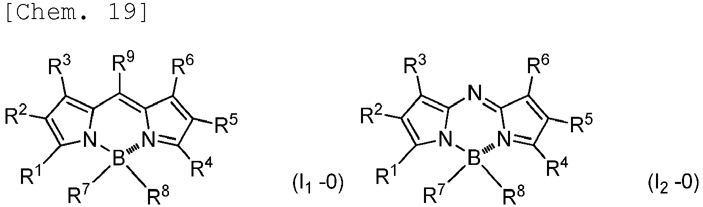

In General Formula (I₁-0) or (I₂-0), R¹, R², and R³ satisfy any one of the following (p1) to (p3).
(p1) each of R¹, R², and R³ independently represents a hydrogen atom, a halogen atom, a C₁₋₂₀ alkyl group, a C₁₋₂₀ alkoxy group, an aryl group, or a heteroaryl group,
(p2) R¹ and R² together form an aromatic 5-membered ring or an aromatic 6-membered ring, and R³ represents a hydrogen atom, a halogen atom, a C₁₋₂₀ alkyl group, a C₁₋₂₀ alkoxy group, an aryl group, or a heteroaryl group, or
(p3) R² and R³ together form an aromatic 5-membered ring or an aromatic 6-membered ring, and R¹ represents a hydrogen atom, a halogen atom, a C₁₋₂₀ alkyl group, a C₁₋₂₀ alkoxy group, an aryl group, or a heteroaryl group.

In General Formula (I₁-0) or (I₂-0), R⁴, R⁵, and R⁶ satisfy any one of the following (q1) to (q3).
(q1) each of R⁴, R⁵, and R⁶ independently represents a hydrogen atom, a halogen atom, a C₁₋₂₀ alkyl group, a C₁₋₂₀ alkoxy group, an aryl group, or a heteroaryl group,
(q2) R⁴ and R⁵ together form an aromatic 5-membered ring or an aromatic 6-membered ring, and R⁶ represents a hydrogen atom, a halogen atom, a C₁₋₂₀ alkyl group, a C₁₋₂₀ alkoxy group, an aryl group, or a heteroaryl group, or
(q3) R⁵ and R⁶ together form an aromatic 5-membered ring or an aromatic 6-membered ring, and R⁴ represents a hydrogen atom, a halogen atom, a C₁₋₂₀ alkyl group, a C₁₋₂₀ alkoxy group, an aryl group, or a heteroaryl group.

As the halogen atom, the C₁₋₂₀ alkyl group, the C₁₋₂₀ alkoxy group, the aryl group, or the heteroaryl group in (p1) to (p3) or (q1) to (q3), those exemplified as "any group which does not inhibit fluorescence of a compound" represented by each of R^{a} and R^{b} can be used.

In (p2) and (p3) or (q2) and (q3), as an aromatic 5-membered ring or an aromatic 6-membered ring which R¹ and R² together form, an aromatic 5-membered ring or an aromatic 6-membered ring which R⁴ and R⁵ together form, an aromatic 5-membered ring or an aromatic 6-membered ring which R² and R³ together form, or an aromatic 5-membered ring or an aromatic 6-membered ring which R⁵ and R⁶ together form, a ring represented by any one of the following General Formulas (C-1) to (C-9) is preferable, and a ring represented by any one of the following General Formulas (C-1), (C-2), and (C-9) is more preferable. In the following General Formulas (C-1) to (C-9), the place to which an asterisk is attached is a portion to which a boron dipyrromethene skeleton in General Formula (I₁-0) or (I₂-0) is bonded.

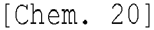

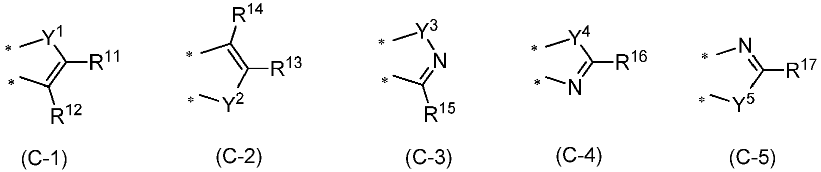

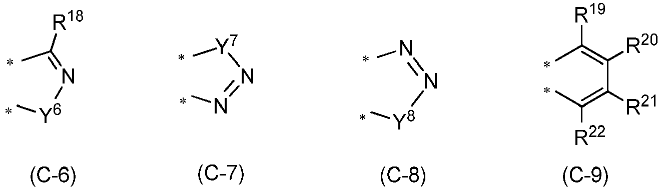

In General Formulas (C-1) to (C-8), each of Y¹ to Y⁸ independently represents a sulfur atom, an oxygen atom, a nitrogen atom, or a phosphorus atom. Each of Y¹ to Y⁸ is independently preferably a sulfur atom, an oxygen atom, or a nitrogen atom, and more preferably a sulfur atom or an oxygen atom.

In General Formulas (C-1) to (C-9), each of R¹¹ to R²² independently represents a hydrogen atom or any group which does not inhibit fluorescence of a compound described above. As "any group which does not inhibit fluorescence of a compound", those exemplified as "any group which does not inhibit fluorescence of a compound" represented by each of R^{a} and R^{b} can be used. Each of R¹¹ to R²² is independently preferably a hydrogen atom, an unsubstituted aryl group, an aryl group having a substituent, an unsubstituted heteroaryl group, or a heteroaryl group having a substituent, more preferably a hydrogen atom, an (unsubstituted) phenyl group, a p-methoxyphenyl group, a p-ethoxyphenyl group, a p-dimethylaminophenyl group, a dimethoxyphenyl group, a thienyl group, or a furanyl group, and still more preferably a hydrogen atom, an (unsubstituted) phenyl group, or a p-methoxyphenyl group. Since the electron donicity can be increased and aggregation of a BODIPY skeleton can suppressed by a bulky substituent, the compound is particularly preferably substituted with at least one of the unsubstituted aryl group, the aryl group having a substituent, the unsubstituted heteroaryl group, and the heteroaryl group having a substituent.

In the compound of General Formula (I₁-0) or (I₂-0), R¹ and R⁴, R² and R⁵, and R³ and R⁶ may be different from each other, respectively, but are preferably the same group. That is, in a case where R¹, R², and R³ satisfy (p1), R⁴, R⁵, and R⁶ preferably satisfy (q1), in a case where R¹, R², and R³ satisfy (p2), R⁴, R⁵, and R⁶ preferably satisfy (q2), and in a case where R¹, R², and R³ satisfy (p3), R⁴, R⁵, and R⁶ preferably satisfy (q3) .

As the compound of General Formula (I₁-0) or (I₂-0), a compound in which R¹ and R² form a ring, and R⁴ and R⁵ form a ring, or R² and R³ form a ring, and R⁵ and R⁶ form a ring is preferable. That is, it is preferable that R¹, R², and R³ satisfy (p2) or (p3), and R⁴, R⁵, and R⁶ satisfy (q2) or (q3) . This is because the maximum fluorescence wavelength becomes a longer wavelength side by further condensation of the aromatic ring or the heteroaromatic ring with a boron dipyrromethene skeleton.

In General Formula (I₁-0) or (I₂-0), each of R⁷ and R⁸ represents a halogen atom or an oxygen atom. In a case where R⁷ and R⁸ are oxygen atoms, R⁷, the boron atom bonded to R⁷, the nitrogen atom bonded to the boron atom, R¹, and the carbon atom bonded to R¹ may together form a ring, and R⁸, the boron atom bonded to R⁸, the nitrogen atom bonded to the boron atom, R⁴, and the carbon atom bonded to R⁴ may together form a ring. That is, each of the ring which R⁷, a boron atom, R¹, and the like form and the ring which R⁸, a boron atom, R⁴, and the like form is condensed with a boron dipyrromethene skeleton. Each of the ring which R⁷, a boron atom, R¹, and the like form and the ring which R⁸, a boron atom, R⁴, and the like form is preferably a 6-membered ring.

In General Formula (I₁-0) or (I₂-0), in a case where R⁷ is an oxygen atom and does not form a ring, R⁷ is an oxygen atom having a substituent (an oxygen atom bonded to a substituent) . Examples of the substituent include a C₁₋₂₀ alkyl group, an aryl group, or a heteroaryl group. Similarly, in General Formula (I₁-0) or (I₂-0), in a case where R⁸ is an oxygen atom and does not form a ring, R⁸ is an oxygen atom having a substituent (an oxygen atom bonded to a substituent). Examples of the substituent include a C₁₋₂₀ alkyl group, an aryl group, or a heteroaryl group. Moreover, in a case where both of R⁷ and R⁸ are oxygen atoms having a substituent, the substituent which R⁷ has and the substituent which R⁸ has may be the same as or different from each other.

In General Formula (I₁-0), R⁹ represents a hydrogen atom or an electron withdrawing group. Examples of the electron withdrawing group include the same as the groups exemplified as R^{g}. Among these, from the viewpoint of making the maximum fluorescence wavelength to be longer, a fluoroalkyl group, a nitro group, a cyano group, an aryl group, or a sulfonyl group which can function as a strong electron withdrawing group is preferable, a trifluoromethyl group, a nitro group, a cyano group, a phenyl group, or a sulfonyl group is more preferable, and from the viewpoint of safety with respect to a living body, a trifluoromethyl group, a cyano group, a phenyl group, or a sulfonyl group is still more preferable. However, the present specification is not limited to these substituents.

As the BODIPY material used in the present specification, among the compounds represented by General Formula (I₁-0) or (I₂-0), a compound in which R¹ and R² together form a ring in which, in the ring represented by General Formula (C-1), one of R¹¹ and R¹² is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R⁴ and R⁵ together form the same type of ring as the ring formed by R¹ and R², R³ and R⁶ are hydrogen atoms, and R⁷ and R⁸ are halogen atoms; a compound in which R¹ and R² together form a ring in which, in the ring represented by General Formula (C-2), one of R¹³ and R¹⁴ is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R⁴ and R⁵ together form the same type of ring as the ring formed by R¹ and R², R³ and R⁶ are hydrogen atoms, and R⁷ and R⁸ are halogen atoms; a compound in which R² and R³ together form a ring in which, in the ring represented by General Formula (C-1), one of R¹¹ and R¹² is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R⁵ and R⁶ together form the same type of ring as the ring formed by R² and R³, R¹ and R⁴ are hydrogen atoms, and R⁷ and R⁸ are halogen atoms; a compound in which R² and R³ together form a ring in which, in the ring represented by the following General Formula (C-2), one of R¹³ and R¹⁴ is a hydrogen atom, and the remaining one is a phenyl group, a thienyl group, or a furanyl group in which one to three hydrogen atoms may be substituted with halogen atoms, C₁₋₂₀ alkyl groups, or C₁₋₂₀ alkoxy groups, R⁵ and R⁶ together form the same type of ring as the ring formed by R² and R³, R¹ and R⁴ are hydrogen atoms, and R⁷ and R⁸ are halogen atoms; or a compound in which R² and R³ together form a ring in which, in the ring represented by the following General Formula (C-9), one of R¹⁹ and R²² is a phenyl group, a thienyl group, or a furanyl group in which one to three hydrogen atoms may be substituted with halogen atoms, C₁₋₂₀ alkyl groups, or C₁₋₂₀ alkoxy groups, and the remaining three are hydrogen atoms, R⁵ and R⁶ together form the same type of ring as the ring formed by R² and R³, R¹ and R⁴ are phenyl groups, thienyl groups, or furanyl groups in which may be substituted with hydrogen atoms, halogen atoms, C₁₋₂₀ alkyl groups, or C₁₋₂₀ alkoxy groups, and R⁷ and R⁸ are halogen atoms is preferable. In a case where the compound is a compound represented by General Formula (I₁-0), R⁹ is more preferably a trifluoromethyl group, a cyano group, a nitro group, or a phenyl group, and a trifluoromethyl group or a phenyl group is particularly preferable.

Examples of a preferable compound of the near infrared fluorescent material according to the present specification include compounds represented by the following General Formulas (I₁-1), (I₁-2), (I₁-3), (I₂-1), (I₂-2), or (I₂-3). In the following General Formula (I₁-1), each of R¹, R³, R⁴, and R⁶ to R⁸ has the same meaning as that described above, ED represents an electron donating group, EW represents an electron withdrawing group, and each of Z¹ to Z⁴ ring independently represents a 5- or 6-membered ring aryl group or a 5- or 6-membered ring heteroaryl group.

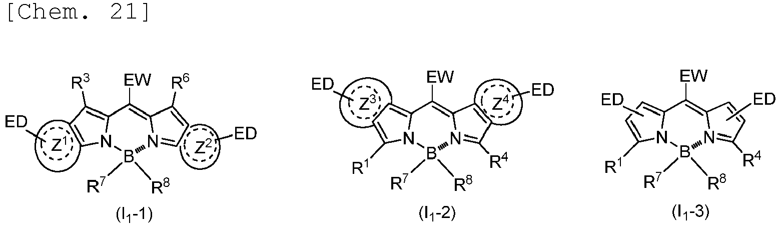

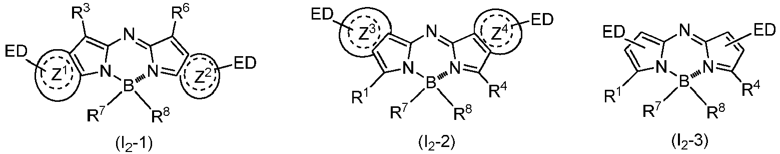

The following General Formula (I₁-1) is preferably a compound represented by each of the following General Formulas (I₁-1-1) to (I₁-1-6), the following General Formula (I₁-2) is preferably a compound represented by each of the following General Formulas (I₁-2-1) to (I₁-2-12), the following General Formula (I₂-1) is preferably a compound represented by each of the following General Formulas (I₂-1-1) to (I₂-1-6), and the following General Formula (I₂-2) is preferably a compound represented by each of the following General Formulas (I₂-2-1) to (I₂-2-12).

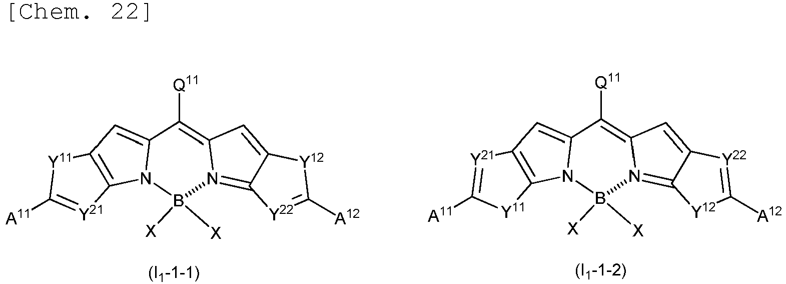

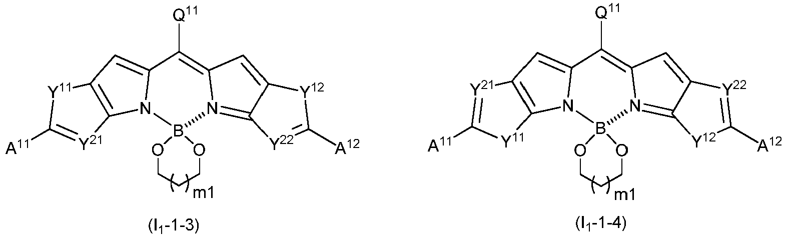

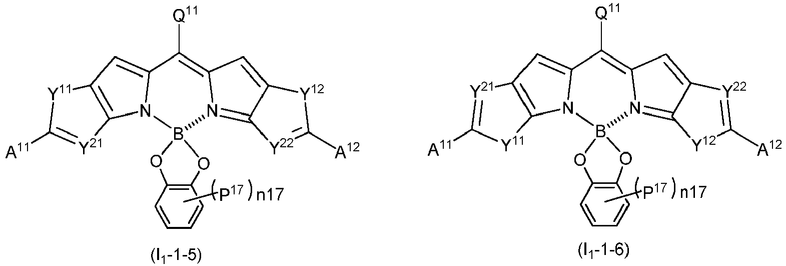

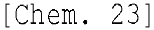

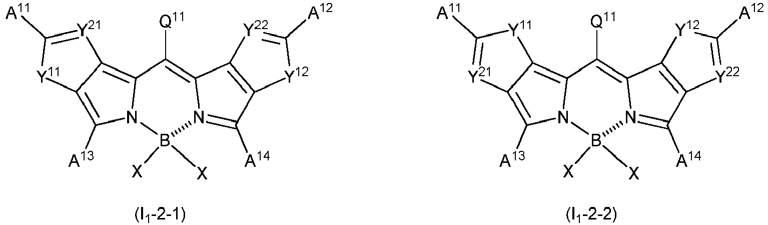

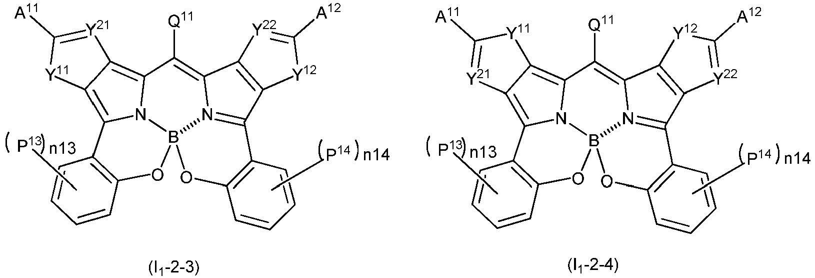

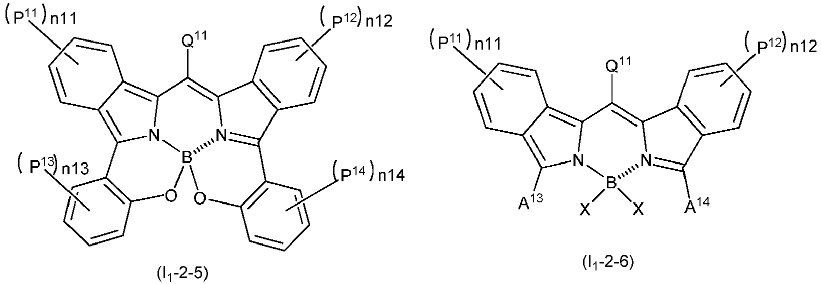

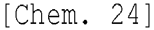

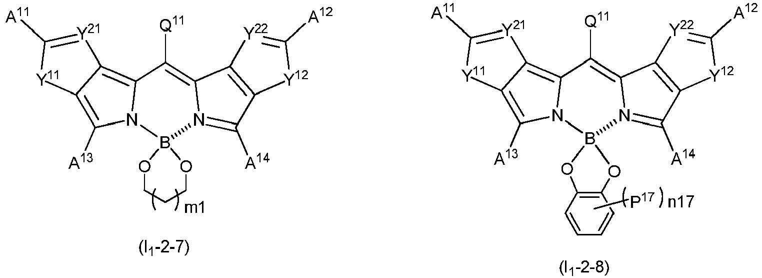

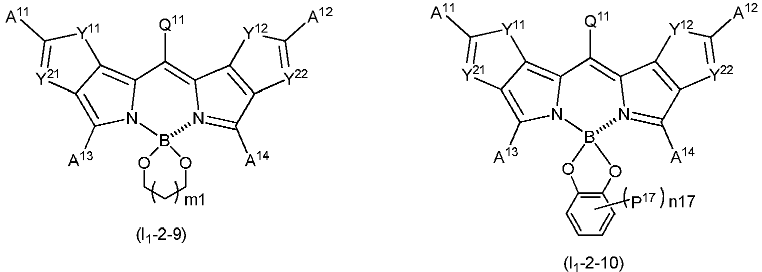

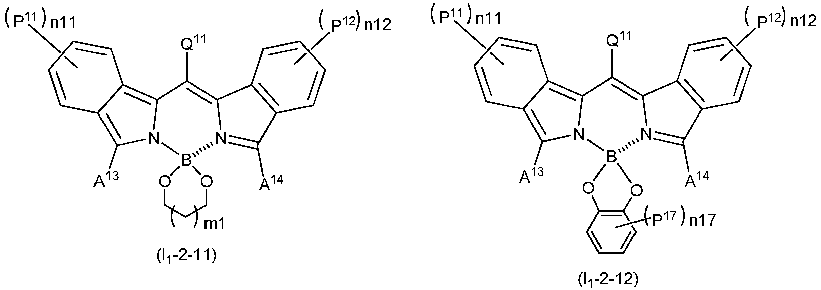

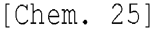

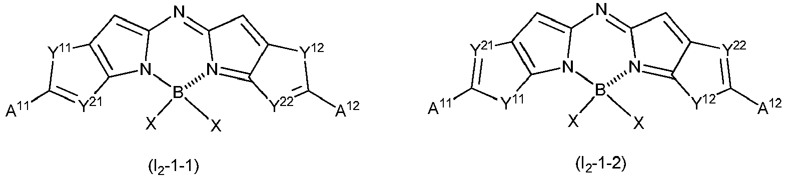

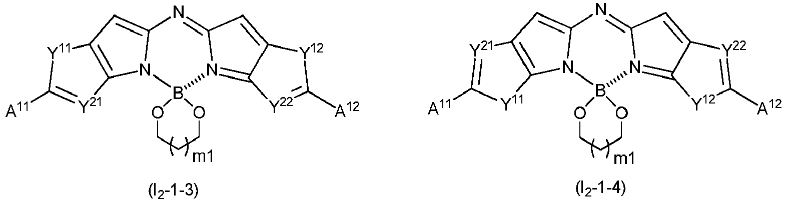

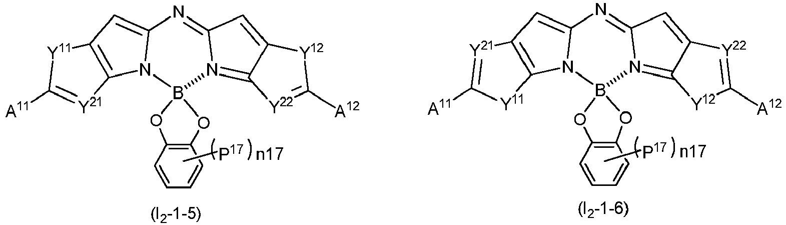

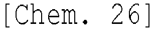

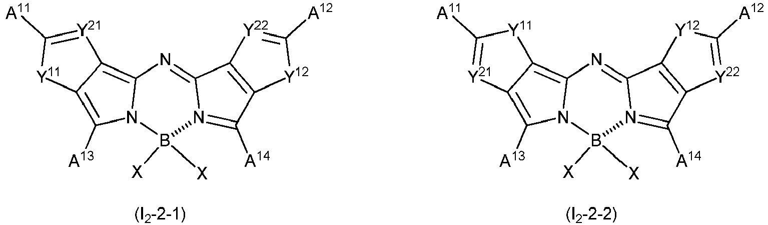

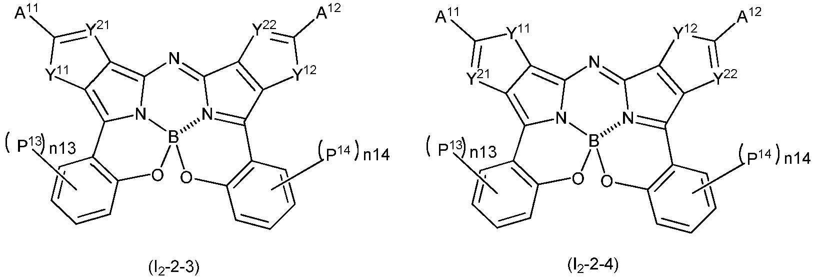

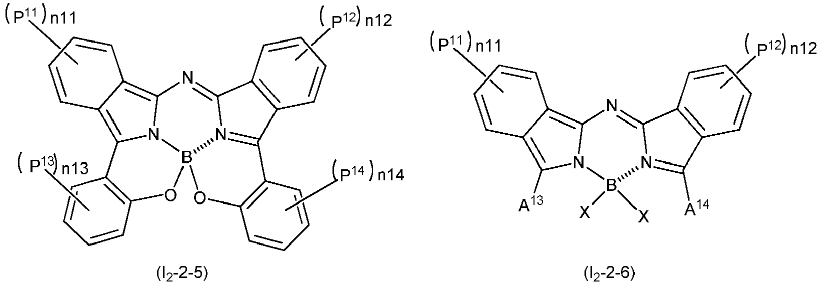

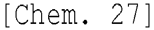

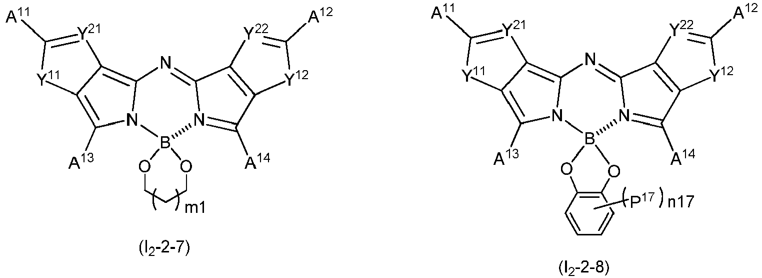

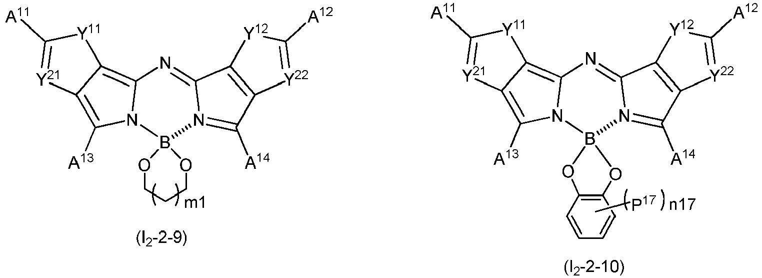

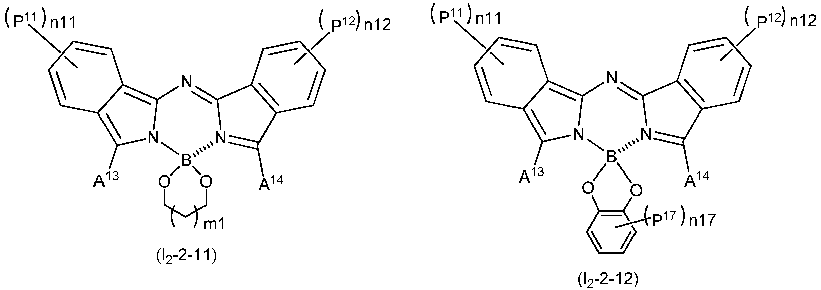

In General Formulas (I₁,-1-1) to (I₁-1-6), (I₁-2-1) to (I₁-2-4), (I₁-2-7) to (I₁-2-10), (I₂-1-1) to (I₂-1-6), (I₂-2-1) to (I₂-2-4), and (I₂-2-7) to (I₂-2-10), each of Y¹¹ and Y¹² independently represents an oxygen atom or a sulfur atom, and each of Y²¹ and Y²² independently represents a carbon atom or a nitrogen atom. As the compounds represented by General Formulas (I₁-1-1) or the like, a compound in which Y¹¹ and Y¹² are the same type of atoms and Y²¹ and Y²² are the same type of atoms is preferable.

In General Formulas (I₁-1-1) to (I₁-1-6) and (I₁-2-1) to (I₁-2-12), Q¹¹ represents a hydrogen atom or an electron withdrawing group. Examples of the electron withdrawing group include the same as the groups exemplified as R^{g}. As the composition represented by General Formula (I₁-1-1), a compound in which Q¹¹ is a trifluoromethyl group, a cyano group, a nitro group, or a phenyl group which may have a substituent is preferable, and a compound in which Q¹¹ is a trifluoromethyl group or a phenyl group which may have a substituent is more preferable.

In General Formulas (I₁-1-1) and (I₁-1-2), (I₁-2-1), (I₁-2-2), and (I₁-2-6), (I₂-1-1) and (I₂-1-2), and (I₂-2-1), (I₂-2-2), and (I₂-2-6) each of X's independently represents a halogen atom, a C₁₋₂₀ alkoxy group, an aryloxy group, or an acyloxy group.

In a case where X is a C₁₋₂₀ alkoxy group, the alkyl group portion of the alkoxy group may be linear, branched, or cyclic (aliphatic cyclic group). Examples of the alkoxy group include a methoxy group, an ethoxy group, a propyloxy group, an isopropyloxy group, an n-butyloxy group, an isobutyloxy group, a t-butyloxy group, a pentyloxy group, an isoamyloxy group, a hexyloxy group, a heptyloxy group, an octyloxy group, a nonyloxy group, a decyloxy group, a undecyloxy group, and a dodecyloxy group.

In a case where X is an aryloxy group, examples of the aryloxy group include a phenyloxy group, a naphthyloxy group, an indenyloxy group, and a biphenyloxy group.

In a case where X is an acyloxy group, as the acyloxy group, an alkylcarbonyloxy group or an arylcarbonyl group is preferable. Examples of the alkylcarbonyloxy group include a methylcarbonyloxy group (acetoxy group), an ethylcarbonyloxy group, a propylcarbonyloxy group, an isopropylcarbonyloxy group, an n-butylcarbonyloxy group, an isobutylcarbonyloxy group, a t-butylcarbonyloxy group, a pentylcarbonyloxy group, an isoamylcarbonyloxy group, a hexylcarbonyloxy group, a heptylcarbonyloxy group, an octylcarbonyloxy group, a nonylcarbonyloxy group, a decylcarbonyloxy group, a undecylcarbonyloxy group, and a dodecylcarbonyloxy group. Examples of the arylcarbonyloxy group include a phenylcarbonyloxy group (benzoyloxy group), a naphthylcarbonyloxy group, an indenylcarbonyloxy group, and a biphenylcarbonyloxy group.

As a compound represented by any one of General Formulas (I₁-1-1), (I₁-1-2), (I₂-2-1), (I₁-2-2), (I₁-2-6), (I₂-1-1), (I₂-1-2), (I₂-2-1), (I₂-2-2), and (I₂-2-6), a compound in which all X's are halogen atoms is preferable, and a compound in which all X's are fluorine atoms is particularly preferable.

In General Formulas (I₁-1-3), (I₁-1-4), (I₁-2-7), (I₁-2-9), (I₁-2-11), (I₂-1-3), (I₂-1-4), (I₂-2-7), (I₂-2-9), and (I₂-2-11), m1 represents 0 or 1.

In General Formulas (I₁-1-5), (I₁-1-6), (I₁-2-3) to (I₁-2-6), (I₁-2-8), (I₁-2-10), (I₁-2-12), (I₂-1-5), (I₂-1-6), (I₂-2-3) to (I₁-2-6), (I₂-2-8), (I₂-2-10), and (I₂-2-12), each of P¹¹ to P¹⁴ and P¹⁷ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹¹ to P¹⁴, and P¹⁷ include the same as those exemplified as R^{g}, (p1) to (p3), or (q1) to (q3). Each of P¹¹ to P¹⁴, and P¹⁷ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present specification is not limited to these substituents .

In General Formulas (I₁-1-5), (I₁-1-6), (I₁-2-3) to (I₁-2-6), (I₁-2-8), (I₁-2-10) to (I₁-2-12), (I₂-1-5), (I₂-1-6), (I₂-2-3) to (I₁-2-6), (I₂-2-8), and (I₂-2-10) to (I₂-2-12), each of n11 to n14 and n17 independently represents an integer of 0 to 3. In a case where a plurality of P¹¹'s are present in one molecule (that is, in a case where n11 is 2 or 3), all of the plurality of P¹¹'s may be the same type of functional groups, or may be the different types of functional groups. The same applies to P¹² to P¹⁴ and P¹⁷.

In General Formulas (I₁-1-1) to (I₁-1-6), (I₁-2-1) to (I₁-2-4), (I₁-2-6) to (I₁-2-12), (I₂-1-1) to (I₂-1-6), (I₂-2-1) to (I₂-2-4), and (I₂-2-6) to (I₂-2-12), each of A¹¹ to A¹⁴ independently represents a phenyl group which may have one to three substituents selected from the group consisting of a halogen atom, a C₁₋₂₀ alkyl group, a C₁₋₂₀ alkoxy group, an amino group, a monoalkylamino group, and a dialkylamino group, or a heteroaryl group which may have one to three substituents selected from the group consisting of a halogen atom, a C₁₋₂₀ alkyl group, a C₁₋₂₀ alkoxy group, an amino group, a monoalkylamino group, and a dialkylamino group. Examples of the heteroaryl group include the same as those represented by R¹, R^{m}, Rⁿ, or R^{o} in General Formula (I₃), and the heteroaryl group is preferably a thienyl group or a furanyl group. Examples of the C₁₋₂₀ alkyl group, the C₁₋₂₀ alkoxy group, the monoalkylamino group, or the dialkylamino group as the substituent which the phenyl group or the heteroaryl group may have the same as those exemplified as R^{g}, (p1) to (p3), or (q1) to (q3). Each of A¹¹ to A¹⁴ is preferably an unsubstituted phenyl group, a phenyl group having one or two C₁₋₂₀ alkoxy groups as the substituent, or a heteroaryl group, more preferably an unsubstituted phenyl group or a phenyl group having one C₁₋₂₀ alkoxy group as the substituent, still more preferably an unsubstituted phenyl group or a phenyl group having one C₁₋₁₀ alkoxy group as the substituent, and still more preferably an unsubstituted phenyl group or a phenyl group having one C₁₋₆ alkoxy group as the substituent. In addition, the compound represented by General Formula (I₁-1-1) is preferably a compound in which all of A¹¹ to A¹⁴ are the same type of functional groups.

As the near infrared fluorescent material according to the present specification, in particular, a compound represented by any one of the following General Formulas (1-1) to (1-37), (2-1) to (2-7), (3-1) to (3-37), (4-1) to (4-7), (5-1), and (5-2) is preferable, a compound represented by any one of the following General Formulas (1-1) to (1-12), (1-25) to (1-31), (2-1) to (2-7), and (3-25) to (3-31) is more preferable, and a compound represented by any one of the following General Formulas (1-1), (1-3), (1-4), (1-6), (1-25), (1-27), (2-1), (3-1), (3-3), (3-4), (3-6), (3-25), (3-27), and (4-1) is still more preferable.

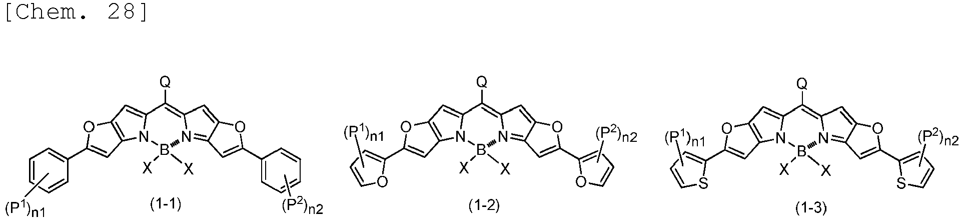

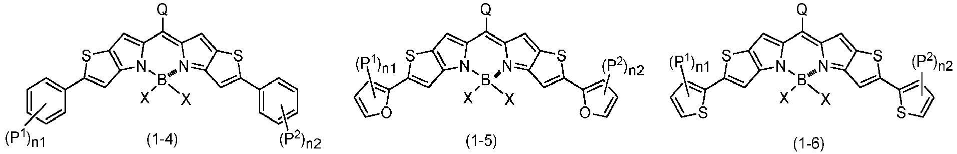

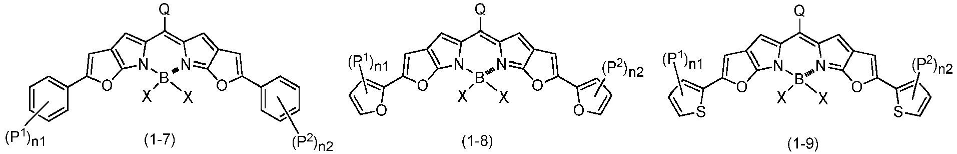

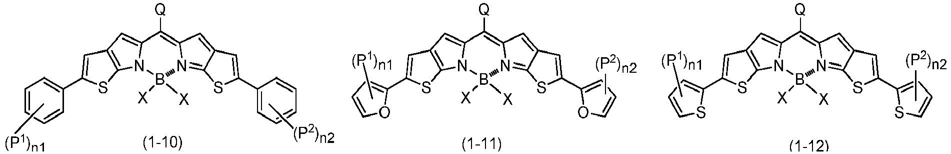

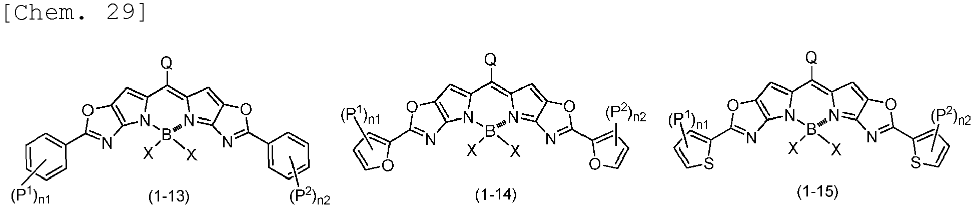

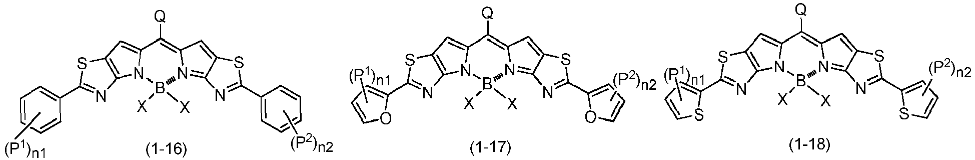

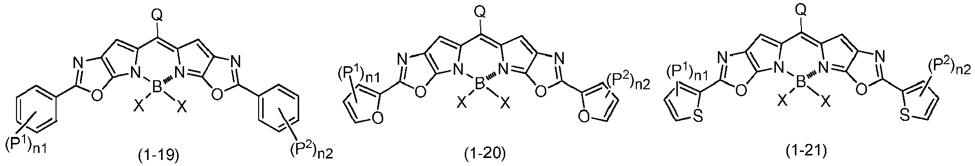

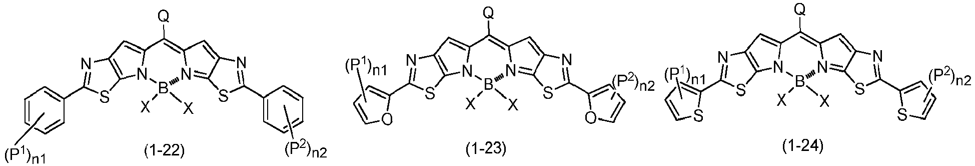

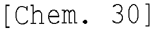

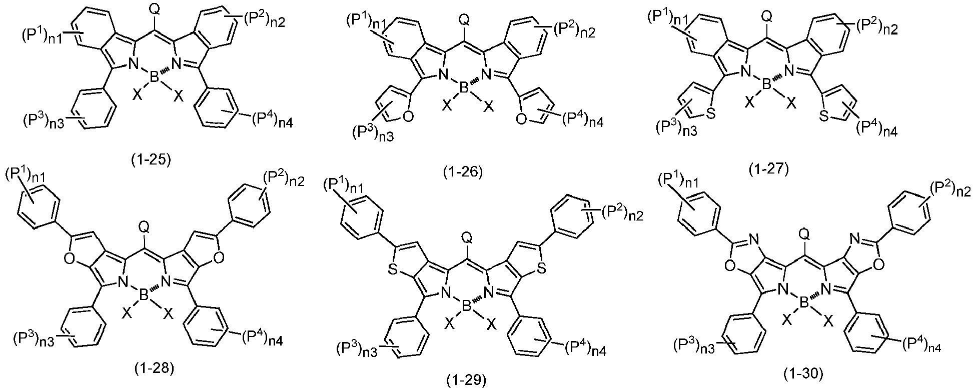

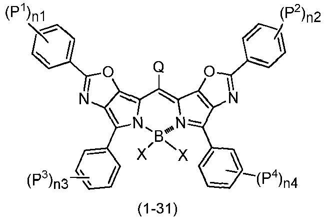

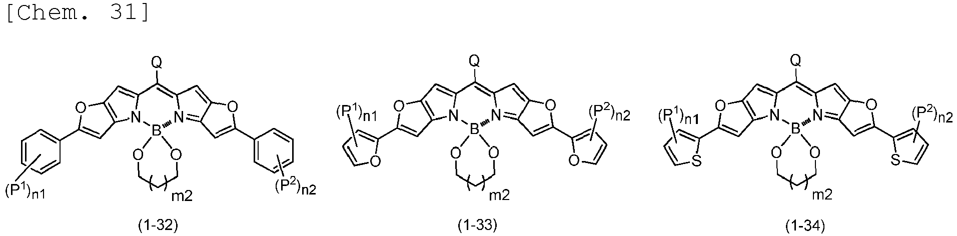

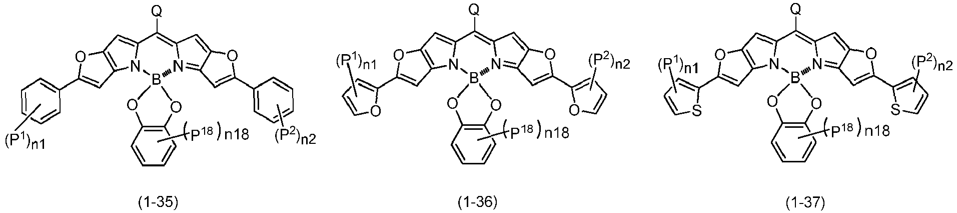

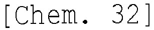

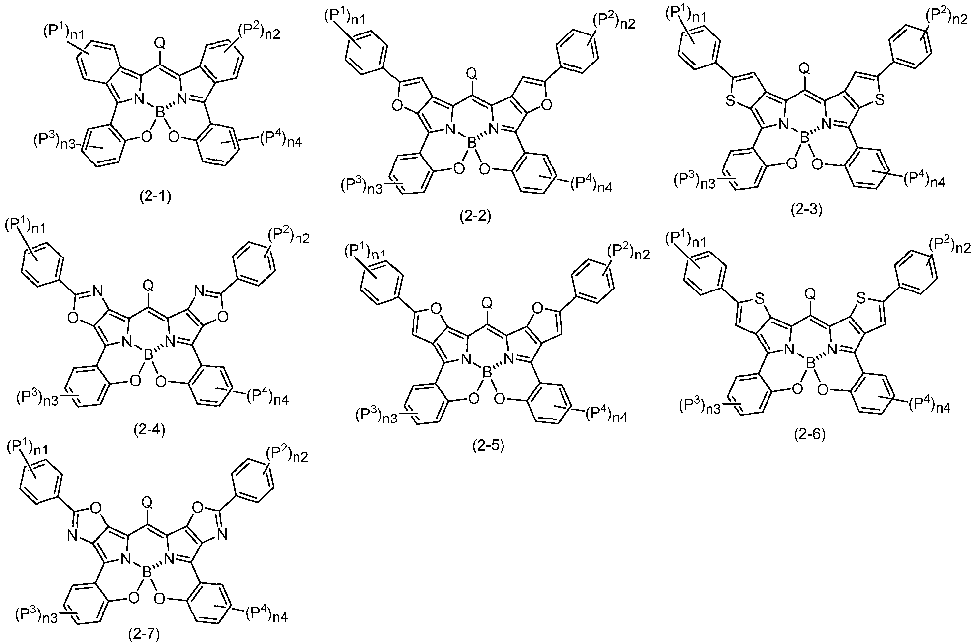

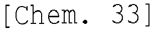

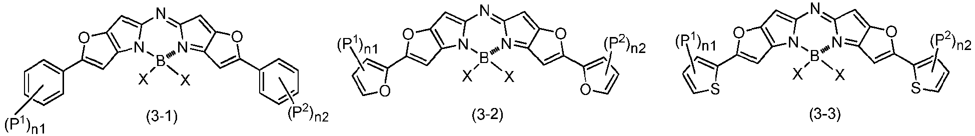

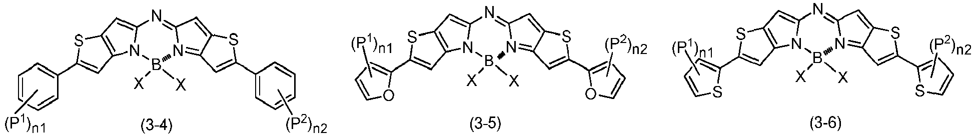

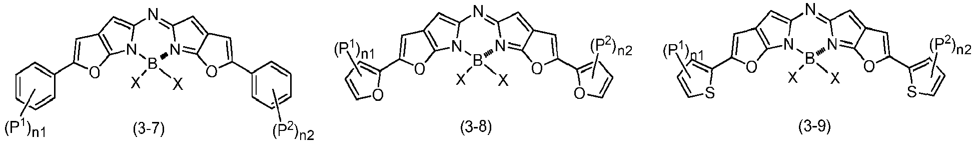

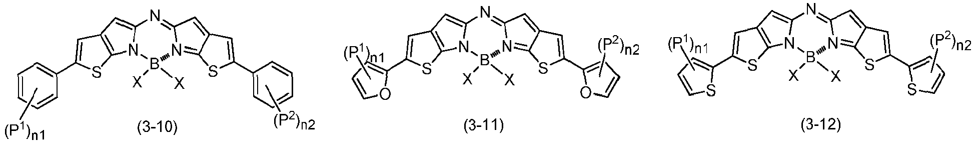

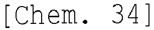

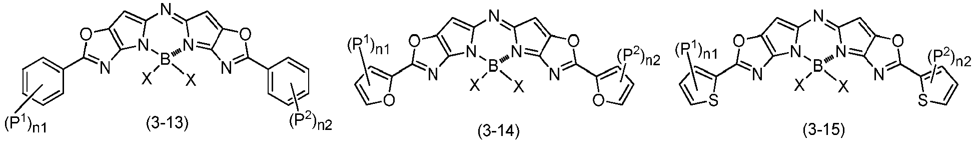

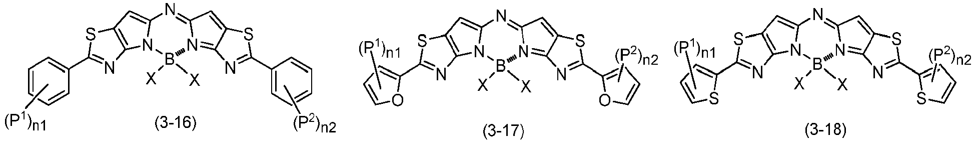

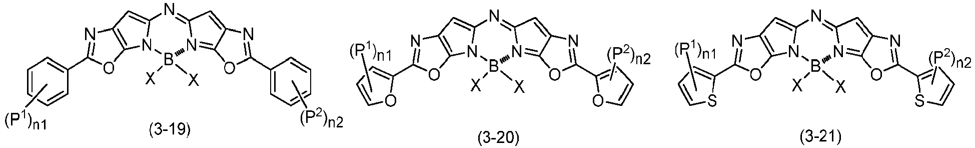

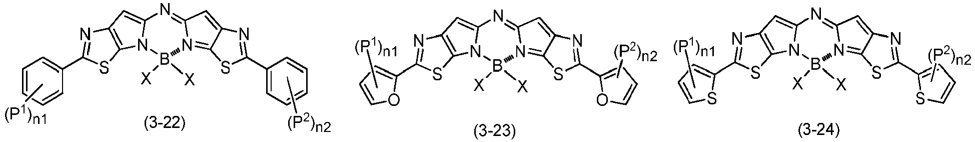

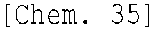

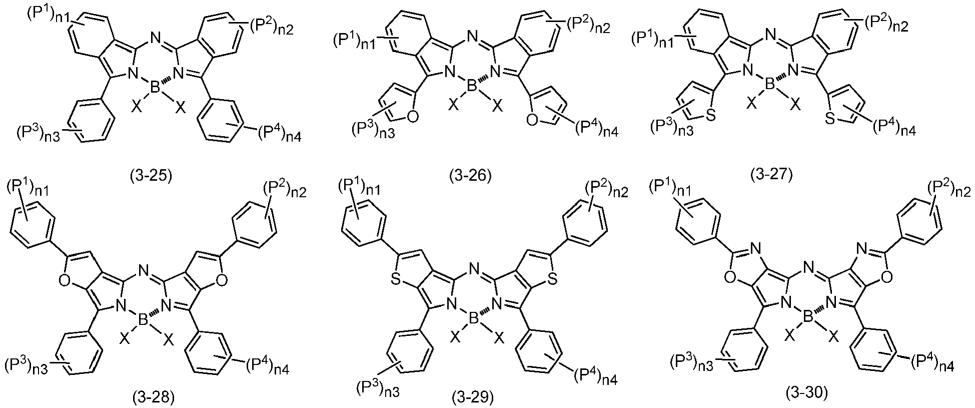

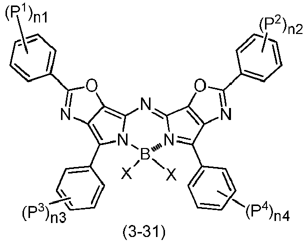

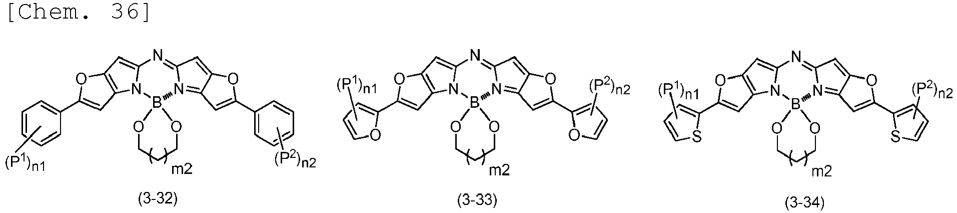

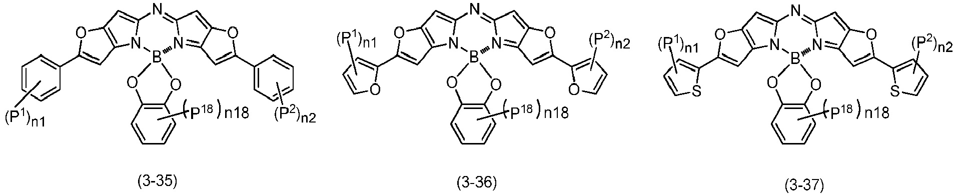

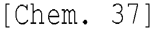

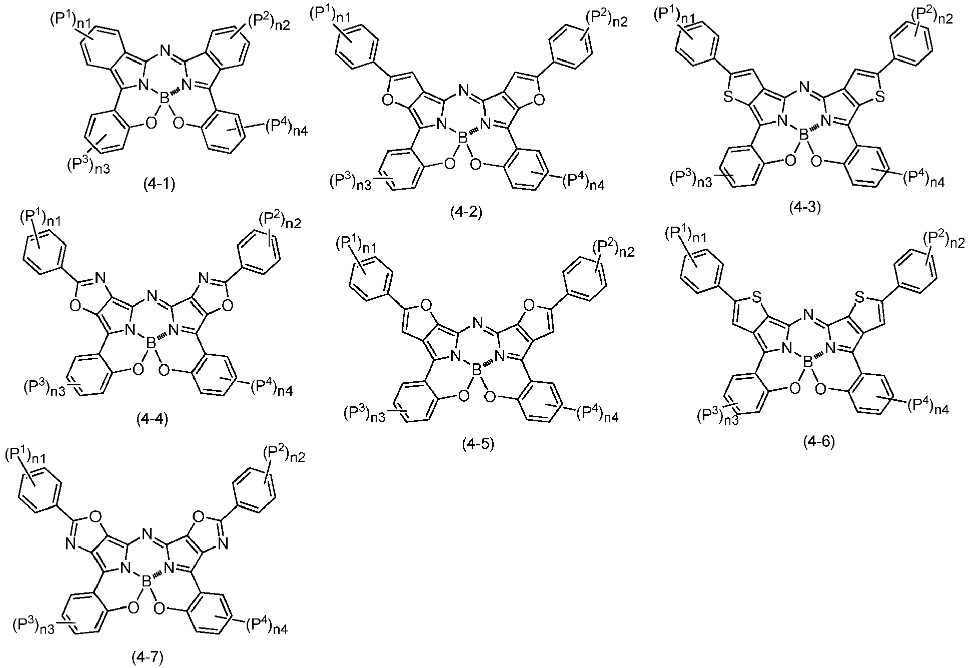

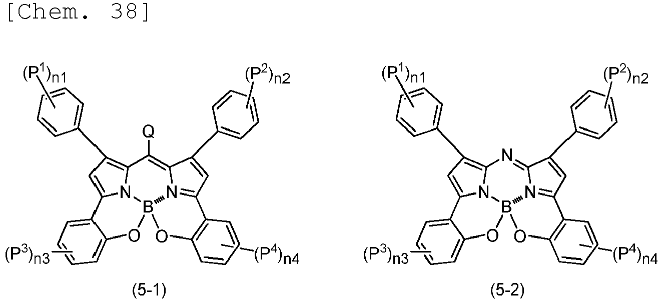

In General Formulas (1-1) to (1-37), (2-1) to (2-7), (3-1) to (3-37), (4-1) to (4-7), (5-1), and (5-2), each of P¹ to P⁴ and P¹⁸ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹ to P⁴ include the same as those exemplified as R^{g}, (p1) to (p3), or (q1) to (q3). Each of P¹ to P⁴ and P¹⁸ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present specification is not limited to these substituents .

In General Formulas (1-1) to (1-37), (2-1) to (2-7), (3-1) to (3-37), (4-1) to (4-7), (5-1), and (5-2), each of n1 to n4 and n18 independently represents an integer of 0 to 3. In a case where a plurality of P¹'s are present in one molecule (that is, in a case where n1 is 2 or 3), all of the plurality of P¹'s may be the same type of functional groups, or may be the different types of functional groups. The same applies to P² to P⁴ and P¹⁸.

In General Formulas (1-1) to (1-37), (2-1) to (2-7) and (5-1), Q represents a trifluoromethyl group, a cyano group, a nitro group, or a phenyl group which may have a substituent, preferably a trifluoromethyl group or a phenyl group which may have a substituent, and more preferably a trifluoromethyl group or an unsubstituted phenyl group. Examples of the substituent which the phenyl group may have a halogen atom, a C₁₋₂₀ alkyl group, a C₁₋₂₀ alkoxy group, an amino group, a monoalkylamino group, and a dialkylamino group.

In General Formula (1-1) to (1-31), and (3-1) to (3-31), X is the same as in General Formulas (I₁-1-1) and the like. As the compound represented by General Formula (1-1) or the like, a compound in which X is a halogen atom is preferable, and a compound in which X is a fluorine atom is particularly preferable.

In General Formulas (1-32) to (1-34) and (3-32) to (3-34), m2 is 0 or 1. As the compound represented by General Formula (1-32) or the like, a compound in which m2 is 1 is preferable.

The compound represented by General Formula (1-1) to (1-37), (2-1) to (2-7), or (5-1), a compound in which each of P¹ to P⁴ and P¹⁸ is independently a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, each of n1 to n4 and n18 is independently 0 to 2, and Q is a trifluoromethyl group or a phenyl group is preferable. Similarly, the compound represented by General Formula (3-1) to (3-37), (4-1) to (4-7), or (5-2), a compound in which each of P¹ to P⁴ and P¹⁸ is independently a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, and each of n1 to n4 and n18 is independently 0 to 2 is preferable.

The near infrared fluorescent material according to the present invention is a compound represented by any one of the following General Formulas (I₃-1) to (I₃-6) or a compound represented by any one of General Formulas (I₄-1) to (I₄-6), which is preferable since the maximum fluorescent wavelength is a longer wavelength.

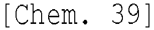

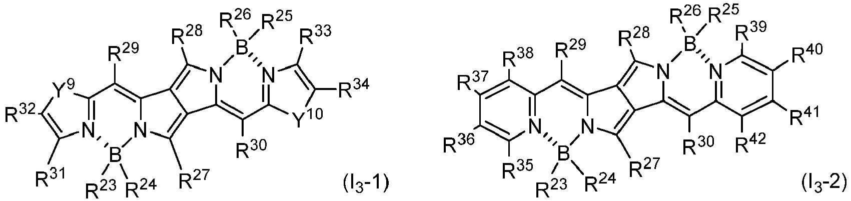

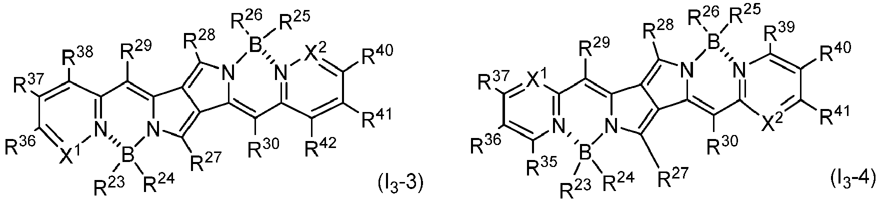

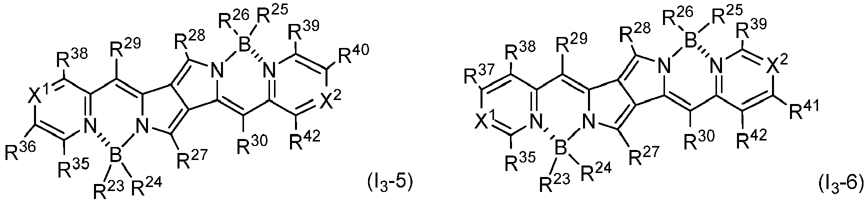

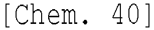

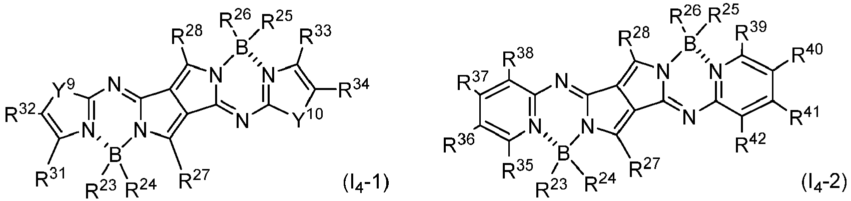

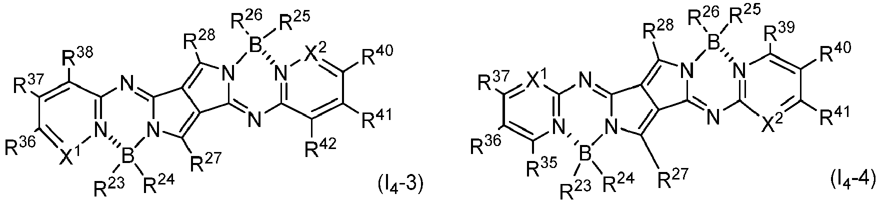

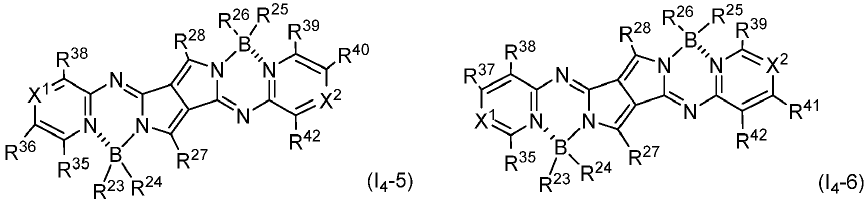

In General Formulas (I₃-1) to (I₃-6) and (I₄-1) to (I₄-6), each of R²³, R²⁴, R²⁵, and R²⁶ independently represents a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by each of R²³, R²⁴, R²⁵, and R²⁶ include the same as those represented by each of R^{l}, R^{m}, Rⁿ, and R^{o} in General Formula (I₃). As the compound represented by any one of General Formulas (I₃-1) to (I₃-6) or the compound represented by any one of General Formulas (I₄-1) to (I₄-6), from the viewpoint of high thermal stability of a compound, a compound in which each of R²³, R²⁴, R²⁵, and R²⁶ is a halogen atom, an unsubstituted aryl group, or an aryl group having a substituent is preferable, specifically, a compound in which each of R²³, R²⁴, R²⁵, and R²⁶ is a fluorine atom, a chlorine atom, a bromine atom, an unsubstituted phenyl group, or a phenyl group substituted with a C₁₋₂₀ alkyl group or a C₁₋₂₀ alkoxy group is preferable, a compound in which each of R²³, R²⁴, R²⁵, and R²⁶ is a fluorine atom, a chlorine atom, an unsubstituted phenyl group, or a phenyl group substituted with a C₁₋₁₀ alkyl or a C₁₋₁₀ alkoxy group is more preferable, and from the viewpoint of obtaining a compound having both high light emitting efficiency and thermal stability, a compound in which each of R²³, R²⁴, R²⁵, and R²⁶ is a fluorine atom or an unsubstituted phenyl group is particularly preferable.

In General Formulas (I₃-1) to (I₃-6) and (I₄-1) to (I₄-6), each of R²⁷ and R²⁸ independently represents a hydrogen atom, a halogen atom, a C₁₋₂₀ alkyl group, a C₁₋₂₀ alkoxy group, an aryl group, or a heteroaryl group. Examples of the halogen atoms, the C₁₋₂₀ alkyl group, the C₁₋₂₀ alkoxy group, the aryl group, or the heteroaryl group represented by R²⁷ or R²⁸ include the same as those represented by R^{p} or R^{q} in General Formula (I₃). As the compound represented by any one of General Formulas (I₃-1) to (I₃-6) or the compound represented by any one of General Formulas (I₄-1) to (I₄-6), a compound in which each of R²⁷ and R²⁸ is a hydrogen atom or an aryl group is preferable, from the viewpoint of obtaining a compound having high light emitting efficiency, a compound in which each of R²⁷ and R²⁸ is a hydrogen atom, an unsubstituted phenyl group, or a phenyl group substituted with a C₁₋₂₀ alkyl group or a C₁₋₂₀ alkoxy group is preferable, a compound in which each of R²⁷ and R²⁸ is a hydrogen atom, an unsubstituted phenyl group, or a phenyl group substituted with a linear or branched C₁₋₂₀ alkoxy group is more preferable, and from the viewpoint of obtaining a compound having high light emitting efficiency and excellent compatibility with respect to a resin, a compound in which each of R²⁷ and R²⁸ is an unsubstituted phenyl group, or a phenyl group substituted with a linear or branched C₁₋₁₀ alkoxy group is particularly preferable.

In General Formulas (I₃-1) to (I₃-6), each of R²⁹ and R³⁰ independently represents a hydrogen atom or an electron withdrawing group. The electron withdrawing group represented by R²⁹ or R³⁰ is selected from a methyl halide group such as a trifluoromethyl group; a nitro group; a cyano group; an aryl group; a heteroaryl group; an alkynyl group; an alkenyl group; a substituent having a carbonyl group such as a carboxyl group, an acyl group, a carbonyloxy group, an amide group, and an aldehyde group; a sulfoxide group; a sulfonyl group; an alkoxymethyl group; and an aminomethyl group. As the compound represented by any one of General Formulas (I₃-1) to (I₃-6), from the viewpoint of obtaining a compound having high light emitting efficiency or having longer fluorescence wavelength, a compound in which each of R²⁹ and R³⁰ is a fluoroalkyl group, a nitro group, a cyano group, or an aryl group which can function as a strong electron withdrawing group is preferable, a compound in which each of R²⁹ and R³⁰ is a trifluoromethyl group, a nitro group, a cyano group, or a phenyl group which may have a substituent is more preferable, and from the viewpoint of obtaining a compound having high light emitting efficiency and excellent compatibility with respect to a resin, a compound in which each of R²⁹ and R³⁰ is a trifluoromethyl group or a cyano group is still more preferable.

In General Formulas (I₃-1) and (I₄-1), each of Y⁹ and Y¹⁰ independently represents a sulfur atom, an oxygen atom, a nitrogen atom, or a phosphorus atom. The compound represented by General Formulas (I₃-1) or (I₄-1), from the viewpoint of obtaining a compound having high light emitting efficiency, a compound in which each of Y⁹ and Y¹⁰ is independently a sulfur atom, an oxygen atom, or a nitrogen atom is preferable, a compound in which each of Y⁹ and Y¹⁰ is independently a sulfur atom or an oxygen atom is more preferable, and from the viewpoint of obtaining a compound having both high light emitting efficiency and thermal stability, a compound in which Y⁹ and Y¹⁰ together are sulfur atoms or oxygen atoms is still more preferable.

In General Formulas (I₃-3) to (I₃-6) and (I₄-3) to (I₄-6), each of X¹ and X² independently represents a nitrogen atom or a phosphorus atom. The compound represented by General Formulas (I₃-3) to (I₃-6) or (I₄-3) to (I₄-6), from the viewpoint of obtaining a compound having high light emitting efficiency, a compound in which X¹ and X² together are nitrogen atoms or phosphorus atoms is preferable, and from the viewpoint of obtaining a compound having both high light emitting efficiency and thermal stability, a compound in which X¹ and X² together are nitrogen atoms is more preferable.

In General Formulas (I₃-1) and (I₄-1), R³¹ and R³² satisfy the following (p4) or (p5).

(p4) each of R³¹ and R³² independently represents a hydrogen atom, a halogen atom, a C₁₋₂₀ alkyl group, a C₁₋₂₀ alkoxy group, an aryl group, or a heteroaryl group.

(p5) R³¹ and R³² together form an aromatic 5-membered ring which may have a substituent or an aromatic 6-membered ring which may have a substituent.

In General Formulas (I₃-1) and (I₄-1), R³³ and R³⁴ satisfy the following (q4) or (q5).

(q4) each of R³³ and R³⁴ independently represents a hydrogen atom, a halogen atom, a C₁₋₂₀ alkyl group, a C₁₋₂₀ alkoxy group, an aryl group, or a heteroaryl group, or (q5) R³³ and R³⁴ together form an aromatic 5-membered ring which may have a substituent or an aromatic 6-membered ring which may have a substituent.

In General Formulas (I₃₋2) to (I₃-6) and (I₄-2) to (I₄-6), R³⁵, R³⁶, R³⁷, and R³⁸ satisfy any one of the following (p6) to (p9) .

(p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group.

(p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group.

(p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group.

(p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group.

In General Formulas (I₃-2) to (I₃-6) and (I₄-2) to (I₄-6), R³⁹, R⁴⁰, R⁴¹, and R⁴² satisfy any one of the following (q6) to (q9) .

(q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group.

(q7) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group.

(q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group.

(q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group.

As the halogen atom, the C₁₋₂₀ alkyl group, the C₁₋₂₀ alkoxy group, the aryl group, or the heteroaryl group in (p4), (p6) to (p9), (q4), or (q6) to (q9), those exemplified as "any group which does not inhibit fluorescence of a compound" represented by each of R^{a} and R^{b} can be used.

In (p5), (p7) to (p9), (q5), (q7) to (q9), as an aromatic 5-membered ring or an aromatic 6-membered ring which R³¹ and R³² together form, an aromatic 5-membered ring or an aromatic 6-membered ring which R³³ and R³⁴ together form, an aromatic 5-membered ring or an aromatic 6-membered ring which R³⁵ and R³⁶ together form, an aromatic 5-membered ring or an aromatic 6-membered ring which R³⁶ and R³⁷ together form, an aromatic 5-membered ring or an aromatic 6-membered ring which R³⁷ and R³⁸ together form, an aromatic 5-membered ring or an aromatic 6-membered ring which R³⁹ and R⁴⁰ together form, an aromatic 5-membered ring or an aromatic 6-membered ring which R⁴⁰ and R⁴¹ together form, or an aromatic 5-membered ring or an aromatic 6-membered ring which R⁴¹ and R⁴² together form, the ring represented by any one of General Formulas (C-1) to (C-9) is preferable, and the ring represented by General Formula (C-9) is more preferable since a compound having high thermal stability can be obtained.

As the compound represented by (I₃-1), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; Y⁹ and Y¹⁰ together are sulfur atoms or oxygen atoms; each of R³¹ and R³² is independently a hydrogen atom or a C₁₋₂₀ alkyl group, or R³¹ and R³² together form a phenyl group which may have a substituent; and each R³³ and R³⁴ is independently a hydrogen atom or a C₁₋₂₀ alkyl group or R³³ and R³⁴ together form a phenyl group which may have a substituent is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; Y⁹ and Y¹⁰ together are sulfur atoms or oxygen atoms; each of R³¹ and R³² is independently a hydrogen atom or a C₁₋₂₀ alkyl group or R³¹ and R³² together form an unsubstituted phenyl group or a phenyl group substituted with a C₁₋₁₀ alkyl group; and each R³³ and R³⁴ is independently a hydrogen atom or a C₁₋₂₀ alkyl group, or R³³ and R³⁴ together form a phenyl group substituted with a C₁₋₁₀ alkyl group is more preferable since the light emitting efficiency is high and the compatibility with respect to a resin is excellent.

As the compound represented by (I₃-2), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; each of R³⁵, R³⁶, R³⁷, and R³⁸ is independently a hydrogen atom or a C₁₋₂₀ alkyl group or R³⁵ and R³⁶ together form a phenyl group which may have a substituent, each of R³⁷ and R³⁸ is independently a hydrogen atom or a C₁₋₂₀ alkyl group or R³⁶ and R³⁷ together form a phenyl group which may have a substituent, each of R³⁵ and R³⁸ is independently a hydrogen atom or a C₁₋₂₀ alkyl group or R³⁷ and R³⁸ together form a phenyl group which may have a substituent, and each of R³⁵ and R³⁶ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form a phenyl group which may have a substituent, each of R⁴¹ and R⁴² is independently a hydrogen atom or a C₁₋₂₀ alkyl group or R⁴⁰ and R⁴¹ together form a phenyl group which may have a substituent, each of R³⁹ and R⁴² is independently a hydrogen atom or a C₁₋₂₀ alkyl group, or R⁴¹ and R⁴² together form a phenyl group which may have a substituent, and each of R³⁹ and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; each of R³⁵, R³⁶, R³⁷, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group; each of R³⁷ and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, each of R³⁵ and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and each of R³⁵ and R³⁶ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, each of R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, each of R³⁹ and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and each R³⁹ and R⁴⁰ is independently a hydrogen atom or a C₁₋₂₀ alkyl group is more preferable since the light emitting efficiency is high and the compatibility with respect to a resin is excellent.

As the compound represented by (I₃-3), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁶, R³⁷, and R³⁸ is independently a hydrogen atom or a C₁₋₂₀ alkyl group or R³⁶ and R³⁷ together form a phenyl group which may have a substituent, R³⁸ is a hydrogen atom or a C₁₋₂₀ alkyl group or R³⁷ and R³⁸ together form a phenyl group which may have a substituent, and R³⁶ is a hydrogen atom or a C₁₋₂₀ alkyl group; each of R⁴⁰, R⁴¹, and R⁴² is independently a hydrogen atom or a C₁₋₂₀ alkyl group or R⁴⁰ and R⁴¹ together form a phenyl group which may have a substituent, R⁴² is a hydrogen atom or a C₁₋₂₀ alkyl group or R⁴¹ and R⁴² together form a phenyl group which may have a substituent, and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁶, R³⁷, and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, R³⁸ is a hydrogen atom or a C₁₋₂₀ alkyl group or R³⁷ and R³⁸ together form an unsubstituted phenyl group or a phenyl group substituted with a C₁₋₁₀ alkyl group, and R³⁶ is a hydrogen atom or a C₁₋₂₀ alkyl group; each of R⁴⁰, R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, R⁴² is a hydrogen atom or a C₁₋₂₀ alkyl group or R⁴¹ and R⁴² together form an unsubstituted phenyl group or a phenyl group substituted with a C₁₋₁₀ alkyl group, and R⁴⁰ is a hydrogen atom or a C₁₋₂₀ alkyl group is more preferable since the light emitting efficiency is high and the compatibility with respect to a resin is excellent.

As the compound represented by (I₃-4), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁶, and R³⁷ is independently a hydrogen atom or a C₁₋₂₀ alkyl group or R³⁵ and R³⁶ together form a phenyl group which may have a substituent, R³⁷ is a hydrogen atom or a C₁₋₂₀ alkyl group or R³⁶ and R³⁷ together form a phenyl group which may have a substituent, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form a phenyl group which may have a substituent, R⁴¹ is a hydrogen atom or a C₁₋₂₀ alkyl group or R⁴⁰ and R⁴¹ together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁶, and R³⁷ is independently a hydrogen atom or a C₁₋₂₀ alkyl group or R³⁵ and R³⁶ together form an unsubstituted phenyl group or a phenyl group substituted with a C₁₋₁₀ alkyl group, R³⁷ is a hydrogen atom or a C₁₋₂₀ alkyl group or R³⁶ and R³⁷ together form an unsubstituted phenyl group or a phenyl group substituted with a C₁₋₁₀ alkyl group, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form an unsubstituted phenyl group or a phenyl group substituted with a C₁₋₁₀ alkyl group, R⁴¹ is a hydrogen atom or a C₁₋₂₀ alkyl group or R⁴⁰ and R⁴¹ together form an unsubstituted phenyl group or a phenyl group substituted with a C₁₋₁₀ alkyl group, and R³⁹ is a hydrogen atom or a C₁₋₂₀ alkyl group is more preferable since the light emitting efficiency is high and the compatibility with respect to a resin is excellent.

As the compound represented by (I₃-5), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁶, and R³⁸ is independently a hydrogen atom or a C₁₋₂₀ alkyl group or R³⁵ and R³⁶ together form a phenyl group which may have a substituent, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form a phenyl group which may have a substituent, and R⁴² is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁶, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, and R⁴² is a hydrogen atom or a C₁₋₂₀ alkyl group is more preferable since the light emitting efficiency is high and the compatibility with respect to a resin is excellent.

As the compound represented by (I₃-6), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; R²⁹ and R³⁰ together are trifluoromethyl groups, nitro groups, cyano groups, or phenyl groups; X¹ and X² together are nitrogen atoms; each of R³⁵, R³⁷, and R³⁸ is independently a hydrogen atom or a C₁₋₂₀ alkyl group or R³⁷ and R³⁸ together form a phenyl group which may have a substituent, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; R²⁹ and R³⁰ together are trifluoromethyl groups, nitro groups, or cyano groups; X¹ and X² together are nitrogen atoms; each of R³⁵, R³⁷, and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and R³⁹ is a hydrogen atom or a C₁₋₂₀ alkyl group is more preferable since the light emitting efficiency is high and the compatibility with respect to a resin is excellent.

As the compound represented by (I₄-1), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; Y⁹ and Y¹⁰ together are sulfur atoms or oxygen atoms; each of R³¹ and R³² is independently a hydrogen atom or a C₁₋₂₀ alkyl group, or R³¹ and R³² together form a phenyl group which may have a substituent; and each R³³ and R³⁴ is independently a hydrogen atom or a C₁₋₂₀ alkyl group or R³³ and R³⁴ together form a phenyl group which may have a substituent is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; Y⁹ and Y¹⁰ together are sulfur atoms or oxygen atoms; each of R³¹ and R³² is independently a hydrogen atom or a C₁₋₂₀ alkyl group or R³¹ and R³² together form an unsubstituted phenyl group or a phenyl group substituted with a C₁₋₁₀ alkyl group; and each R³³ and R³⁴ is independently a hydrogen atom or a C₁₋₂₀ alkyl group, or R³³ and R³⁴ together form an unsubstituted phenyl group or a phenyl group substituted with a C₁₋₁₀ alkyl group is more preferable since the light emitting efficiency is high and the compatibility with respect to a resin is excellent.

As the compound represented by (I₄-2), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; each of R³⁵, R³⁶, R³⁷, and R³⁸ is independently a hydrogen atom or a C₁₋₂₀ alkyl group or R³⁵ and R³⁶ together form a phenyl group which may have a substituent, each of R³⁷ and R³⁸ is independently a hydrogen atom or a C₁₋₂₀ alkyl group or R³⁶ and R³⁷ together form a phenyl group which may have a substituent, each of R³⁵ and R³⁶ is independently a hydrogen atom or a C₁₋₂₀ alkyl group or R³⁷ and R³⁸ together form a phenyl group which may have a substituent, and each of R³⁵ and R³⁶ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form a phenyl group which may have a substituent, each of R⁴¹ and R⁴² is independently a hydrogen atom or a C₁₋₂₀ alkyl group or R⁴⁰ and R⁴¹ together form a phenyl group which may have a substituent, each of R³⁹ and R⁴² is independently a hydrogen atom or a C₁₋₂₀ alkyl group, or R⁴¹ and R⁴² together form a phenyl group which may have a substituent, and each of R³⁹ and R⁴² is independently a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; each of R³⁵, R³⁶, R³⁷, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group; each of R³⁷ and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, each of R³⁵ and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and each of R³⁵ and R³⁸ is independently a hydrogen atom or a C₁₋₂₀ alkyl group; each of R³⁹, R⁴⁰, R⁴¹, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, each of R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, each of R³⁹ and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and each R³⁹ and R⁴² is independently a hydrogen atom or a C₁₋₂₀ alkyl group is more preferable since the light emitting efficiency is high and the compatibility with respect to a resin is excellent.

As the compound represented by (I₄-3), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁶, R³⁷, and R³⁸ is independently a hydrogen atom or a C₁₋₂₀ alkyl group or R³⁶ and R³⁷ together form a phenyl group which may have a substituent, R38 is a hydrogen atom or a C₁₋₂₀ alkyl group or R³⁷ and R³⁸ together form a phenyl group which may have a substituent, and R³⁶ is a hydrogen atom or a C₁₋₂₀ alkyl group; each of R⁴⁰, R⁴¹, and R⁴² is independently a hydrogen atom or a C₁₋₂₀ alkyl group or R⁴⁰ and R⁴¹ together form a phenyl group which may have a substituent, R42 is a hydrogen atom or a C₁₋₂₀ alkyl group or R⁴¹ and R⁴² together form a phenyl group which may have a substituent, and R⁴⁰ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; each of R³⁶, R³⁷, and R³⁸ is independently a hydrogen atom or a C₁₋₂₀ alkyl group or R³⁶ and R³⁷ together form an unsubstituted phenyl group or a phenyl group substituted with a C₁₋₁₀ alkyl group, R³⁸ is a hydrogen atom or a C₁₋₂₀ alkyl group or R³⁷ and R³⁸ together form an unsubstituted phenyl group or a phenyl group substituted with a C₁₋₁₀ alkyl group, and R³⁶ is a hydrogen atom or a C₁₋₂₀ alkyl group; each of R⁴⁰, R⁴¹ and R⁴² is independently a hydrogen atom or a C₁₋₂₀ alkyl group or R⁴⁰ and R⁴¹ together form an unsubstituted phenyl group or a phenyl group substituted with a C₁₋₁₀ alkyl group, R⁴² is a hydrogen atom or a C₁₋₂₀ alkyl group or R⁴¹ and R⁴² together form an unsubstituted phenyl group or a phenyl group substituted with a C₁₋₁₀ alkyl group, and R⁴⁰ is a hydrogen atom or a C₁₋₂₀ alkyl group is more preferable since the light emitting efficiency is high and the compatibility with respect to a resin is excellent.

As the compound represented by (I₄-4), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁶, and R³⁷ is independently a hydrogen atom or a C₁₋₂₀ alkyl group or R³⁵ and R³⁶ together form a phenyl group which may have a substituent, R³⁷ is a hydrogen atom or a C₁₋₂₀ alkyl group or R³⁶ and R³⁷ together form a phenyl group which may have a substituent, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form a phenyl group which may have a substituent, R⁴¹ is a hydrogen atom or a C₁₋₂₀ alkyl group or R⁴⁰ and R⁴¹ together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; X¹ and X² together are nitrogen atoms; each of R³⁵, R³⁶, and R³⁷ is independently a hydrogen atom or a C₁₋₂₀ alkyl group or R³⁵ and R³⁶ together form an unsubstituted phenyl group or a phenyl group substituted with a C₁₋₁₀ alkyl group, R³⁷ is a hydrogen atom or a C₁₋₂₀ alkyl group or R³⁶ and R³⁷ together form an unsubstituted phenyl group or a phenyl group substituted with a C₁₋₁₀ alkyl group, and R³⁵ is a hydrogen atom or a C₁₋₂₀ alkyl group; each of R³⁹, R⁴⁰, and R⁴¹ is independently a hydrogen atom or a C₁₋₂₀ alkyl group or R³⁹ and R⁴⁰ together form an unsubstituted phenyl group or a phenyl group substituted with a C₁₋₁₀ alkyl group, R⁴¹ is a hydrogen atom or a C₁₋₂₀ alkyl group or R⁴⁰ and R⁴¹ together form an unsubstituted phenyl group or a phenyl group substituted with a C₁₋₁₀ alkyl group, and R³⁹ is a hydrogen atom or a C₁₋₂₀ alkyl group is more preferable since the light emitting efficiency is high and the compatibility with respect to a resin is excellent.

As the compound represented by (I₄-5), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁶, and R³⁸ is independently a hydrogen atom or a C₁₋₂₀ alkyl group or R³⁵ and R³⁶ together form a phenyl group which may have a substituent, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form a phenyl group which may have a substituent, and R⁴² is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; X¹ and X² together are nitrogen atoms; each of R³⁵, R³⁶, and R³⁸ is independently a hydrogen atom or a C₁₋₂₀ alkyl group or R³⁵ and R³⁶ together form an unsubstituted phenyl group or a phenyl group substituted with a C₁₋₁₀ alkyl group, and R³⁸ is a hydrogen atom or a C₁₋₂₀ alkyl group; each of R³⁹, R⁴⁰, and R⁴² is independently a hydrogen atom or a C₁₋₂₀ alkyl group or R³⁹ and R⁴⁰ together form an unsubstituted phenyl group or a phenyl group substituted with a C₁₋₁₀ alkyl group, and R⁴² is a hydrogen atom or a C₁₋₂₀ alkyl group is more preferable since the light emitting efficiency is high and the compatibility with respect to a resin is excellent.

As the compound represented by (I₄-6), a compound in which R²³, R²⁴, R²⁵, and R²⁶ together are halogen atoms, unsubstituted phenyl groups, or phenyl groups substituted with a C₁₋₁₀ alkyl group or a C₁₋₁₀ alkoxy group; R²⁷ and R²⁸ together are hydrogen atoms, unsubstituted phenyl groups, or phenyl groups substituted with a C₁₋₂₀ alkyl group or a C₁₋₂₀ alkoxy group; X¹ and X² together are nitrogen atoms; each of R³⁵, R³⁷, and R³⁸ is independently a hydrogen atom or a C₁₋₂₀ alkyl group or R³⁷ and R³⁸ together form a phenyl group which may have a substituent, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form a phenyl group which may have a substituent, and R³⁹ is a hydrogen atom or a C₁₋₂₀ alkyl group is preferable, and a compound in which R²³, R²⁴, R²⁵, and R²⁶ together are halogen atoms or unsubstituted phenyl groups; R²⁷ and R²⁸ together are unsubstituted phenyl groups, or phenyl groups substituted with a linear or branched C₁₋₂₀ alkoxy group; X¹ and X² together are nitrogen atoms; each of R³⁵, R³⁷, and R³⁸ is independently a hydrogen atom or a C₁₋₂₀ alkyl group or R³⁷ and R³⁸ together form an unsubstituted phenyl group or a phenyl group substituted with a C₁₋₁₀ alkyl group, and R³⁵ is a hydrogen atom or a C₁₋₂₀ alkyl group; each of R³⁹, R⁴¹, and R⁴² is independently a hydrogen atom or a C₁₋₂₀ alkyl group or R⁴¹ and R⁴² together form an unsubstituted phenyl group or a phenyl group substituted with a C₁₋₁₀ alkyl group, and R³⁹ is a hydrogen atom or a C₁₋₂₀ alkyl group is more preferable since the light emitting efficiency is high and the compatibility with respect to a resin is excellent.

As the compound represented by any one of General Formulas (I₃-1) to (I₃-6), a compound represented by any one of the following General Formulas (I₃-7) to (I₃-9) is preferable, and as the compound represented by any one of General Formulas (I₄-1) to (I₄-6), a compound represented by any one of the following General Formulas (I₄-7) to (I₄-9) is preferable.

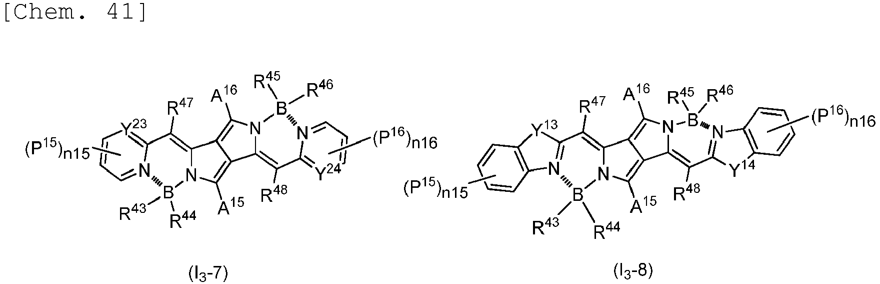

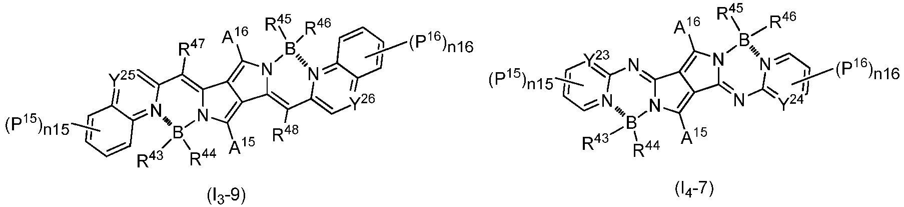

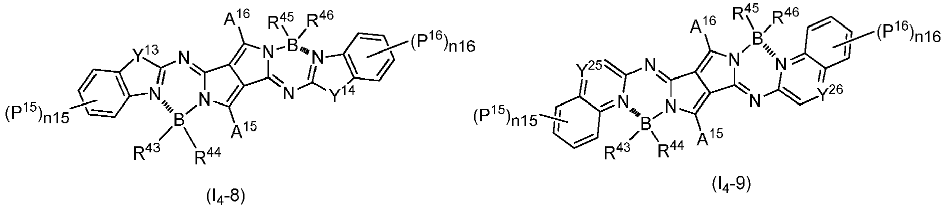

In General Formulas (I₃-7) and (I₄-7), each of Y²³ and Y²⁴ independently represents a carbon atom or a nitrogen atom. In General Formula (I₃-7), Y²³ and Y²⁴ are preferably the same type of atoms.

In General Formulas (I₃-8) and (I₄-8), each of Y¹³ and Y¹⁴ independently represents an oxygen atom or a sulfur atom. In General Formula (I₃-8), Y¹³ and Y¹⁴ are preferably the same type of atoms.

In General Formulas (I₃-9) and (I₄-9), each of Y²⁵ and Y²⁶ independently represents a carbon atom or a nitrogen atom. In General Formula (I₂-9), Y²⁵ and Y²⁶ are preferably the same type of atoms.

In General Formulas (I₃-7) to (I₃-9), each of R⁴⁷ and R⁴⁸ independently represents a hydrogen atom or an electron withdrawing group, and since fluorescence intensity becomes high, each of R⁴⁷ and R⁴⁸ is preferably a trifluoromethyl group, a cyano group, a nitro group, a sulfonyl group, or a phenyl group, and particularly preferably a trifluoromethyl group or a cyano group. In General Formula (I₃-7), R⁴⁷ and R⁴⁸ are preferably the same type of functional groups.

In General Formulas (I₃-7) to (I₃-9) and (I₄-7) to (I₄-9), each of R⁴³, R⁴⁴, R⁴⁵, and R⁴⁶ represents a halogen atom or an aryl group which may have a substituent. As the aryl group, those exemplified as "any group which does not inhibit fluorescence of a compound" represented by each of R^{a} and R^{b} can be used. In addition, the substituent which the aryl group may have may be "any group which does not inhibit fluorescence of a compound", and examples thereof include a C₁₋₆ alkyl group, a C₁₋₆ alkoxy group, an aryl group, and a heteroaryl group. In General Formulas (I₃-7) to (I₃-9) and (I₄-7) to (I₄-9), all of R⁴³ to R⁴⁶ may be different groups or may be the same type of groups. As the compound represented by any one of General Formulas (I₃-7) to I₃-9) and (I₄-7) to (I₄-9), a compound in which all of R⁴³ to R⁴⁶ are the same type of halogen atoms or phenyl groups which may have the same type of substituents is preferable, a compound in which all of R⁴³ to R⁴⁶ are fluorine atoms or unsubstituted phenyl groups is more preferable, and a compound in which all of R⁴³ to R⁴⁶ are fluorine atoms is particularly preferable.

In General Formulas (I₃-7) to (I₃-9) and (I₄-7) to (I₄-9), each of P¹⁵ and P¹⁶ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P¹⁵ and P¹⁶ include the same as those exemplified as R^{g}, (p1) to (p3), or (q1) to (q3). Each of P¹⁵ and P¹⁶ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group from the viewpoint of safety with respect to a living body, and these substituents may further have a substituent. Here, since, even in the case of a substituent other than these substituents, it is possible to improve safety by further introducing a suitable substituent, the present invention is not limited to these substituents.

In General Formulas (I₃-7) to (I₃-9) and (I₄-7) to (I₄-9), each of n15 and n16 independently represents an integer of 0 to 3. In a case where a plurality of P¹⁵'s are present in one molecule (that is, in a case where n15 is 2 or 3), all of the plurality of P¹⁵'s may be the same type of functional groups, or may be the different types of functional groups. The same applies to P¹⁶.

In General Formulas (I₃-7) to (I₃-9) and (I₄-7) to (I₄-9), each of A¹⁵ and A¹⁶ independently represents a phenyl group which may have one to three substituents selected from the group consisting of a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group as the substituent which the phenyl group may have the same as those exemplified as R^{g}, (p1) to (p3), or (q1) to (q3) . Each of A¹⁵ and A¹⁶ is preferably an unsubstituted phenyl group, a phenyl group having one or two C₁₋₂₀ alkoxy groups as the substituent, more preferably an unsubstituted phenyl group or a phenyl group having one C₁₋₂₀ alkoxy group as the substituent, and still more preferably an unsubstituted phenyl group or a phenyl group having one C₁₋₁₀ alkoxy group as the substituent. In addition, the compound represented by General Formula (I₃-7) is preferably a compound in which A¹⁵ and A¹⁶ are the same type of functional groups.

As the compound represented by any one of General Formulas (I₃-1) to (I₃-6) and (I₄-1) to (I₄-6), a compound represented by any one of the following General Formulas (6-1) to (6-12) and (7-1) to (7-12) is exemplified. In General Formulas (6-7) to (6-12) and (7-7) to (7-12), Ph means an unsubstituted phenyl group. As the compound represented by any one of General Formulas (I₃-1) to (I₃-6) and (I₄-1) to (I₄-6), in particular, compounds represented by General Formulas (6-4), (6-5), (6-7), (6-8), (7-4), (7-5), (7-7), or (7-8) are preferable, and compounds represented by General Formulas (6-4), (6-5), (6-7), or (6-8) are more preferable.

In General Formulas (6-1) to (6-12) and (7-1) to (7-12), each of P⁵ to P⁸ independently represents a halogen atom, a C₁₋₂₀ alkyl group, a C₁₋₂₀ alkoxy group, an amino group, a monoalkylamino group, or a dialkylamino group. Examples of the C₁₋₂₀ alkyl group, the C₁₋₂₀ alkoxy group, the monoalkylamino group, or the dialkylamino group represented by each of P⁵ to P⁸ include the same as those exemplified as R^{g}, (p1) to (p3), or (q1) to (q3). Each of P⁵ to P⁸ is preferably a C₁₋₂₀ alkyl group, a C₁₋₂₀ alkoxy group, an (unsubstituted) phenyl group, a p-methoxyphenyl group, a p-ethoxyphenyl group, a p-dimethylaminophenyl group, a dimethoxyphenyl group, a thienyl group, or a furanyl group, from the viewpoint of safety with respect to a living body, more preferably a C₁₋₂₀ alkyl group, a C₁₋₂₀ alkoxy group, a phenyl group, a p-methoxyphenyl group, a p-ethoxyphenyl group, a dimethoxyphenyl group, a thienyl group, or a furanyl group, still more preferably a C₁₋₂₀ alkyl group or a C₁₋₂₀ alkoxy group, and still more preferably a C₁₋₁₀ alkyl group or a C₁₋₁₀ alkoxy group, and these substituents may further have a substituent. Here, since, even in the case of a substituent other than these substituents, it is possible to improve safety by further introducing a suitable substituent, the present invention is not limited to these substituents.

In General Formulas (6-1) to (6-12) and (7-1) to (7-12), each of n5 to n8 independently represents an integer of 0 to 3. In a case where a plurality of P⁵'s are present in one molecule (that is, in a case where n5 is 2 or 3), all of the plurality of P⁵'s may be the same type of functional groups, or may be the different types of functional groups. The same applies to P⁶ to P⁸.

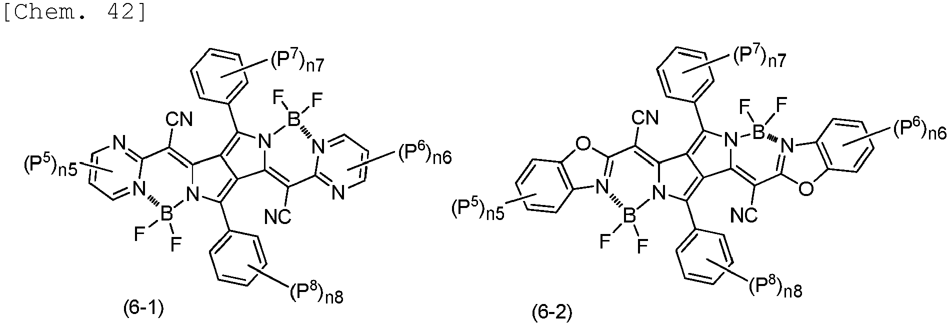

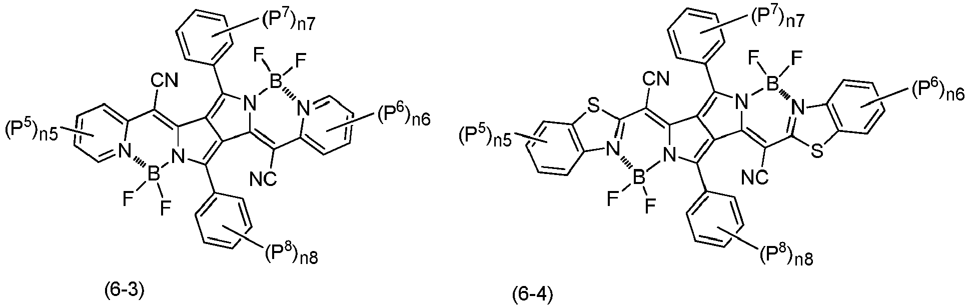

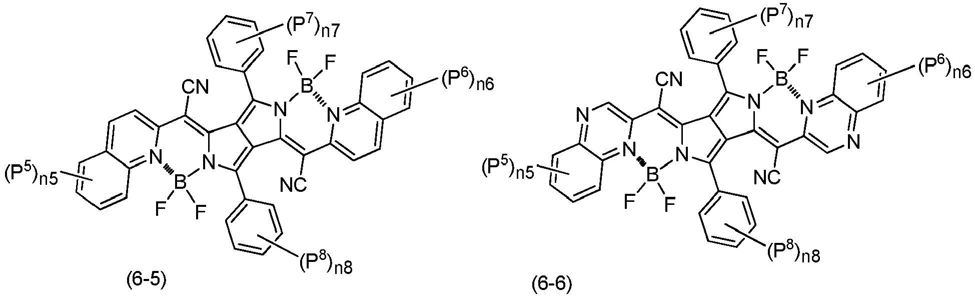

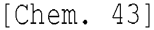

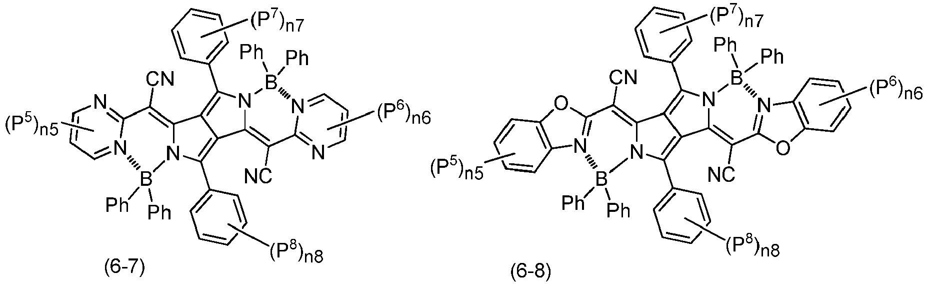

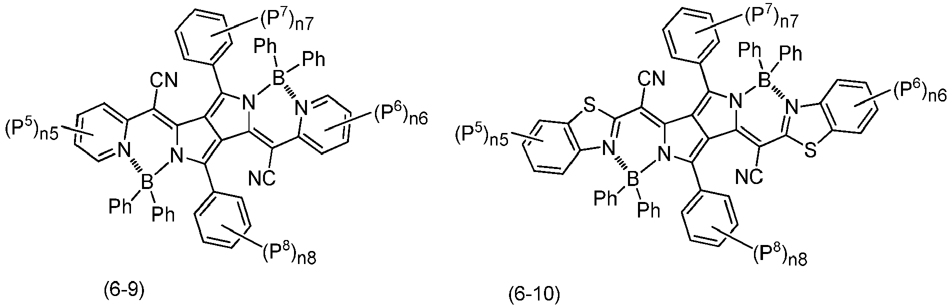

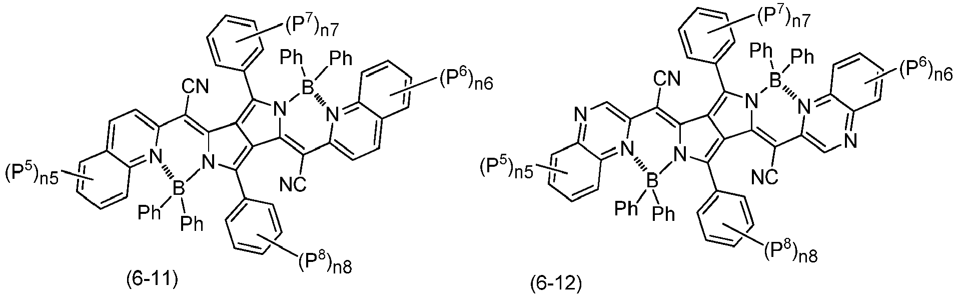

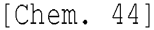

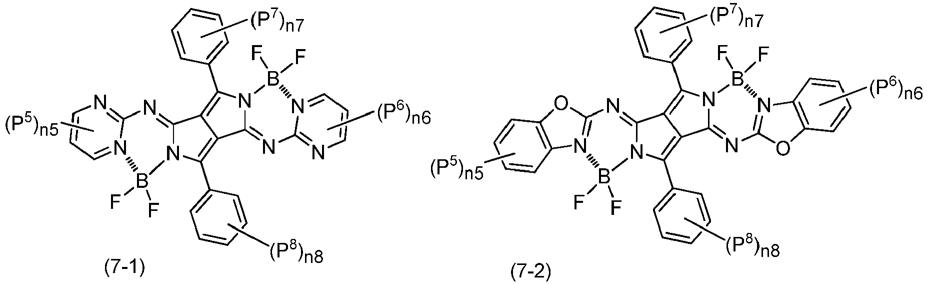

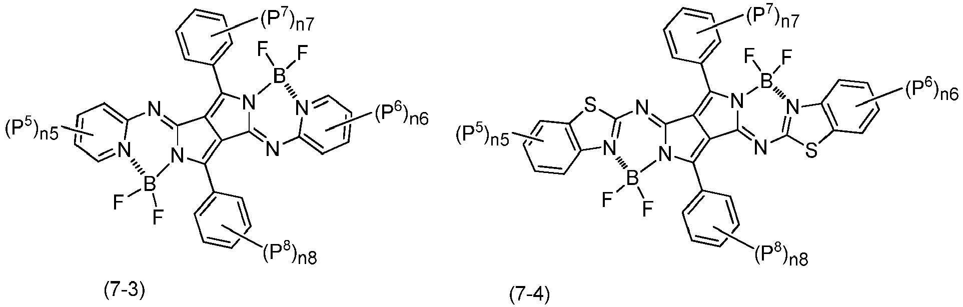

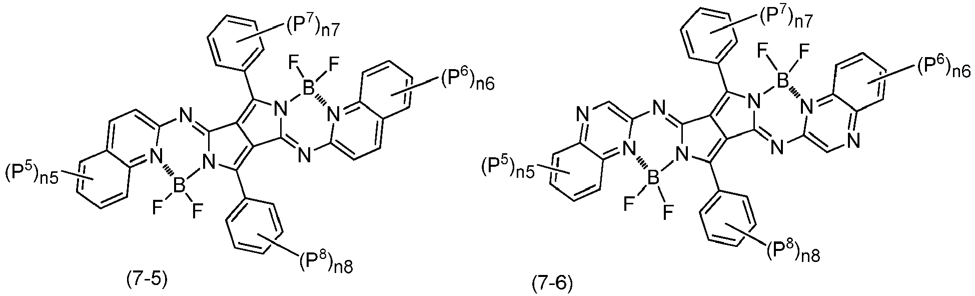

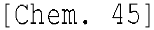

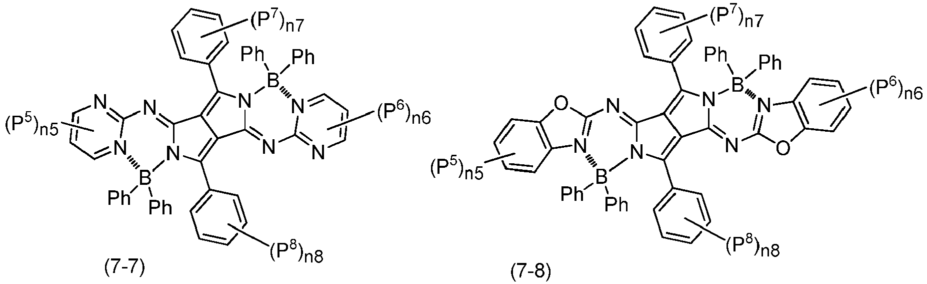

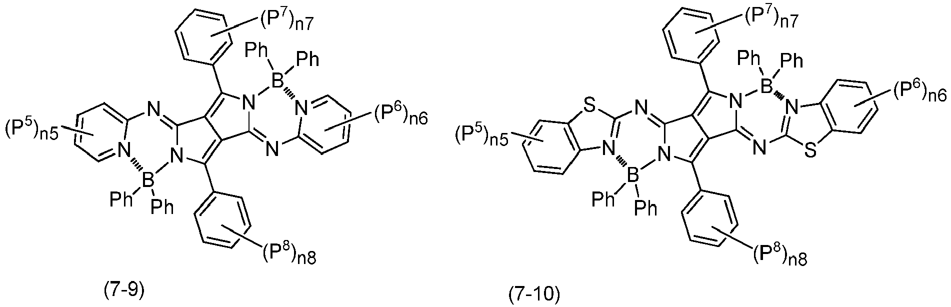

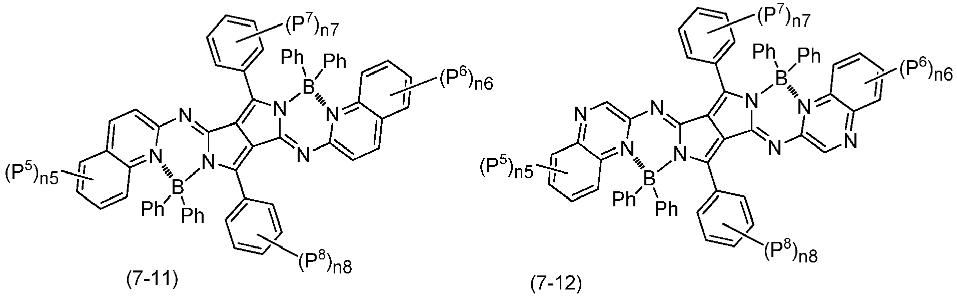

As the compounds represented by General Formulas (6-1) to (6-12) or (7-1) to (7-12), a compound in which each of P⁵ to P⁸ is independently a C₁₋₂₀ alkyl group or a C₁₋₂₀ alkoxy group and each of n5 to n8 is independently is 0 to 2 is preferable, a compound in which each of P⁵ and P⁶ is independently a C₁₋₂₀ alkyl group, each of n5 and n6 is independently 0 to 2, each of P⁷ and P⁸ is independently a C₁₋₂₀ alkoxy group, and each of n7 and n8 is independently 0 or 1 is more preferable, and a compound in which each of P⁵ and P⁶ is independently a C₁₋₂₀ alkyl group, each of n5 and n6 is independently 1 or 2, each of P⁷ and P⁸ is independently a C₁₋₂₀ alkoxy group, and each of n7 and n8 is independently 1 is still more preferable.

Examples of the compound represented by each of General Formulas (6-1) to (6-12) include a compound represented by each of the following General Formulas (6-1-1) to (6-12-1). "λ" is the peak wavelength of an absorption spectrum of each compound, and "Em" is the peak wavelength of a fluorescence spectrum.

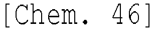

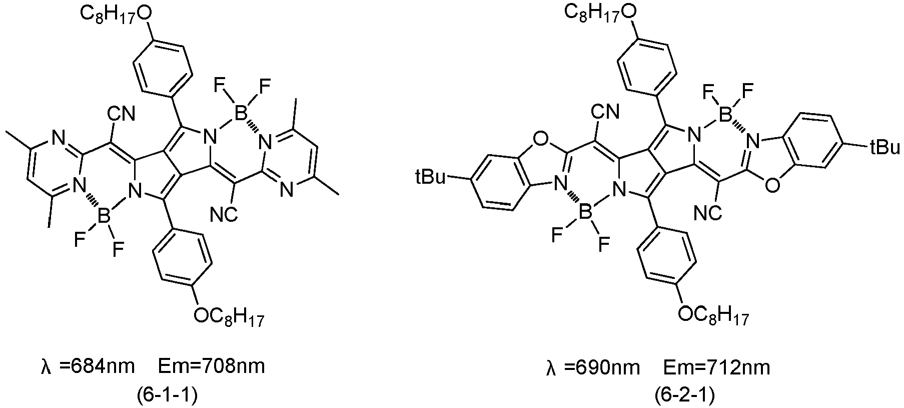

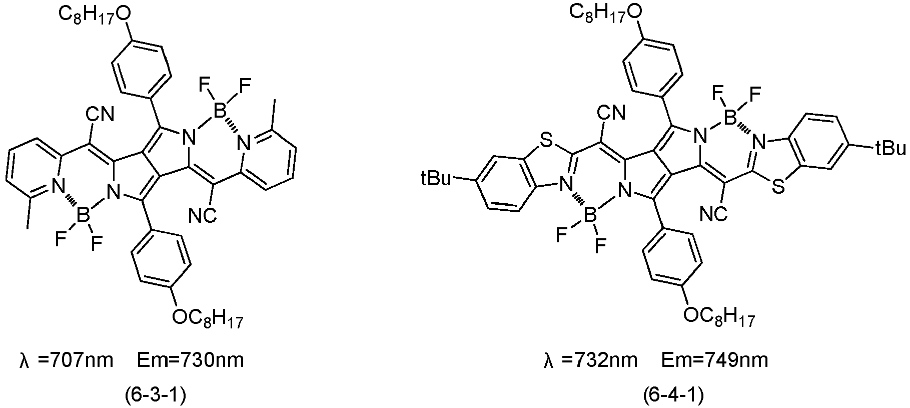

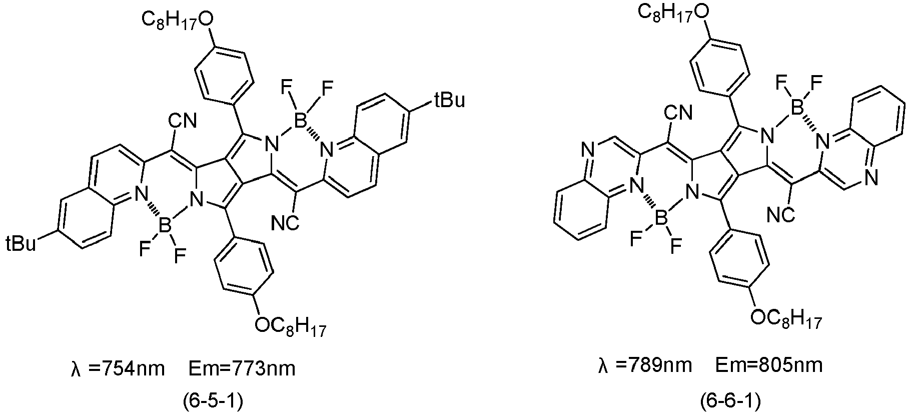

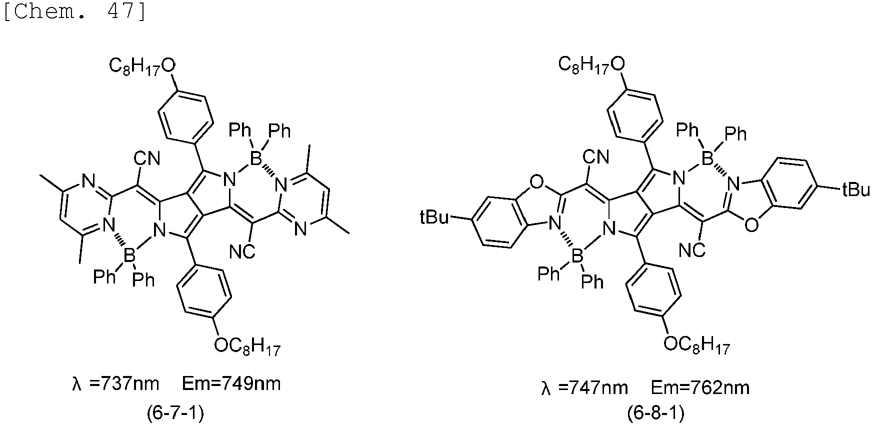

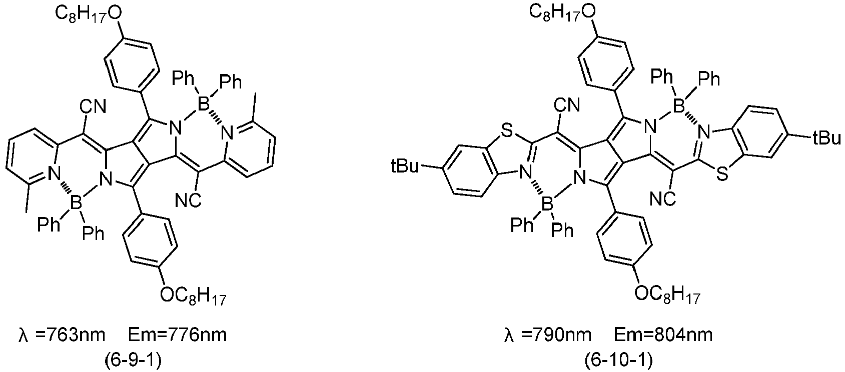

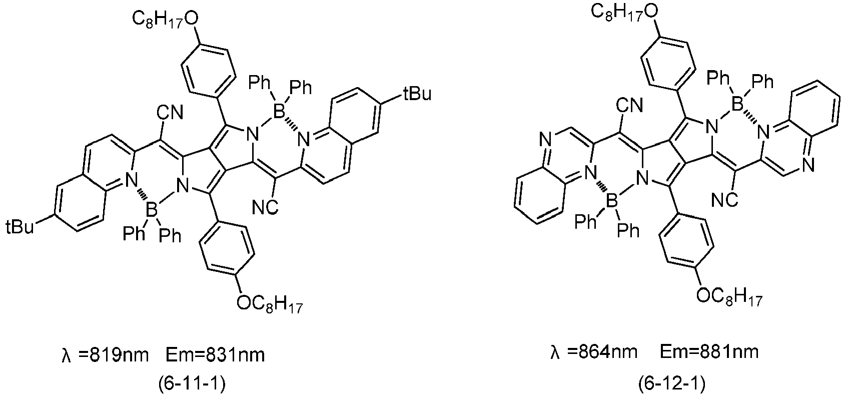

### <Resin Component>

Although the resin component contained in the resin composition according to the present invention is not particularly limited, in consideration of the types of the near infrared fluorescent material to be blended, product quality required at the time of forming a molded article, or the like, the resin component can be suitably selected from known resin compositions or improved products thereof and used. For example, the resin component may be a thermoplastic resin or may be a thermosetting resin. In the case of being used in a molded article, as the resin component contained in the resin composition according to the present invention, a thermoplastic resin is preferable since a thermosetting resin is likely to be cured at the time of melt-kneading. The resin component used in the present invention may be used alone or in combination of two or more types thereof. In a case where two or more types thereof are used in combination, a combination of resins having high compatibility is preferably used.

Examples of the resin component used in the present invention include urethane resins such as polyurethane (PU) and thermoplastic polyurethane (TPU); polycarbonate (PC); vinyl chloride-based resins such as polyvinyl chloride (PVC) and a vinyl chloride-vinyl acetate copolymer resin; acrylic resins such as polyacrylic acid, polymethacrylic acid, polymethyl acrylate, polymethyl methacrylate (PMMA), and polyethyl methacrylate; polyester resins such as polyethylene terephthalate (PET), polybutylene terephthalate, polytrimethylene terephthalate, polyethylene naphthalate, and polybutylene naphthalate; polyamide-based resins such as Nylon (registered trademark); polystyrene-based resins such as polystyrene (PS), imide-modified polystyrene, an acrylonitrile-butadiene-styrene (ABS) resin, an imide-modified ABS resin, a styrene-acrylonitrile copolymer (SAN) resin, and a acrylonitrile-ethylene-propylene-diene-styrene (AES) resin and olefin-based resins such as a polyethylene (PE) resin, a polypropylene (PP) resin, and a cycloolefin resin; cellulose-based resins such as nitrocellulose and cellulose acetate; silicone-based resins; thermoplastic resins such as a fluorine-based resin; epoxy-based resins such as a bisphenol A type epoxy resin, a bisphenol F-type epoxy resin, an isocyanurate-based epoxy resin, and a hydantoin-based epoxy resin; amino-based resins such as a melamine-based resin and a urea resin; phenol-based resins; and thermosetting resins such as an unsaturated polyester-based resin.

As the resin component contained in the resin composition according to the present invention, since the dispersion of the near infrared fluorescent material according to the present invention is high, as the resin component, a fluorine-based resin, a silicone-based resin, a urethane-based resin, an olefin-based resin, a vinyl chloride-based resin, a polyester-based resin, a polystyrene-based resin, a polycarbonate resin, a polyamide-based resin, or an acryl-based resin is preferable, and a urethane-based resin, an olefin-based resin, a polystyrene-based resin, a polyester-based resin, or a vinyl chloride-based resin is more preferable. In particular, in a case where the resin composition according to the present invention is used as a medical material, in consideration of low solubility in body fluid such as blood and difficult elution in a use environment or biocompatibility, PTFE, Teflon (registered trademark), silicone, PU, TPU, PP, PE, PC, PET, PS, polyamide, or PVC is preferable, and TPU, PU, PP, PE, PET, or PS is more preferable.

Moreover, in a case where the resin composition according to the present invention contains a thermoplastic resin composition, as the resin component, all the resin components may be thermoplastic resins, or a small amount of non-thermoplastic resin may be contained. Similarly, in a case where the resin composition according to the present invention contains a thermosetting resin composition, as the resin component, all the resin components may be thermosetting resins, or a small amount of non-thermosetting resin may be contained.

### <Resin Composition>

The resin composition according to the present invention can be prepared by mixing and dispersing the near infrared fluorescent material according to the present invention in a resin component. The near infrared fluorescent material according to the present invention contained in the resin composition according to the present invention may be only one type, or two or more types thereof may be contained.

The content of the near infrared fluorescent material in the resin composition according to the present invention is a concentration at which the near infrared fluorescent material can be mixed with the resin, and the content is 0.0001% by mass or greater from the viewpoint of the fluorescence intensity and the detection sensitivity thereof, and the content is 0.5% by mass or less, preferably within the range of 0.001% to 0.5% by mass, and more preferably within the range of 0.001% to 0.05% by mass from the viewpoint of detection sensitivity by the concentration quenching or the re-absorption of fluorescence. In addition, since the near infrared fluorescent material according to the present invention has a high molar absorption coefficient and a high quantum yield even in the resin, even in a case where the material concentration in the resin is relatively low, it is possible to sufficiently observe the emission using a camera. It is desirable that the material concentration is low from the viewpoint of low possibility to elute, low possibility to bleed out from a molded article processed from the resin composition, and being capable of processing a molded article which requires transparency.

A method of mixing and dispersing the near infrared fluorescent material according to present invention is not particularly limited, and the mixing and dispersing may be performed by any method known in the related art, and an additive may further used in combination. In addition, the resin composition according to the present invention can be obtained by adding the near infrared fluorescent material according to present invention to the resin composition and melt-kneading. In this manner, a resin composition having a state in which the near infrared fluorescent materials are evenly dispersed in the resin is obtained. Among these known methods of mixing and dispersing, a melt-kneading method suitable for actual production is preferable.

Moreover, in a case where, by melt-kneading a resin and a fluorescent material, the fluorescent material is dispersed in a thermoplastic resin, even in a case where melt-kneading is performed at a temperature lower than the decomposition point of the fluorescent material, depending on the type of the resin or the fluorescent material and the kneading conditions, fluorescence is not emitted by poor dispersion or decomposition of the fluorescent material, in some cases. Furthermore, whether the fluorescent material can be dispersed in a thermoplastic resin or the like or not is difficult to predict from the thermal physical properties of the fluorescent material.

In contrast, the near infrared fluorescent material according to the present invention can be evenly mixed with various resin components and dispersed therein, and can emit fluorescence at a high quantum yield even in the resin. The reason for this is not clear, but, it is thought to be as follows. In a case where a material is dispersed by a method such as melt-kneading, it is thought that the quantum yield of the fluorescence is decreased by concentration quenching when aggregation or the like occurs. Therefore, for efficient emission of fluorescence by the material, it is desired that the compatibility with a resin is high and the fluorescent material can be evenly dispersed. An SP value can be exemplified as one indicator of whether the compatibility is high or not. As the difference between the SP value of a material and the SP value of a resin is smaller, the compatibility is high and the material can be evenly dispersed in the resin. On the other hand, in a case where the SP values or the like are different, description by other physical property parameters is also possible. For example, calculated values such as the solubility of the material, the partition coefficient, the relative dielectric constant, and the polarizability of the material or the compatibility with the resin from the measured values can be explained. In addition, the compatibility between the resin and the fluorescent material varies depending on the crystallinity of the resin in some cases.

Additionally, the compatibility between the resin and the fluorescent material can be controlled by the functional group which the molecule itself of the fluorescent material has. For example, in a case where the fluorescent material is dispersed in a fat-soluble (hydrophobic) polyolefin-based resin such as polypropylene or polyethylene, the material molecule preferably has a hydrophobic group. For example, by introducing a hydrophobic group such as an alicyclic alkyl group, a long-chain alkyl group, a halogenated alkyl group, or an aromatic ring into the fluorescent material molecule, the compatibility with the resin can be improved. However, the present invention is not limited to these functional groups. In addition, in a case where the fluorescent material is dispersed in a resin having high polarity such as polyurethane or polyamide resin, the fluorescent material molecule preferably has a hydrophilic group such as a carboxyl group, a hydroxyl group, an amino group, an alkoxy group, an aryloxy group, an alkylamino group, an ester, or an amide. However, the present invention is not limited thereto.

To increase the compatibility with a resin, it is necessary to suppress aggregation of the material molecules. In the case of a fluorescent material, introduction of an aromatic ring or a heterocycle into the molecule to ensure extension and planarity of a conjugated system is performed. However, by introduction of the ring, there is a tendency that the flatness is increased and stacking is likely to occur, or aggregation is likely to occur. It is thought that, since the near infrared fluorescent material according to the present invention has a material skeleton formed of a wide conjugate plane around the boron atom, the compound is likely to be aggregated, but by polarizing by introducing an electron donating group or an electron withdrawing substituent or by introducing a bulky functional group, aggregation of a material is suppressed, and the compatibility with various resins can be achieved.

The partition coefficient or the SP value which is an index of compatibility can be estimated as a water/octanol partition coefficient or a SP value of Hildebrand from "Hansen solubility parameter" obtained by calculation using a commercially available software. For example, among the near infrared fluorescent materials according to the present invention the partition coefficients and the SP values of compounds represented by the following compounds (8-1) to (8-8) are as follows.

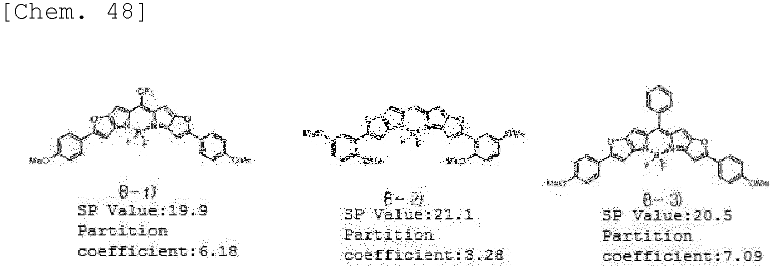

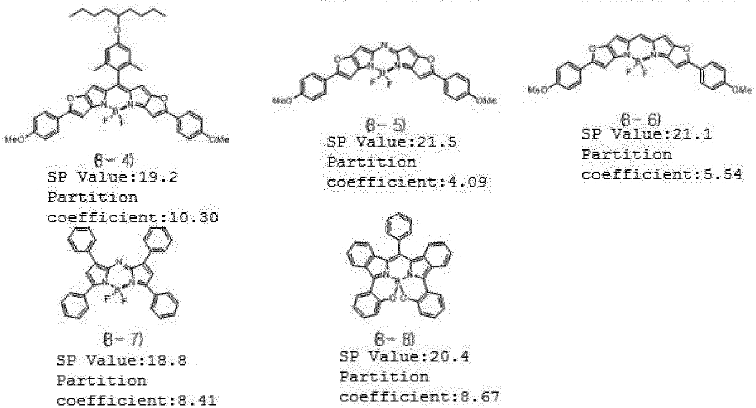

The near infrared fluorescent material according to the present invention can be evenly dispersed and mixed by being melt-kneaded with a resin component such as PP, and the kneaded resin composition or a molded article processed from the resin composition can stably emit near infrared fluorescence at a higher emission quantum yield. The reason why the near infrared fluorescent material according to the present invention exhibits emission characteristics even in the case of being melt-kneaded with the resin composition unlike other many organic near infrared fluorescent material is not clear, but it is thought that, since the near infrared fluorescent material according to the present invention has a rigid material skeleton configured of a wide conjugate plane, the heat resistance thereof is high and the compatibility thereof with the resin is excellent. Moreover, it is knowledge found by the present inventors for the first time that, even in a case where the BODIPY material or the DPP-based boron complex is subjected to a high-load treatment such as melt-kneading, fluorescence characteristics thereof is not impaired.

In a case where a general emission detector provided with a filter for cutting noise due to excitation light is used, when the difference between the maximum absorption wavelength and the maximum fluorescent wavelength (Stokes shift) of the resin composition according to the present invention is small, fluorescent is cut by the filter, and thus, it is difficult to detect with high sensitivity. Therefore, difference between the maximum absorption wavelength and the maximum fluorescent wavelength of the resin composition according to the present invention is preferably 10 nm or greater, and more preferably 20 nm or greater. As the difference is increased, even in a case where a general detector provided with a filter for cutting noise due to excitation light is used, it is possible to detect the fluorescent emitted from the molded article with high sensitivity.

However, even in a case where the Stokes shift is small, under conditions as described below, it is possible to detect the near infrared fluorescence from the resin composition according to the present invention with high sensitivity. For example, if excitation is possible at shorter-wavelength light than the maximum absorption wavelength, it is possible to detect the fluorescence even when the noise is cut. In addition, in a case where the fluorescence spectrum is broad, it is possible to sufficiently detect fluorescence even in when the noise is cut. On the other hand, some of fluorescent materials have a plurality of fluorescence peaks. In this case, even in a case where the Stokes shift is small, if a fluorescence peak (second peak) is present on the longer wavelength side, it is possible to detect the fluorescence peak with high sensitivity even in the case of using a detector provided with a filter for cutting noise. The difference between the fluorescence peak wavelength on the long wavelength side in a case where the resin composition of the present invention has a plurality of fluorescence and the maximum absorption wavelength may be 30 nm or longer, and is preferably 50 nm or longer. Moreover, the present invention is not limited to the above-described conditions if an excitation light source, a cut filter, or the like is suitably selected.

Even when the resin composition according to the present invention is excited by excitation light in the near infrared region, the color thereof is not changed in a visual observation state, and the resin composition emits fluorescence in the invisible near infrared region, and thus, this can be detected by a detector. Therefore, the maximum absorption wavelength with respect to the excitation light in the near infrared region may be 600 nm or longer, and from the viewpoint of the absorption efficiency, the maximum absorption wavelength is preferably close to the wavelength of the excitation light, more preferably 650 nm or longer, and particularly preferably 680 nm or longer. Furthermore, in a case where the resin composition is used as medical tools such as that of implant, the maximum absorption wavelength is preferably 700 nm or longer.

The resin composition according to the present invention or a molded article obtained from the composition according to the present invention are having the maximum fluorescence wavelength of 650 nm or longer. In consideration of no change in the color of the irradiated object and detection sensitivity, although the resin composition according to the present invention or a molded article obtained from the composition, having the maximum fluorescence wavelength of 650 nm or longer, has no practical problem, the maximum fluorescence wavelength is preferably 700 nm or longer, and more preferably 720 nm or longer. In a case where the resin composition or a molded article obtained from the composition has a plurality of fluorescence peaks, although the wavelength of the maximum fluorescence peak thereof is 720 nm or less, the resin composition or a molded article obtained from the composition may have a fluorescence peak having a sufficient detection sensitivity at 740 nm or greater. In this case, the intensity of the fluorescence peak on the longer wavelength side (second peak) is preferably 5% or greater and more preferably 10% or greater, with respect to the intensity of the maximum fluorescence wavelength.

The resin composition according to the present invention and a molded article obtained from the composition preferably has strong absorption in the range of 650 nm to 1500 nm and emits a strong fluorescence peak in this range. Light of 650 nm or longer is less likely to be affected by hemoglobin, and light of 1500 nm or less is less likely to be affected by water. That is, since light within the range of 650 nm to 1500 nm has a high skin transparency is less likely to be affected by foreign substances in a living body, the light within the range of 650 nm to 1500 nm is suitable as a wavelength range of light used to visualize a medical implant embedded subcutaneously or the like. In a case where the maximum absorption wavelength and the maximum fluorescence wavelength are within the range of 650 nm to 1500 nm, the resin composition according to the present invention and a molded article obtained from the composition is suitable for detection by light within the range of 650 nm to 1500 nm and suitable as a medical tool or the like used in vivo.

The resin composition according to the present invention may contain components other than the resin components and the near infrared fluorescent material, as long as the components do not impair the effect of the present invention. Examples of the other components include an ultraviolet absorber, a heat stabilizer, a light stabilizer, an antioxidant, a flame retardant, a flame retardant auxiliary agent, a crystallization accelerator, a plasticizer, an antistatic agent, a colorant, and a release agent.

### <Molded Article>

By processing the resin composition according to the present invention, a molded article to which the detection is possible by the near infrared fluorescent is obtained. The molding method is not particularly limited, and examples thereof include a casting method, an injection molding method using a mold, a compression molding method, an extrusion molding method using a T-die, and a blow molding method.

In the production of a molded article, the molded article may be formed of only the resin composition according to the present invention, or the resin composition according to the present invention and other resin compositions may be used as the raw materials. For example, all of the molded article may be molded from the resin composition according to the present invention, or only a part of the molded article may be molded from the resin composition according to the present invention. The resin composition according to the present invention is preferably used as a raw material constituting the surface portion of the molded article. For example, in a case where a catheter is molded, by molding only the tip portion of the catheter from the resin composition according to the present invention and by molding the remaining portion from a resin composition not containing a near infrared fluorescent material, it is possible to produce a catheter of which only the tip portion emits near infrared fluorescence. In addition, by molding by alternately stacking the resin composition according to the present invention and a resin composition not containing a near infrared fluorescent material, it is possible to produce a molded article which emits near infrared fluorescence in the form of a stripe. In addition, surface coating may be performed to enhance the visibility of the molded article.

Fluorescent detection can be performed by using a commercially available a fluorescent detection apparatus or the like by an ordinary method. As the excitation light used in fluorescence detection, any light source can be used, and, in addition to a near infrared lamp having a wide wavelength width, a laser having a narrow wavelength width, an LED, or the like can be used.

Even when a molded article obtained from the resin composition containing the near infrared fluorescent material is irradiated with light in the near infrared region, the color thereof is not changed and the molded article emits near infrared fluorescence which can be detected with higher sensitivity than that in the related art, and thus, the molded article is particularly suitable for medical tools that are inserted or indwelled in the body of a patient.

In a case where fluorescence detection is performed on the molded article obtained from the resin composition containing the near infrared fluorescent material, it is preferable to irradiate with excitation light in the near infrared region, and in a case where the irradiated object may exhibit somewhat reddish color, the excitation light in the near infrared region is not necessarily used. For example, in a case where fluorescence detection is used to detect the medical tool in the body by irradiating with excitation light, it is necessary to use excitation light in a wavelength region having high transparency with respect to a living body such as the skin, and in this case, excitation light of 650 nm or longer having high transparency with respect to a living body may be used.

Examples of the medical tool include a stent, a coil embolus, a catheter tube, an injection needle, an indwelling needle, a port, a shunt tube, a drain tube, and an implant. Examples

Hereinafter, the present invention will be described in more detail with reference to examples, reference examples and comparative examples, but the present invention is not limited thereto.

### [Preparation Example 1] Synthesis of Near Infrared Fluorescent Material A

Under argon stream, 4-methoxyphenyl boronic acid (2.99 g, 19.7 mmol) was put into a 500 mL three-neck flask, then, this was dissolved in toluene (120 mL), and [1,1'-bis(diphenylphosphino)-ferrocene]palladium (II) dichloride-dichloromethane complex (1:1) (100 mg), 30 mL of ethanol, 5-bromo-2-furaldehyde (3.46 g, 19.8 mmol), and a 2 mol/L sodium carbonate aqueous solution (20 mL) were added thereto, followed by stirring at 80°C for 14 hours. After the reaction ended, the organic phase was washed with water and a saturated saline solution and dried over anhydrous sodium sulfate, then, the desiccant was separated by filtration, and the solvent was concentrated under reduced pressure. The obtained crude product was separated and purified by flash silica gel chromatography (eluent: hexane/ethyl acetate = 19/1 → 4/1), whereby 5-(4-methoxyphenyl)-furan-2-carbaldehyde (a-1) was obtained as a pale yellow liquid (obtained amount: 3.39 g, yield: 84.8%).

Next, under an argon stream, the compound (a-1) (3.39 g, 16.8 mmol) and ethyl azidoacetate (8.65 g, 67.0 mmol) were dissolved in ethanol (300 mL) in a 1 L three-neck flask, and a 20% by mass sodium ethoxide ethanol solution (22.8 g, 67.0 mmol) was slowly added dropwise to the obtained solution at 0°C in an ice bath, followed by stirring for 2 hours. After the reaction ended, a saturated ammonium chloride aqueous solution was added thereto to adjust the pH to be weakly acidic, water was added thereto, suction filtration was performed, and the obtained material was dried, whereby ethyl 2-azido-3-[5-(4-methoxyphenyl)-furan-2-yl] acrylate (a-2) was obtained as a yellow solid (obtained amount: 3.31 g, yield: 63.1%) .

Furthermore, the compound (a-2) (3.31 g, 10.6 mmol) was put into a 200 mL egg-plant shaped flask, and this was dissolved in toluene (60 mL), followed by refluxing and stirring for 1.5 hours. After the solution after refluxing and stirring was concentration under reduced pressure, the obtained crude product was recrystallized (solution: hexane and ethyl acetate), then, the resultant product was subjected to suction filtration, and the obtained material was dried, whereby 2-(4-methoxyphenyl)-4H-furo[3.2-b]pyrrole-5-carboxylicacid ethyl ester (a-3) was obtained as a brown crystal (obtained amount: 2.32 g, yield: 76.8%).

Next, the compound (a-3) (1.90 g, 6.66 mmol) was put into a 300 mL flask, and an aqueous solution obtained by dissolving ethanol (60mL) and sodium hydroxide (3.90 g, 97.5 mmol) in 30 mL of water was added thereto, followed by refluxing and stirring for 1 hour. After the solution after refluxing and stirring was cooled, a 6 mol/L hydrochloric acid aqueous solution was added thereto to adjust the solution to be acidic, water was added thereto, suction filtration was performed, and the obtained material was vacuum-dried, whereby 2-(4-methoxyphenyl)-4H-furo[3.2-b]pyrrole-5-carboxylicacid (a-4) was obtained as a gray solid (obtained amount: 1.56 g, yield: 91%).

Subsequently, the compound (a-4) (327 mg, 5.52 mmol) and trifluoroacetic acid (16.5 mL) were put into a 200 mL three-neck flask, followed by stirring at 45°C. After the compound (a-4) was dissolved, stirring was performed for 15 minutes until the bubbles subsided. Trifluoroacetic anhydride (3.3 mL) was added to the solution after stirring, and the resultant product was allowed to react at 80°C for 1 hour. After the reaction ended, a saturated sodium hydrogen carbonate aqueous solution and ice were added thereto to neutralize the solution, then, suction filtration was performed, and the filtered material was vacuum-dried, whereby a compound (a-5) was obtained as a black solid (obtained amount: 320 mg). The compound (a-5) was used in the next reaction without purification.

Under an argon stream, the compound (a-5) (320 mg) was put into a 200 mL three-neck flask, and toluene (70 mL), triethylamine (1.0 mL), and boron trifluoride diethylether complex (1.5 mL) were added dropwise thereto, followed by heating to reflux for 30 minutes. After the reaction ended, a saturated sodium hydrogen carbonate aqueous solution was added thereto, and the organic phase was collected. The organic phase was washed with water and a saturated saline solution and dried over anhydrous magnesium sulfate, then, the desiccant was separated by filtration, and the solvent was concentrated under reduced pressure. The obtained crude product was separated and purified by silica gel chromatography (eluent: toluene/ethyl acetate = 20/1 (in volume ratio)), whereby a near infrared fluorescent material A was obtained as a green crystal (obtained amount: 20 mg, yield: 6%).

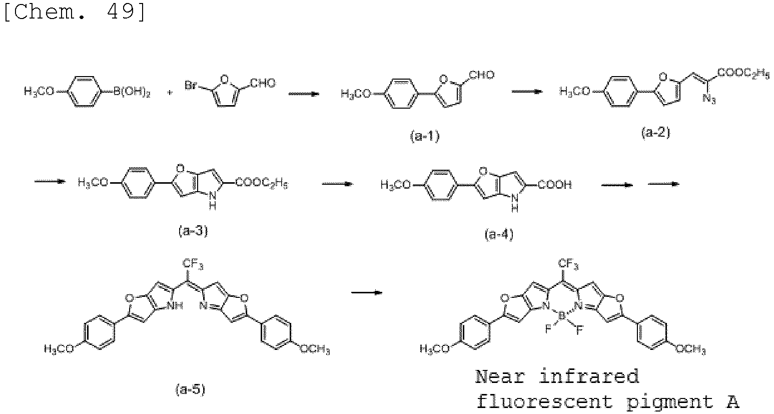

### [Reference Example 1]

TPU pellets (Tecoflex EG85A, manufactured by Lubrizol Corp.) (100 g) and a near infrared fluorescent material A (5 mg) synthesized in Preparation Example 1 were mixed, and the mixture was attached to the pellet surfaces.
Next, the pellets were put into Labo Plastomill, and melt-kneaded (kneading) at a set temperature of 190°C for 10 minutes. Thereafter, the kneaded material-containing resin was taken out, and made to be a film.

The film was obtained in the following manner. First, the material-containing resin was heated for 5 minutes while being sandwiched between iron plates heated to 200°C, and pressed at 5 to 10 mPa while the steel plates were cooled.

The absorption spectrum of the obtained film was measured using an ultraviolet visible near infrared spectrophotometer "UV3600" manufactured by SHIMADZU Co., and when the emission spectrum was measured using an Absolute PL quantum yields measurement system "Quantaurus-QY C11347" manufactured by Hamamatsu Photonics K.K., it was confirmed that the maximum absorption wavelength was 730 nm, the maximum fluorescence wavelength was around 755 nm, and a fluorescence peak was observed at 823 nm. A fluorescence quantum yield at this time was 26%. In addition, the visibility of the film in the near infrared fluorescent detection camera was high.

### [Reference Example 2]

In Reference Example 1, a material-containing resin was obtained in the same manner as in Reference Example 1 except that PP pellets (product name: PC630A, manufactured by SunAllomer Ltd.) were used instead of the TPU pellets, PP pellets (100g) and the near infrared fluorescent material A synthesized in Preparation Example 1 (10 mg) was mixed, and the material was attached to the pellet surfaces. The obtained material-containing resin made to be a film. The fluorescent spectrum of the obtained film was measured in the same manner as that of Reference Example 1, the maximum fluorescent wavelength was 810 nm, and the fluorescent quantum yield was 24%. In addition, the visibility of the film in the near infrared fluorescent detection camera was high.

### [Reference Example 3]

Eluting of the near infrared fluorescent material A was examined from the resin film which contains the near infrared fluorescent material A.

In Reference Example 1, the operation was performed in the same manner as in Reference Example 1 except that 100 mg of the near infrared fluorescent material A was used, a material-containing TPU film having a thickness of about 300 µm and having a material concentration of the near infrared fluorescent material A of 0.1% by mass was prepared.

An Eluting test of the obtained material-containing TPU film was performed. The eluting operation of the film was performed as follows according to ISO10993-10AnnexE. 5 g of the material-containing TPU film was cut into a size of 2 cm x 2 cm, and then was put into a 300 mL conical flask with 100 mL of methanol, followed by shaking at 25°C of room temperature for 8 hours. Next, the methanol was separated by filtration once, and the resultant product was extracted two times with the same amount of methanol by using the same film fine piece. The methanol extracted liquid obtained by the total three times of operations was concentrated by an evaporator, and the residues were melted with 5 mL of dichloromethane to obtain a test liquid.

The absorption spectrum and the light emission spectrum of the obtained test liquid were measured. As a result, the absorption and the fluorescent derived from the near infrared fluorescent material A could not be confirmed. Accordingly, it is found that the near infrared fluorescent material A was hardly eluted from the TPU film. Since the near infrared fluorescent material A was hardly eluted, a medical implant having high safeness which is molded using the resin composition according to the present invention was obtained.

### [Preparation Example 2] Synthesis of Near Infrared Fluorescent Material B

Synthesis of a near infrared fluorescent material B was performed in the following manner based on Organic Letters, 2012, Vol. 4, 2670-2673 and Chmestry A European Journal, 2009, Vol. 15, 4857-4864.

4-Hydroxybenzonitrile (25.3 g, 212 mmol), 800 mL of acetone, potassium carbonate (100 g, 724 mmol), and 1-bromooctane (48 g, 249 mmol) were put into a 2 L four-neck flask, followed by heating to reflux overnight. After the inorganic salt was filtered, acetone was removed under reduced pressure. Ethyl acetate was added to the obtained residues, and the organic layer was washed with water and a saturated saline solution, and treated with anhydrous magnesium sulfate. After the magnesium sulfate was separated by filtration, the solvent was removed under reduced pressure, and the residues were purified by silica gel column chromatography (eluent: hexane/ethyl acetate), whereby 4-octoxybenzonitrile (b-1) was obtained as colorless transparent liquid (obtained amount: 45.2 g, yield: 92%).

Next, under an argon stream, tert-butyloxy potassium (25.18 g, 224.4 mmol) and 160 mL of tert-amyl alcohol were put into a 500 mL four-neck flask, and a solution obtained by mixing the compound (b-1) (14.8 g, 64 mmol) synthesized above and 7 mL of tert-amyl alcohol was added thereto. While heating to reflux, a solution obtained by mixing succinic acid diisopropyl ester (6.5 g, 32 mmol) and 10 mL of tert-amyl alcohol was added dropwise thereto over a period of about 3 hours, and after dropping ended, the resultant product was heated to reflux for 6 hours. After the temperature was returned to room temperature, the obtained reaction liquid having high viscosity was put into a solution of acetic acid:methanol:water = 1:1:1, and the resultant product was heated to reflux for several minutes, whereby a red solid precipitated. The solid was separated by filtration, and washed with heated methanol and water, whereby 3,6-(4-octyloxyphenyl)pyrrolo[3,4-c]pyrrole-1,4(2H,5H)-dio ne (b-2) was obtained as a red solid (obtained amount: 5.6 g, yield: 32%).

In addition, 4-tert-butylaniline (10 g, 67 mmol), 70 mL of acetic acid, and sodium thiocyanate (13 g, 160 mmol) were put into a 200 mL three-neck flask. While maintaining the inside of the system at 15°C or lower, bromine (4.5 mL, 87 mmol) was added dropwise thereto over a period of about 20 minutes, and then, the resultant product was stirred at 15°C or lower for 3.5 hours. After the reaction liquid was put into 28% ammonia water (150 mL), the resultant product was stirred for a while, the precipitated solid was separated by filtration, the solid was extracted with diethyl ether, and the organic layer was washed with water. After the diethyl ether was removed under reduced pressure, the residues were purified by silica gel column chromatography (eluent: dichloromethane/ethyl acetate), whereby 2-amino-6-tert-butyl benzothiazole (b-3) was obtained as a pale yellow solid (obtained amount: 10.32 g, yield: 69%).

Next, under water-cooling, potassium hydroxide (75.4 g, 1340 mmol) and ethylene glycol (175 mL) was put into a 1 L four-neck flask. After an argon atmosphere was established in the inside of the system, the compound (b-3) (7.8 g, 37.8 mmol) was put thereinto, and the resultant product was allowed to react at 110°C for 18 hours after bubbling was performed with argon to remove the oxygen in the system. The reaction liquid was cooled with water to 40°C or lower, and 2 mol/L hydrochloric acid which was subjected to argon bubbling in advance was added dropwise to the inside of the system to neutralize the reaction liquid (around pH 7). The precipitated white solid was separated by filtration, washed with water, and dried under reduced pressure. And then, the white solid was purified by silica gel column chromatography (eluent: hexane/ethyl acetate), whereby 4-tert-butyl-2-mercaptoaniline (b-4) was obtained as a white solid (obtained amount: 2.39 g, yield: 35%).

Furthermore, acetic acid (872 mg, 14.5 mmol) and 30 mL of acetonitrile were put into a 100 mL three-neck flask, and an argon atmosphere was established in the inside of the system. Under the argon atmosphere, malononitrile (2.4 g, 36.3 mmol) and the compound (b-4) (2.39 g, 13.2 mmol) were added thereto, followed by heating to reflux for 2 hours. After the acetonitrile was removed under reduced pressure, the residues were dissolved in ethyl acetate, then, the organic layer was washed with water and a saturated saline solution, and treated with anhydrous magnesium sulfate. After the magnesium sulfate was separated by filtration, the solvent was removed under reduced pressure, and the residues were purified by silica gel column chromatography (eluent: hexane/ethyl acetate), whereby 2-(6-tert-butylbenzothiazol-2-yl) acetonitrile (b-5) was obtained as a yellow solid (obtained amount: 1.98 g, yield: 65%) .

Subsequently, under argon stream, the compound (b-2) (1.91 g, 3.5 mmol), the compound (b-5) (1.77 g, 7.68 mmol), and toluene (68 mL) were put into a 200 mL three-neck flask, followed by heating to reflux. While heating to reflux, phosphorous oxychloride (2.56mL, 27.4 mmol) was added dropwise thereto using a syringe, followed by further heating to reflux for 2 hours. After the reaction ended, 40mL of dichloromethane and 40 mL of a saturated sodium hydrogen carbonate aqueous solution were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: hexane/dichloromethane), whereby a precursor (b-6) was obtained as a green solid (obtained amount: 1.56 g, yield: 46%) .

Finally, under an argon stream, the precursor (b-6) (1.52 g, 1.57 mmol), toluene (45 mL), triethylamine (4.35 mL, 31.4 mmol), and boron trifluoride diethylether complex (7.88 mL, 62.7 mmol) were put into a 200 mL three-neck flask, followed by heating to reflux for 1 hours. The reaction liquid was cooled with ice, and the precipitated solid was separated by filtration, washed with water, a saturated sodium hydrogen carbonate aqueous solution, a 50% methanol aqueous solution and methanol, and dried under reduced pressure. The obtained residues were dissolved in toluene, and methanol was added thereto to precipitate a solid, whereby a near infrared fluorescent material B was obtained as a dark green solid (obtained amount: 1.25 g, yield: 75%).

¹H-NMR(300MHz,CDCl₃):**δ**7.90ppm(d,2H),7.72-7.69(m,6H),7 .51(dd,2H),7.08(d,2H),4.07(t,4H),1.84(m,4H),1.52(s,18H),1. 35-1.32(m,24H),0.92(t,6H)

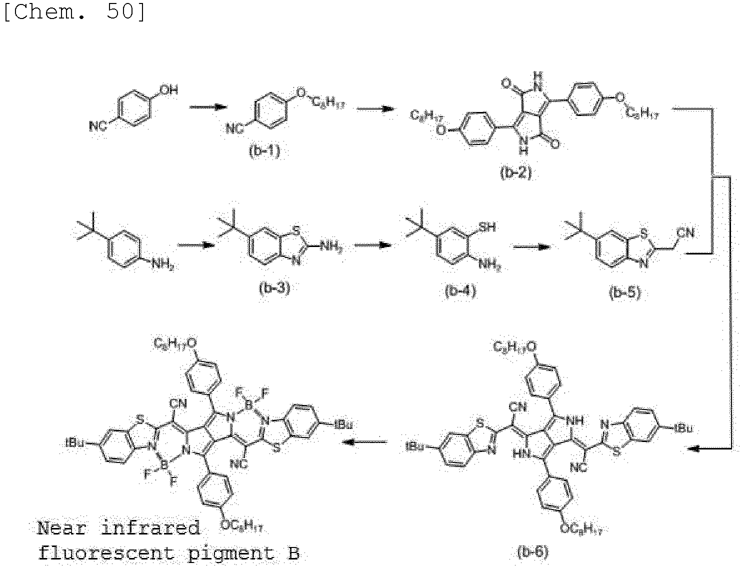

### [Preparation Example 3] Synthesis of Near Infrared Fluorescent Material C

A near infrared fluorescent material C was synthesized according to the method described in Journal of Organic Chemistry, 2011, Vol. 76, pp. 4489-4505.

Under argon stream, 2-ethylthiophene (11.2 g, 100 mmol) and dehydrated THF (80 mL) were put into a 500 mL four-neck flask, followed by stirring at -78°C. n-Butyllithium (68.8 mL, a 1.6mol/L hexane solution) was added dropwise to this solution, followed by stirring at the same temperature for 1 hour, and a dehydrated THF solution (50 mL) of ethyl chloroformate (10.9 mL, 120 mmol) was added dropwise, followed by further stirring for 1 hour. After the temperature of the reaction liquid was returned to room temperature, a saturated ammonium chloride aqueous solution (110 mL) was added thereto, and the resultant product was extracted with dichloromethane. The organic phase was washed sequentially with water and a saturated saline solution, dried over anhydrous magnesium sulfate, and concentrated. The residues were separated and purified by silica gel chromatography (eluent: dichloromethane/cyclohexane = 6/4 (in volume ratio)), whereby 5-ethylthiophene-2-carboxylate (c-1) was obtained as colorless liquid (obtained amount: 15.4 g, yield: 83.7%).

Next, the compound (c-1) (15.0 g, 81.5 mmol) and ethanol (40 mL) were put into a 200 mL four-neck flask, and hydrazine monohydrate (12.2 g, 244 mmol) was added dropwise to this solution, followed by refluxing and stirring for 12 hours. After the reaction liquid was cooled, the solvent was distilled off under reduced pressure, and the residues were dissolved in dichloromethane, washed sequentially with water and a saturated saline solution, dried over anhydrous magnesium sulfate, and concentrated. The residues were recrystallized from cyclohexane, collected by filtration, and dried, whereby 5-ethylthiophene-2-carbohydrazine (c-2) was obtained as a white solid (obtained amount: 8.6 g, yield: 62.1%).

Furthermore, the compound (c-2) (8.5 g, 50 mmol) and 2-hydroxy-4-methoxyacetophenone (7.5 g, 50 mmol) were put into a 50 mL three-neck flask, followed by stirring at 75°C for 1 hour. The residues were recrystallized from dichloromethane/methanol, collected by filtration, and dried, whereby (E)-5-ethyl-N'-(1-(2-hydroxy-4-methoxyphenyl)ethylidene)-t hiophene-2-carbohydrazine (c-3) was obtained as a white solid (obtained amount: 12.4 g, yield: 78%).

Subsequently, the compound (c-3) (9.5 g, 29.8 mmol) and THF (300 mL) were put into a 500 mL four-neck flask and dissolved, and lead acetate (15.9 g, 35.9 mmol) was added to this solution, followed by stirring at room temperature for 1 hour. The reaction liquid was filtered, then, the filtrate was concentrated under reduced pressure, and the obtained residues were extracted with water/dichloromethane. The organic phase was washed sequentially with water and a saturated saline solution, dried over anhydrous magnesium sulfate, and concentrated under reduced pressure. The residues were separated and purified by alumina chromatography (eluent: dichloromethane/cyclohexane = 4/6 (in volume ratio)), whereby (5-ethyl-2-thienyl)(2-acetyl-5-methoxy-1-phenyl) ketone (c-4) was obtained as a white solid (obtained amount: 7.6 g, yield: 88.6%).

Furthermore, under an argon stream, the compound (c-4) (6.6 g, 22.8 mmol), acetic acid (48 mL), and ethanol (240 mL) were put into a 500 mL four-neck flask, followed by stirring at 65°C, and ammonium chloride (1.22 g, 22.8 mmol) and ammonium acetate (10.7 g, 139 mmol) were added to this solution, followed by refluxing and stirring for 30 minutes. The reaction liquid was filtered, then, the filtrate was concentrated under reduced pressure, and the obtained residues were extracted with water/dichloromethane. The organic phase was washed sequentially with water and a saturated saline solution, dried over anhydrous magnesium sulfate, and concentrated under reduced pressure. The residues were separated and purified by silica gel chromatography (eluent: dichloromethane), whereby a compound (c-5) was obtained as a dark blue solid (obtained amount: 2.1 g, yield: 35.2%).

Finally, under an argon stream, the compound (c-5) (2.0 g, 3.8 mmol) and dichloromethane (250 mL) were put into a 2 L flask, followed by stirring at room temperature for 5 minutes . N,N-diisopropylethylamine (1.48 g, 11.5 mmol) and boron trifluoride diethylether complex (3.27 g, 23 mmol) were added dropwise thereto, followed by stirring at room temperature for 1 hour. The reaction liquid was concentrated, and the residues were separated and purified by silica gel column chromatography (eluent: dichloromethane), whereby a near infrared fluorescent material C was obtained as a dark green solid (obtained amount: 1.66 g, yield: 76%).

¹H-NMR (300MHz, CDCl₃/CCl₄=1/1) : δ7.85(s, 2H), δ7.64(d, 2H), δ7.39(s, 1H), δ7.29(s, 2H), δ6.98(m, 4H), δ3.86(s, 6H) , δ2.98(q, 4H), δ1.43(t, 6H)ppm.

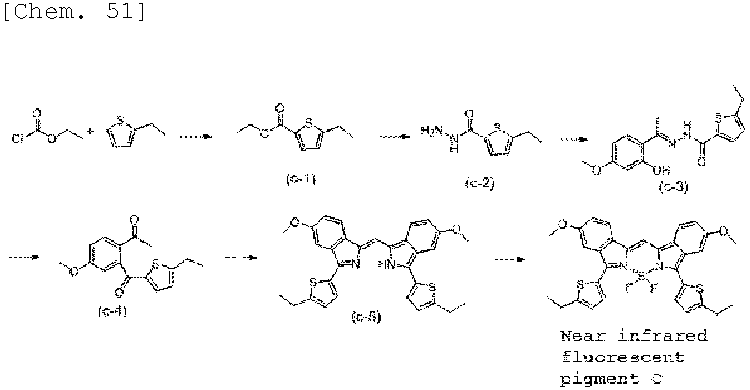

### [Preparation Example 4] Synthesis of Near Infrared Fluorescent Material D

A near infrared fluorescent material D was synthesized according to the method described in Chemistry An Asian Journal, 2013, Vol. 8, pp. 3123-3132.

Under an argon stream, 5-bromo-2-thiophenecarboxaldehyde (19.1 g, 0.1 mol) and ethyl azidoacetate (51.6 g, 0.4 mol) were dissolved in ethanol (800 mL) in a 2 L four-neck flask, and a 20% by mass sodium ethoxide ethanol solution (136 g, 0.4 mol) was slowly added dropwise to the obtained solution at 0°C in an ice bath, followed by stirring for 2 hours. After the reaction ended, a saturated ammonium chloride aqueous solution was added thereto to adjust the pH to be weakly acidic. Furthermore, water was added thereto, and the precipitate was collected by filtration, and dried, whereby ethyl 2-azido-3-(5-bromo-thiophen-2-yl)-acrylate was obtained as a yellow solid (obtained amount: 18.4 g, yield: 61.3%).

Next, ethyl 2-azido-3-(5-bromo-thiophen-2-yl)-acrylate (18.1 g, 60 mmol) was put into a 500 mL egg-plant shaped flask, and dissolved in o-xylene (200 mL), followed by refluxing and stirring for 1.5 hours. After the solution after refluxing and stirring was concentration under reduced pressure, the obtained crude product was recrystallized (solution: hexane and ethyl acetate), then, the resultant product was subjected to suction filtration, and the obtained filtered material was dried, whereby ethyl 2-bromo-4H- thieno [3.2-b] pyrrole-5-carboxylate (d-1) was obtained (obtained amount: 12.1 g, yield: 73.8%).

Furthermore, the compound (d-1) (6.0 g, 22 mmol) was put into a 500 mL flask, and an aqueous solution obtained by dissolving ethanol (200 mL) and sodium hydroxide (12.4 g, 310 mmol) in water (100 mL) was added thereto, followed by refluxing and stirring for 1 hour. After the solution after refluxing and stirring was cooled, a 6 mol/L hydrochloric acid was added thereto to adjust the solution to be acidic, water was added thereto, suction filtration was performed, and the obtained filtered material was vacuum-dried, whereby 2-bromo-4H-thieno[3.2-b]pyrrole-5-carboxylic acid (d-2) was obtained as a gray solid (obtained amount: 4.1 g, yield: 75.8%).

Subsequently, the compound (d-2) (4.0 g, 16.3 mmol) and trifluoroacetic acid (100 mL) were put into a 300 mL three-neck flask, followed by stirring at 40°C. After the compound (d-2) was dissolved, stirring was performed for 15 minutes until the bubbles subsided. Trifluoroacetic anhydride (36 mL) was added to the solution after stirring, and the resultant product was allowed to react at 80°C for 4 hours. After the reaction ended, the reaction liquid was added to a saturated sodium hydrogen carbonate aqueous solution containing ice to neutralize the solution, then, suction filtration was performed, and the resultant product was vacuum-dried, whereby a compound (d-3) was obtained as a crude product.

Furthermore, under an argon stream, the compound (d-3) and dichloromethane (1 L) were put into a 2 L flask, followed by stirring at room temperature for 5 minutes. Triethylamine (12 mL) and boron trifluoride diethylether complex (16 mL) were added dropwise thereto, followed by stirring at room temperature for 1 hour. The reaction liquid was concentrated, and the residues were separated and purified by silica gel column chromatography (eluent: dichloromethane), whereby 2,8-dibromo-11-trifluoromethyl-dithieno[2,3-b][3,2-g]-5,5-difluoro-5-bora-3a,4a-dithio-s-indacene (g- 4) was obtained as a dark bluish green solid (obtained amount: 580 mg, yield: 13.4%).

Finally, under an argon stream, the compound (d-4) (200 mg, 0.378 mmol), 4-methoxyphenyl boronic acid (240 mg, 1.6 mmol), sodium carbonate (120 mg, 1.2 mmol), toluene/THF/water = 1: 1: 1 (60 mL) were put into a 200 mL three-neck flask, and after bubbling for 30 minutes with argon gas, tetrakis(triphenylphosphine)palladium (0) (22 mg) was added thereto, and the resultant product was allowed to a coupling reaction at 80°C for 4 hours. After cooling, water (10 mL) was added to the reaction liquid, and the resultant product was extracted three times with diethyl ether. The obtained organic phase was washed with water and a saturated saline solution, dried over anhydrous magnesium sulfate, and the solvent was concentrated under reduced pressure. The obtained crude product was separated and purified by silica gel chromatography (eluent: toluene/ethyl acetate = 20/1 (in volume ratio)), whereby a near infrared fluorescent material D was obtained as a dark green crystal (obtained amount: 110 mg, yield: 49.8%).

¹H-NMR (300MHz, CD₂Cl₂): δ7.76(d, 4H), δ7.34(s, 2H), δ7.32(s, 2H) , δ7.03(d, 4H) , δ3.91(s, 6H)ppm.

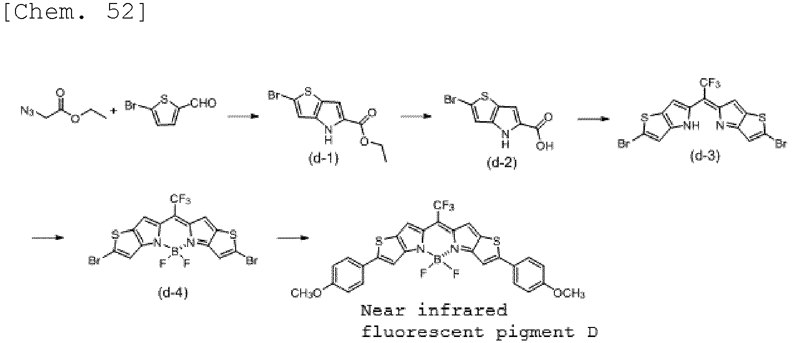

### [Preparation Example 5] Synthesis of Near Infrared Fluorescent Material E

A near infrared fluorescent material E was obtained as a dark green crystal (obtained amount: 94 mg, yield: 46.4%) in the same operation as in Preparation Example 4 except that thiophene-2-boronic acid (205 mg, 1.6 mmol) was used instead of 4-methoxyphenyl boronic acid.

¹H-NMR (300MHz, CD₂Cl₂): δ7.57(m, 4H), δ7.54(d, 2H), δ7.53(s, 2H), δ7.34(s, 2H), δ7.24(m, 2H)ppm

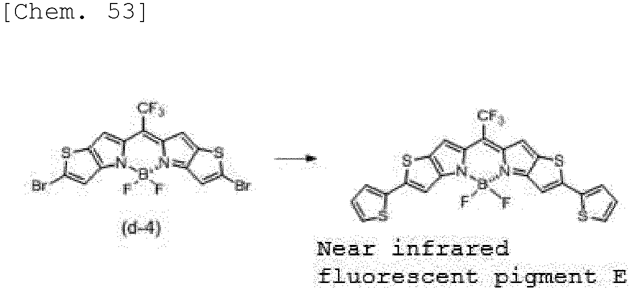

### [Example 4] Characteristic Evaluation of Near Infrared Fluorescent Material B

In Reference Example 1, the operation was performed in the same manner as in Reference Example 1 except that the near infrared fluorescent material B synthesized in Preparation Example 2 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 739 nm, the peak wavelength of the fluorescent spectrum was 758 nm and 833 nm, and the fluorescent quantum yield was 37%. In addition, when the visibility of the film in the near infrared fluorescent detection camera was evaluated, the visibility was excellent.

As the near infrared fluorescent detection camera, a general CMOS camera, which is provided with an LED ring illuminator having excitation light source having a center wavelength of 740 nm, and in which an optical filter passing through light having a wavelength longer than 800 nm is inserted, was used.

In addition, the operation was performed in the same manner as in Reference Example 3 except that the near infrared fluorescent material B synthesized in Preparation Example 2 was used instead of the near infrared fluorescent material A, and the test of the eluting of the material from the obtained material-containing film was performed. As a result, the absorption and the fluorescent derived from the near infrared fluorescent material B could not be confirmed in the test liquid. Accordingly, it is found that the near infrared fluorescent material B was not eluted from the TPU film. Also from the results, it is found that a medical implant which is molded using the resin composition according to the present invention is obtained with high safeness.

### [Reference Example 5] Characteristic Evaluation of Near Infrared Fluorescent Material C

In Reference Example 1, the operation was performed in the same manner as in Reference Example 1 except that the near infrared fluorescent material C synthesized in Preparation Example 3 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 741 nm, the peak wavelength of the fluorescent spectrum was 782 nm, and the fluorescent quantum yield was 14%. In addition, when the visibility of the film in the near infrared fluorescent detection camera was evaluated, the visibility was excellent.

In addition, the operation was performed in the same manner as in Reference Example 3 except that the near infrared fluorescent material C synthesized in Preparation Example 3 was used instead of the near infrared fluorescent material A, and the test of the eluting of the material from the obtained material-containing film was performed. As a result, the absorption and the fluorescent derived from the near infrared fluorescent material C could not be confirmed in the test liquid. Accordingly, it is found that the near infrared fluorescent material C was not eluted from the TPU film. Also from the results, it is found that a medical implant which is molded using the resin composition according to the present invention is obtained with high safeness.

### [Reference Example 6] Characteristic Evaluation of Near Infrared Fluorescent Material D

In Reference Example 1, the operation was performed in the same manner as in Reference Example 1 except that the near infrared fluorescent material D synthesized in Preparation Example 4 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 737 nm, the peak wavelength of the fluorescent spectrum was 765 nm, and the fluorescent quantum yield was 17%. In addition, when the visibility of the film in the near infrared fluorescent detection camera was evaluated, the visibility was excellent.

In addition, the operation was performed in the same manner as in Reference Example 3 except that the near infrared fluorescent material D synthesized in Preparation Example 4 was used instead of the near infrared fluorescent material A, and the test of the eluting of the material from the obtained material-containing film was performed. As a result, the absorption and the fluorescent derived from the near infrared fluorescent material D could not be confirmed in the test liquid. Accordingly, it is found that the near infrared fluorescent material D was not eluted from the TPU film. Also from the results, it is found that a medical implant which is molded using the resin composition according to the present invention is obtained with high safeness.

### [Reference Example 7] Characteristic Evaluation of Near Infrared Fluorescent Material E

In Reference Example 1, the operation was performed in the same manner as in Reference Example 1 except that the near infrared fluorescent material E synthesized in Preparation Example 5 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 741 nm, the peak wavelength of the fluorescent spectrum was 772 nm and the fluorescent quantum yield was 11%. In addition, when the visibility of the film in the near infrared fluorescent detection camera was evaluated, the fluorescent was visible.

In addition, the operation was performed in the same manner as in Reference Example 3 except that the near infrared fluorescent material E synthesized in Preparation Example 5 was used instead of the near infrared fluorescent material A, and the test of the eluting of the material from the obtained material-containing film was performed. As a result, the absorption and the fluorescent derived from the near infrared fluorescent material E could not be confirmed in the test liquid. Accordingly, it is found that the near infrared fluorescent material E was not eluted from the TPU film. Also from the results, it is found that a medical implant which is molded using the resin composition according to the present invention is obtained with high safeness.

### [Example 8] Characteristic Evaluations of Near Infrared Fluorescent Material A and Near Infrared Fluorescent Material B

In Reference Example 1, the operation was performed in the same manner as in Reference Example 1 except that the near infrared fluorescent material A (2.5 mg) and the near infrared fluorescent material B (2.5 mg) synthesized in Preparation Example 2 were used instead of the near infrared fluorescent material A (5 mg) to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 735 nm, the peak wavelength of the fluorescent spectrum was 755 nm and 831 nm, and the fluorescent quantum yield was 32%. In addition, when the visibility of the film in the near infrared fluorescent detection camera was evaluated, the visibility was excellent.

In addition, the operation was performed in the same manner as in Reference Example 3 except that the near infrared fluorescent material A (50 mg) and the near infrared fluorescent material B (50 mg) synthesized in Preparation Example 2 was used instead of the near infrared fluorescent material A (100 mg), and the test of the eluting of the material from the obtained material-containing film was performed. As a result, the absorption and the fluorescent derived from the near infrared fluorescent material E could not be confirmed in the test liquid. Accordingly, it is found that the near infrared fluorescent material E was not eluted from the TPU film. Also from the results, it is found that a medical implant which is molded using the resin composition according to the present invention is obtained with high safeness.

### [Comparative Example 1]

The operation was performed in the same manner as in Reference Example 3 except that a near infrared fluorescent material IR-140 (manufactured by Sigma-Aldrich Co. LLC.) represented by the following formula was used instead of the near infrared fluorescent material A to obtain the material-containing resin, the obtained material-containing resin made to be a film, and the test of the eluting of the material from the obtained material-containing film was performed. As the result, in the obtained test liquid, the peak wavelength derived from IR-140 of the absorption spectrum was 812 nm, and the light emission peak wavelength of the fluorescence spectrum was 846 nm. From the above results, it is found that IR-140 is eluted from the film, and in view of the safeness, the material is difficult to apply to the medical implant.

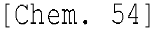

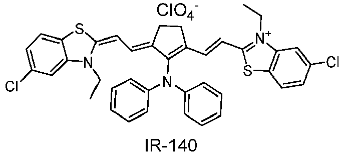

### [Preparation Example 6] Synthesis of Near Infrared Fluorescent Material F

Synthesis of a near infrared fluorescent material F was performed in the following manner based on Organic Letters, 2012, Vol. 4, 2670-2673 and Chmestry A European Journal, 2009, Vol. 15, 4857-4864.

4-tert-Butyl aniline (29.8 g, 0.2 mol) and 6 mol/L hydrochloric acid (100 mL) were put into a 300 mL three-neck flask, and crotonaldehyde (15.4 g, 0.22 mol) was added dropwise thereto while refluxing, followed by further refluxing for 2 hours. The refluxing was stopped, and when being hot, zinc chloride (27.2 g, 0.2 mol) was added to the reaction liquid in the 300 mL three-neck flask, followed by stirring at room temperature overnight. The supernatant was removed, and isopropanol was added to the yellow syrupy residues, followed by refluxing for 2 hours. After the obtained mixture was cooled to 70°C, petroleum ether (200 mL) was added thereto, and the precipitated crystal was collected by filtration, washed with diethyl ether, and dried, whereby zinc complex was obtained. This zinc complex was added to a mixed liquid of water/ammonia (120 mL/60 mL), and the resultant product was extracted three times with diethyl ether diethyl ether (80 mL). The obtained organic layer was dried over anhydrous magnesium sulfate, and concentrated, whereby 6-tert-butyl-2-methyl-quinoline (f-1) was obtained as yellow liquid (obtained amount of 16.2 g, yield of 41%) .

Next, the compound (f-1) (16.0 g, 80 mmol) and chloroform (50 mL) were put into a 200 mL two-neck flask, followed by stirring, and trichloroisocyanuric acid (6.52 g, 28 mmol) was added thereto several times by being divided into several portions. After the obtained mixture was refluxed for 1 hour, the precipitated solid was filtered and washed with chloroform, and the obtained organic layer was extracted three times with 1 mol/L sulfuric acid. The collected aqueous layers were combined, and the resultant product was adjusted to pH 3 with sodium carbonate aqueous solution, and extracted three times with diethyl ether. The organic layer was dried over anhydrous magnesium sulfate, and concentrated, whereby 2-chloromethyl-6-tert-butyl-quinoline (f-2) was obtained as a yellow crystal (obtained amount of 4.8 g, yield of 25.7%).

Furthermore, the compound (f-2) (4.7 g, 20 mmol), sodium cyanide (1.47 g, 30 mmol), a small amount of sodium iodide, and DMF (50 mL) were put into a 100 mL three-neck flask, and the resultant product was allowed to react at 60°C for 2 hours. The reaction liquid was cooled and extracted with water (200 mL)/ethyl acetate (300 mL), and the obtained ethyl acetate layer was further washed with water. The organic layer was dried over anhydrous magnesium sulfate and concentrated, and the resultant product was recrystallized from petroleum ether, whereby 2-(6-tert-butyl-quinolin-2-yl) acetonitrile (f-3) was obtained as a yellow crystal (obtained amount of 1.9 g, yield of 42.4%).

Subsequently, under argon stream, the compound (b-2) (2.18 g, 4.0 mmol) obtained in Preparation Example 2, the compound (f-3) (1.9 g, 8.5 mmol), and dehydrated toluene (68 mL) were put into a 200 mL three-neck flask, followed by heating to reflux. While heating to reflux, phosphorus oxychloride (2.62 mL, 28 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: hexane/dichloromethane), whereby a precursor (f-4) was obtained as a green solid (obtained amount: 1.84 g, yield: 48%) .

Finally, under an argon stream, the precursor (f-4) (1.72 g, 1.8 mmol), toluene (45 mL), triethylamine (4.35 mL, 31.4 mmol), and boron trifluoride diethylether complex (7.88 mL, 62.7 mmol) were put into a 200 mL three-neck flask, followed by heating to reflux for 1 hours. The reaction liquid was cooled with ice, and the precipitated solid was separated by filtration, washed with water, a saturated sodium hydrogen carbonate aqueous solution, a 50% methanol aqueous solution and methanol, and dried under reduced pressure. The obtained residues were dissolved in toluene, and methanol was added thereto to precipitate a solid, whereby a near infrared fluorescent material F was obtained as a green solid (obtained amount: 1.10 g, yield: 58%).

¹H-NMR (300MHz, CDCl₃): δ=8.42(m, 2H), 8.14(d, 2H), 7.74(dd, 2H), 7.72(d, 4H), 7.66(m, 4H), 7.06(m, 4H), 4.08(t, 4H), 1. 85(m,4H),1.53(m,4H),1.45-1.2(m,16H),1.36(s,18H),0.91(t,6H) ppm.

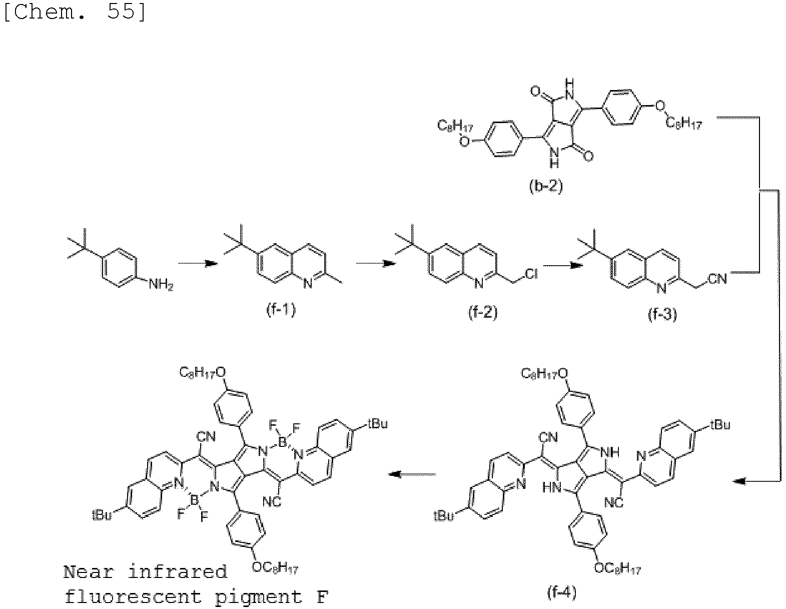

### [Preparation Example 7] Synthesis of Near Infrared Fluorescent Material G

Synthesis of a near infrared fluorescent material G was performed in the following manner based on Organic Letters, 2012, Vol. 4, 2670-2673 and Chmestry A European Journal, 2009, Vol. 15, 4857-4864.

Under argon stream, sodium hydride (60% dispersion, liquid paraffin) (4.0 g, 100 mmol) and dehydrated DMF and (40 mL) were put into a 200 mL three-neck flask, and the resultant product was cooled to 0°C. tert-butyl cyanoacetate (11.9 g, 85 mmol) was slowly added thereto while stirring at the same temperature, followed by stirring at room temperature for 1 hour. Next, 2-chloro-4,6-dimethyl pyrimidine (10 g, 70 mmol) was added thereto, and the resultant product was allowed to react at 90°C for 36 hours. The reaction liquid was poured into a conical flask containing a 5% sodium chloride aqueous solution (200 ml), and the resultant product was neutralized with acetic acid. The precipitated yellow precipitate was collected by filtration, washed with water, and dried, whereby tert-butyl cyano-(4,6-dimethyl-pyrimidin-2-yl) acetate (g-1) was obtained (obtained amount of 9.8 g, yield of 56.9%).

Next, the compound (g-1) (9.8 g, 40 mmol), dichloromethane (60 mL), and trifluoroacetic acid (30 mL) were put into a 300 mL three-neck flask, and the resultant product was allowed to react at room temperature overnight. The reaction liquid was neutralized with a saturated sodium carbonate aqueous solution, and the dichloromethane layer was separated, and washed with water. The organic layer was dried over anhydrous magnesium sulfate and concentrated, and the obtained residues were purified by column chromatography (petroleum ether/ethyl acetate = 1/5), whereby (4,6-dimethyl-pyrimidin-2-yl) acetonitrile (g-2) was obtained as a white crystal (obtained amount of 0.85 g, yield of 14.5%).

Subsequently, under argon stream, the compound (b-2) (1.36 g, 2.5 mmol) obtained in Preparation Example 2, the compound (g-2) (0.81 g, 5.5 mmol), and dehydrated toluene (50 mL) were put into a 200 mL three-neck flask, followed by heating to reflux. While heating to reflux, phosphoryl chloride (2.34 mL, 25 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: dichloromethane/ethyl acetate = 50/1), whereby a precursor (g-3) was obtained as a green solid (obtained amount: 0.54 g, yield: 27%).

Finally, under an argon stream, the precursor (g-3) (522 mg, 0.65 mmol), N,N-diisopropylethylamine (258 mg, 2.0 mmol), and dichloromethane (20 mL) were put into a 100 mL two-neck flask, then, chlorodiphenylborane (600 mg, 3.0 mmol) was added thereto while refluxing, and the resultant product was allowed to react overnight. The reaction liquid was washed with water, and the organic layer was dried over anhydrous magnesium sulfate, and concentrated. The residues were washed with methanol, and purified by column chromatography (eluent: dichloromethane/ethyl acetate = 100/1), whereby a near infrared fluorescent material G was obtained as a green solid (obtained amount: 0.24 g, yield: 32.6%).

¹H-NMR (300MHz, CDCl₃) : δ=7.11 (m, 20H), 6.43(m, 4H), 6.25(s, 2H), 6.02(m, 4H), 3.92(t, 4H), 2.27(s, 6H), 1.78(m, 10H), 1. 5-1.2(m,20H),0.85(t,6H)ppm.

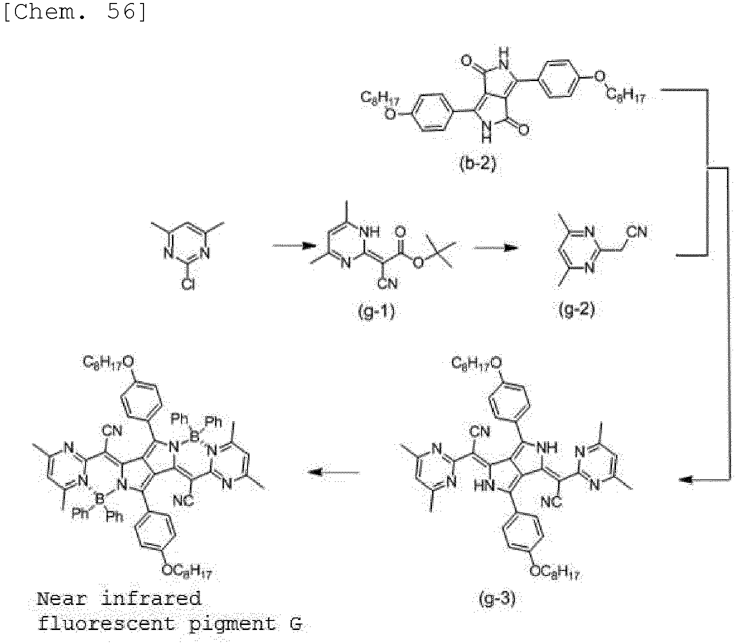

### [Preparation Example 8] Synthesis of Near Infrared Fluorescent Material H

Synthesis of a near infrared fluorescent material H was performed in the following manner based on Organic Letters, 2012, Vol. 4, pp. 2670-2673 and Chmestry A European Journal, 2009, Vol. 15, pp. 4857-4864.

3,6-(4-(2-Ethylhexyl)oxyphenyl)pyrrolo[3,4-c]pyrrole -1,4(2H,5H)-dione (h-2) was obtained as a red solid (obtained amount: 4.6 g) in the same operation as in Preparation Example 2 except that 1-bromo-2-ethylhexane (48 g, 249 mmol) was used instead of 1-bromooctane (48 g, 249 mmol).

Next, 2-amino-4-tert-butylphenol (5.24 g, 31.7 mmol), 2-cyano-acetymytic acid ethyl ester hydrochloride (4.45 g, 33.3 mmol), dichloromethane (30 mL) were put into a 100 mL two-neck flask, followed by refluxing overnight. The reaction liquid was diluted with dichloromethane (100 mL), and the resultant product was washed twice with a 1 mol/L sodium hydroxide aqueous solution. The organic layer was dried over anhydrous magnesium sulfate, and the solvent was removed, whereby (5-tert-butyl-benzoxazol-2-yl)-acetonitrile (h-3) was obtained as yellow liquid (obtained amount of 6.3 g, yield of 88%) .

Subsequently, under argon stream, the compound (h-2) (1.64 g, 3.0 mmol), the compound (h-3) (1.41 g, 6.6 mmol), and dehydrated toluene (50 mL) were put into a 200 mL three-neck flask, followed by heating to reflux. While heating to reflux, phosphoryl chloride (2.34 mL, 25 mmol) was added dropwise thereto using a syringe, followed by further heating to reflux for 2 hours. After the reaction ended, dichloromethane (40 mL) and a saturated sodium hydrogen carbonate aqueous solution (40 mL) were added thereto while ice-cooling, and the resultant product was extracted with dichloromethane. The organic layer was treated with anhydrous magnesium sulfate, the magnesium sulfate was separated by filtration, the solvent was removed under reduced pressure, and silica gel column chromatography (eluent: hexane/ethyl acetate) was used to roughly remove the impurities in the residues. The residues obtained by distilling off the solvent were purified again by silica gel column chromatography (eluent: dichloromethane), whereby a precursor (h-4) was obtained as a bluish green solid (obtained amount: 0.98 g, yield: 35%).

Finally, under an argon stream, the precursor (h-4) (973 mg, 1.0 mmol), N,N-diisopropylethylamine (387 mg, 3.0 mmol), and dichloromethane (30 mL) were put into a 100 mL two-neck flask, then, chlorodiphenylborane (900 mg, 4.5 mmol) was added thereto while refluxing, and the resultant product was allowed to react overnight. The reaction liquid was washed with water, and the organic layer was dried over anhydrous magnesium sulfate, and concentrated. The residues were washed with methanol, and purified by column chromatography (eluent: dichloromethane), whereby a near infrared fluorescent material H was obtained as a green solid (obtained amount: 0.42 g, yield: 35%).

¹H-NMR (300MHz, CDCl₃) : δ=7.11(m, 24H), 6.62(m, 4H), 6.32(m,6H),3.8-3.9(m,4H),2.27(s,6H),1.8(m,2H),1.6-1.3(m,16 H),1.38(s,18H),0.9-1.0(m,12H)ppm.

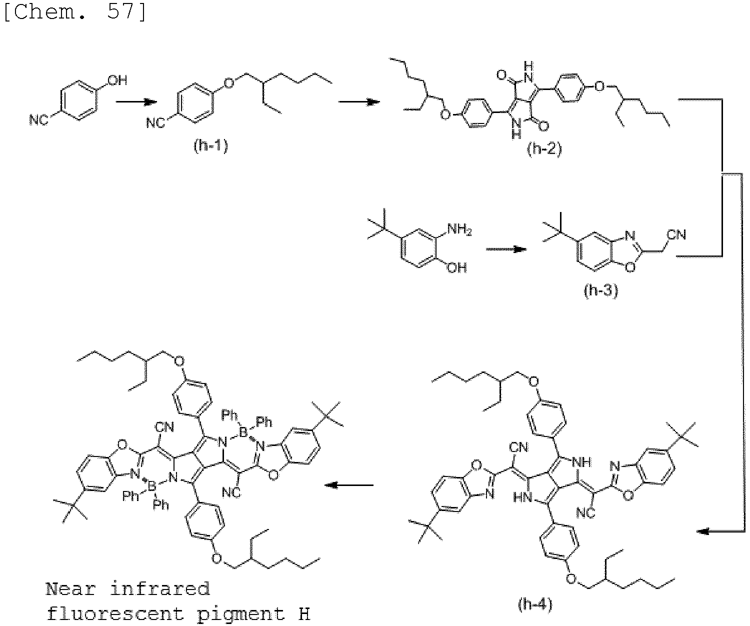

### [Example 9] Characteristic Evaluation Result of Near Infrared Fluorescent Material F

In Reference Example 1, the operation was performed in the same manner as in Reference Example 1 except that the near infrared fluorescent material F synthesized in Preparation Example 6 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 764 nm, the peak wavelength of the fluorescent spectrum was 776 nm and 865 nm, and the fluorescent quantum yield was 38%.

A photograph of which the near infrared fluorescent material F-containing film and a material-free film are arranged taken using the near infrared detection camera disclosed in Example 4 is shown in Fig. 1, and a photograph of which a piece of pork having a thickness of 2 mm is placed over the films taken using the camera is shown in Fig. 2. As considered from the results, the film made of the resin composition according to the present invention has excellent visibility in the near infrared fluorescent camera (Fig. 1), and the fluorescent can be clearly measured even over the pieces of pork having a thickness of 2 mm (Fig. 2).

In addition, the operation was performed in the same manner as in Reference Example 3 except that the near infrared fluorescent material F synthesized in Preparation Example 6 was used instead of the near infrared fluorescent material A, and the test of the eluting of the material from the obtained material-containing film was performed. As a result, the absorption and the fluorescent derived from the near infrared fluorescent material E could not be confirmed in the test liquid. Accordingly, it is found that the near infrared fluorescent material F was not eluted from the TPU film. Also from the results, it is found that a medical implant which is molded using the resin composition according to the present invention is obtained with high safeness.

### [Example 10] Characteristic Evaluation Result of Near Infrared Fluorescent Material G

In Reference Example 1, the operation was performed in the same manner as in Reference Example 1 except that the near infrared fluorescent material G synthesized in Preparation Example 7 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 756 nm, the peak wavelength of the fluorescent spectrum was 778 nm and 870 nm, and the fluorescent quantum yield was 35%. Furthermore, when the visibility of the film in the near infrared fluorescent detection camera was measured, the fluorescent was visible.

In addition, the operation was performed in the same manner as in Reference Example 3 except that the near infrared fluorescent material G synthesized in Preparation Example 7 was used instead of the near infrared fluorescent material A, and the test of the eluting of the material from the obtained material-containing film was performed. As a result, the absorption and the fluorescent derived from the near infrared fluorescent material G could not be confirmed in the test liquid. Accordingly, it is found that the near infrared fluorescent material G was not eluted from the TPU film. Also from the results, it is found that a medical implant which is molded using the resin composition according to the present invention is obtained with high safeness.

### [Example 11] Characteristic Evaluation Result of Near Infrared Fluorescent Material H

In Reference Example 1, the operation was performed in the same manner as in Reference Example 1 except that the near infrared fluorescent material H synthesized in Preparation Example 8 was used instead of the near infrared fluorescent material A to obtain the material-containing resin, and the obtained material-containing resin made to be a film. When the absorption spectrum of the obtained material-containing film, the fluorescent spectrum, and the fluorescent quantum yield were measured in the same manner as in Reference Example 1, the peak wavelength of the absorption spectrum was 744 nm, the peak wavelength of the fluorescent spectrum was 787 nm and 865 nm, and the fluorescent quantum yield was 36%. Furthermore, when the visibility of the film in the near infrared fluorescent detection camera was measured, the fluorescent was visible.

In addition, the operation was performed in the same manner as in Reference Example 3 except that the near infrared fluorescent material H synthesized in Preparation Example 8 was used instead of the near infrared fluorescent material A, and the test of the eluting of the material from the obtained material-containing film was performed. As a result, the absorption and the fluorescent derived from the near infrared fluorescent material H could not be confirmed in the test liquid. Accordingly, it is found that the near infrared fluorescent material H was not eluted from the TPU film. Also from the results, it is found that a medical implant which is molded using the resin composition according to the present invention is obtained with high safeness.

### [Reference Example 12] Camera Visibility Result of Near Infrared Fluorescent Material A

In Reference Example 1, the operation was performed in the same manner as in Reference Example 1 except that 30 mg of the near infrared fluorescent material A was used, and then a film containing the near infrared fluorescent material A having a concentration of 0.03% by mass was obtained (Material A 0.03% film). A photograph of which the material A 0.03% film, a film containing the near infrared fluorescent material A having a concentration of 0.005% by mass which is obtained in Reference Example 1 (Material A 0.005% film), and a film which not contain a material (Material-free film) are arranged taken using the near infrared fluorescent detection camera disclosed in Example 4 is shown in Fig. 3, and a photograph of which a piece of pork having a thickness of 2 mm is placed over the films taken using the camera is shown in Fig. 4. As considered from the results, the film made of the resin composition according to the present invention has excellent visibility in the near infrared fluorescent camera (Fig. 3), and the fluorescent can be clearly measured even over the pieces of pork having a thickness of 2 mm (Fig. 4).

### [Example 13] Camera Visibility Result of Near Infrared Fluorescent Material B

In Reference Example 1, the operation was performed in the same manner as in Reference Example 1 except that the near infrared fluorescent material B (30 mg) synthesized in Preparation Example 2 instead of the near infrared fluorescent material A (5 mg) was used and then, a film (material B 0.03% film) containing the near infrared fluorescent material B having a concentration of 0.03% by mass was obtained. A photograph of which the material B 0.03% film, a film containing the near infrared fluorescent material B having a concentration of 0.005% by mass which is obtained in Example 4 (Material B 0.005% film), and a film which not contain a material (Material-free film) are arranged taken using the near infrared fluorescent detection camera disclosed in Example 4 is shown in Fig. 5, and a photograph of which a piece of pork having a thickness of 2 mm is placed over the films taken using the camera is shown in Fig. 6. As considered from the results, the film made of the resin composition according to the present invention has excellent visibility in the near infrared fluorescent camera (Fig. 5), and the fluorescent can be clearly measured even over the pieces of pork having a thickness of 2 mm (Fig. 6).

### [Example 14] Evaluation of Near Infrared Fluorescent Material B-Containing Polystyrene Film

In Example 4, A polystyrene film having a material concentration of 0.005% by mass was fabricated in the same manner as in Example 4 except that polystyrene (DIC styrene (trade mark) LP-6000, manufactured by DIC Corporation) was used instead of the TPU pellets, and the kneading temperature was 230°C, then, the same evaluation as in Example 4 was performed, and the results are summarized in Tables 1 and 2.

### [Example 15] Evaluation of Near Infrared Fluorescent Material B-Containing PET Film

A PET film having a material concentration of 0.005% by mass was fabricated in the same manner as in Example 4 except that PET (Byron (trade mark) SI-173C, manufactured by Toyobo Co., Ltd.) was used instead of the TPU pellets, and the kneading temperature was 210°C, then, the same evaluation as in Example 4 was performed, and the results are summarized in Tables 1 and 2.

### [Example 16] Evaluation of Near Infrared Fluorescent Material B-Containing Polyethylene Film

A polyethylene film having a material concentration of 0.005% by mass was fabricated in the same manner as in Example 4 except that polyethylene (UBE Polyethylene (trade mark) F522N, manufactured by UBE INDUSTRIES, LTD.) was used instead of the TPU pellets, and the kneading temperature was 130°C, then, the same evaluation as in Example 4 was performed, and the results are summarized in Tables 1 and 2.

### [Example 17] Evaluation of Near Infrared Fluorescent Material B-Containing PP Film

A PP film having a material concentration of 0.005% by mass was fabricated in the same manner as in Example 4 except that PP pellets (product name: PC630A, manufactured by SunAllomer Ltd.) was used instead of the TPU pellets, then, the same evaluation as in Example 4 was performed, and the results are summarized in Tables 1 and 2.

### [Example 18] Evaluation of Near Infrared Fluorescent Material F-Containing PP Film

In Example 4, a PP film having a material concentration of 0.005% by mass was fabricated in the same manner as in Example 4 except that near infrared fluorescent material F was used instead of the near infrared fluorescent material B, PP pellets (product name: PC630A, manufactured by SunAllomer Ltd.) was used instead of the TPU pellets, and then, the same evaluation as in Example 4 was performed, and the results are summarized in Tables 1 and 2.

The results of Reference Examples 1, 2 and 5 to 7, Examples 4, 8 to 11, and 14 to 18 are summarized in Table 1. In a section of the "Camera visibility" in Table 1, "A" indicates an excellent visibility, "B" indicates a good visibility, and "C" indicates a normal visibility, and "D" indicates a poor visibility, respectively. From Table 1, it is found that any of the films obtained from the resin composition of the present invention has a light emission of 700 nm or longer, has a high quantum yield, and has an excellent visibility in the near infrared camera.

**[Table 1]**

| | Materials | Resins | Absorption peak | Fluorescence peak | Quantum yield | Camera visibility |
|---|---|---|---|---|---|---|
| Reference Example 1 | A | TPU | 730 nm | 755 nm 823 nm | 26% | A |
| Reference Example 2 | A | pp | 730 nm | 810 nm | 24% | B |
| Example 4 | B | TPU | 739 nm | 758 nm 833 nm | 37% | A |
| Reference Example 5 | C | TPU | 741 nm | 782 nm | 14% | B |
| Reference Example 6 | D | TPU | 737 nm | 765 nm | 17% | B |
| Reference Example 7 | E | TPU | 741 nm | 772 nm | 11% | C |
| Example 8 | A, B | TPU | 735 nm | 755 nm 831 nm | 32% | A |
| Example 9 | F | TPU | 764 nm | 776 nm 865 nm | 38% | A |
| Example 10 | G | TPU | 756 nm | 778 nm 870 nm | 35% | A |
| Example 11 | H | TPU | 744 nm | 787 nm 865 nm | 36% | A |
| Example 14 | B | PS | 738 nm | 756 nm 834 nm | 36% | A |
| Example 15 | B | PET | 738 nm | 753 nm 833 nm | 34% | A |
| Example 16 | B | PE | 737 nm | 754 nm 834 nm | 39% | A |
| Example 17 | B | pp | 736 nm | 751 nm 825 nm | 46% | A |
| Example 18 | F | pp | 767 nm | 774 nm 870 nm | 35% | A |

The results of the eluting tests of Examples 4, 8 to 11 and 14 to 18, Reference Examples 3 and 5 to 7, and Comparative Example 1 are shown in Table 2. From Table 2, in the film obtained from the resin composition of the present invention, since the light emission caused by the near infrared fluorescent material was not measured from the eluate, there is no eluting of the near infrared fluorescent material. Accordingly, it is confirmed that the film is a safety film which capable of using as a medical purpose. With respect to this, in the film obtained from the resin composition of Comparative Example 1, the light emission caused by near infrared fluorescent material was measured from the eluate. Accordingly, it is confirmed that the material elutes.

**[Table 2]**

| | Materials | Presence or absence of material elution |
|---|---|---|
| Reference Example 3 | A | Absence |
| Example 4 | B | Absence |
| Reference Example 5 | C | Absence |
| Reference Example 6 | D | Absence |
| Reference Example 7 | E | Absence |
| Example 8 | A, B | Absence |
| Example 9 | F | Absence |
| Example 10 | G | Absence |
| Example 11 | H | Absence |
| Example 14 | B | Absence |
| Example 15 | B | Absence |
| Example 16 | B | Absence |
| Example 17 | B | Absence |
| Example 18 | F | Absence |
| Comparative Example 1 | IR-140 | Presence |

## Claims

1. A resin composition, containing:
a near infrared fluorescent material; and
a resin,
wherein the near infrared fluorescent material is one or more of compounds selected from the group consisting of compounds represented by the following General Formula (I₃-1), (I₃-2), (I₃-3), (I₃-4), (I₃-5), (I₃-6), (I₄-1), (I₄-2), (I₄-3), (I₄-4), (I₄-5) and (I₄-6),
the content of the near infrared fluorescent material in the resin composition is within the range of 0.0001% to 0.5% by mass, and
the resin composition has a maximum fluorescence wavelength of 650 nm or longer: 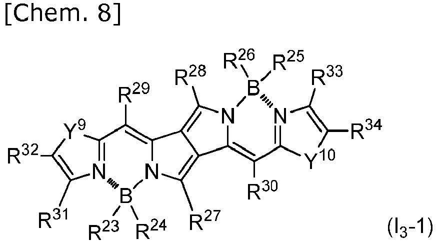
wherein each of R²³, R²⁴, R²⁵, and R²⁶ independently represents a halogen atom, a C₁₋₂₀ alkyl group, a C₁₋₂₀ alkoxy group, an aryl group, or a heteroaryl group;
each of R²⁷ and R²⁸ independently represents a hydrogen atom, a halogen atom, a C₁₋₂₀ alkyl group, a C₁₋₂₀ alkoxy group, an aryl group, or a heteroaryl group;
each of R²⁹ and R³⁰ independently represents a hydrogen atom or an electron withdrawing group selected from a methyl halide group, a nitro group, a cyano group, an aryl group, a heteroaryl group, an alkynyl group, an alkenyl group, a substituent having a carbonyl group, a sulfoxide group, a sulfonyl group, an alkoxymethyl group, and an aminomethyl group;
each of Y⁹ and Y¹⁰ independently represents a sulfur atom, an oxygen atom, a nitrogen atom, or a phosphorus atom;
(p4) each of R³¹ and R³² independently represents a hydrogen atom, a halogen atom, a C₁₋₂₀ alkyl group, a C₁₋₂₀ alkoxy group, an aryl group, or a heteroaryl group, or
(p5) R³¹ and R³² together form an aromatic 5-membered ring which may have a substituent or an aromatic 6-membered ring which may have a substituent; and
(q4) each of R³³ and R³⁴ independently represents a hydrogen atom, a halogen atom, a C₁₋₂₀ alkyl group, a C₁₋₂₀ alkoxy group, an aryl group, or a heteroaryl group, or
(q5) R³³ and R³⁴ together form an aromatic 5-membered ring which may have a substituent or an aromatic 6-membered ring which may have a substituent; 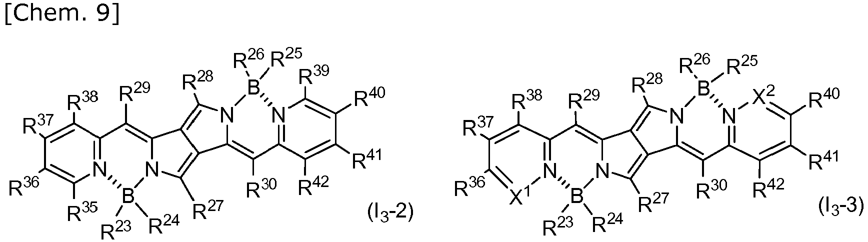 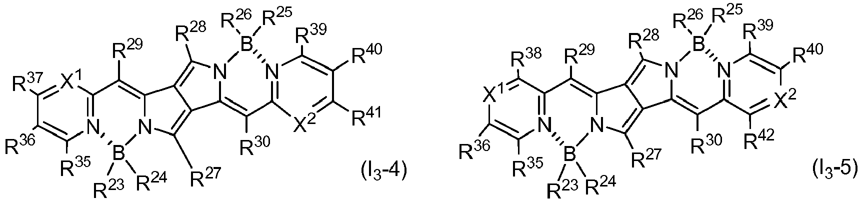 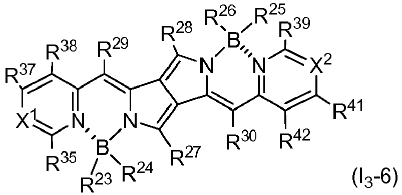
wherein each of R²³ to R³⁰ is the same as in Formula (I₃-1);
each of X¹ and X² independently represents a nitrogen atom or a phosphorus atom;
(p6) each of R³⁵, R³⁶, R³⁷, and R³⁸ independently represents a hydrogen atom, a halogen atom, a C₁₋₂₀ alkyl group, a C₁₋₂₀ alkoxy group, an aryl group, or a heteroaryl group,
(p7) R³⁵ and R³⁶ together form an aromatic 5-membered ring which may have a substituent or an aromatic 6-membered ring which may have a substituent, and each of R³⁷ and R³⁸ independently represents a hydrogen atom, a halogen atom, a C₁₋₂₀ alkyl group, a C₁₋₂₀ alkoxy group, an aryl group, or a heteroaryl group,
(p8) R³⁶ and R³⁷ together form an aromatic 5-membered ring which may have a substituent or an aromatic 6-membered ring which may have a substituent, and each of R³⁵ and R³⁸ independently represents a hydrogen atom, a halogen atom, a C₁₋₂₀ alkyl group, a C₁₋₂₀ alkoxy group, an aryl group, or a heteroaryl group, or
(p9) R³⁷ and R³⁸ together form an aromatic 5-membered ring which may have a substituent or an aromatic 6-membered ring which may have a substituent, and each of R³⁵ and R³⁶ independently represents a hydrogen atom, a halogen atom, a C₁₋₂₀ alkyl group, a C₁₋₂₀ alkoxy group, an aryl group, or a heteroaryl group; and
(q6) each of R³⁹, R⁴⁰, R⁴¹, and R⁴² independently represents a hydrogen atom, a halogen atom, a C₁₋₂₀ alkyl group, a C₁₋₂₀ alkoxy group, an aryl group, or a heteroaryl group,
(q7) R³⁹ and R⁴⁰ together form an aromatic 5-membered ring which may have a substituent or an aromatic 6-membered ring which may have a substituent, and each of R⁴¹ and R⁴² independently represents a hydrogen atom, a halogen atom, a C₁₋₂₀ alkyl group, a C₁₋₂₀ alkoxy group, an aryl group, or a heteroaryl group,
(q8) R⁴⁰ and R⁴¹ together form an aromatic 5-membered ring which may have a substituent or an aromatic 6-membered ring which may have a substituent, and each of R³⁹ and R⁴² independently represents a hydrogen atom, a halogen atom, a C₁₋₂₀ alkyl group, a C₁₋₂₀ alkoxy group, an aryl group, or a heteroaryl group, or
(q9) R⁴¹ and R⁴² together form an aromatic 5-membered ring which may have a substituent or an aromatic 6-membered ring which may have a substituent, and each of R³⁹ and R⁴⁰ independently represents a hydrogen atom, a halogen atom, a C₁₋₂₀ alkyl group, a C₁₋₂₀ alkoxy group, an aryl group, or a heteroaryl group; 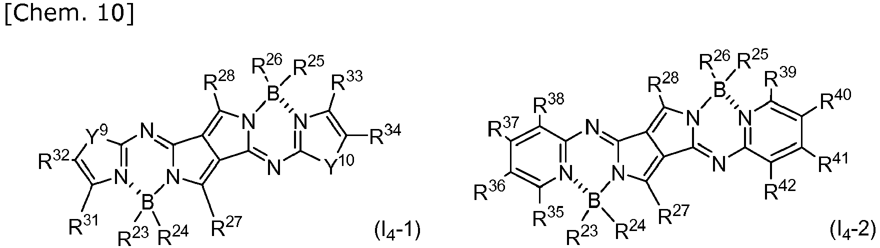 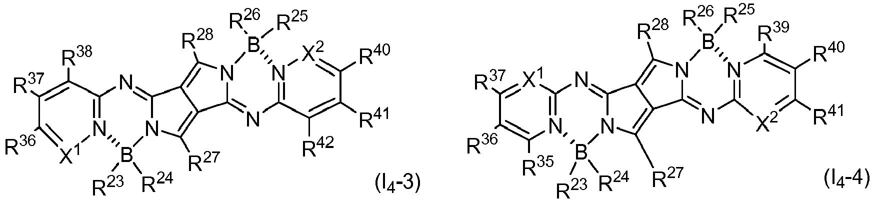 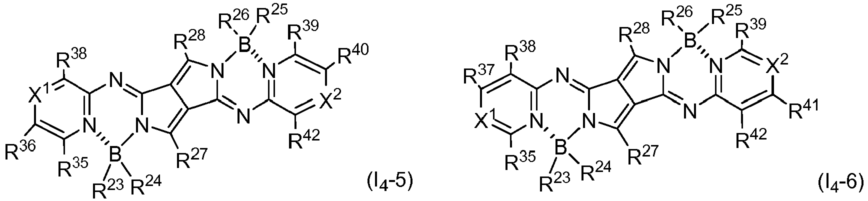
wherein, in Formulas (I₄-1) to (I₄-6), each of R²³ to R²⁸ is the same as in Formula (I₃-1), and in Formula (I₄-1), each of R³¹ to R³⁴, Y⁹, and Y¹⁰ is the same as in Formula (I₃-1), in Formulas (I₄-2) to (I₄-6),each of R³⁵ to R⁴² is the same as in Formula (I₃-2), and in Formulas (I₄-3) to (I₄-6),each of X¹ and X² is the same as in Formula (I₃-3).

2. The resin composition according to Claim 1, containing one or more of compounds selected from the group consisting of compounds represented by any one of the following General Formulas (I₃-7) to (I₃-9) and (14-7) to (I₄-9): 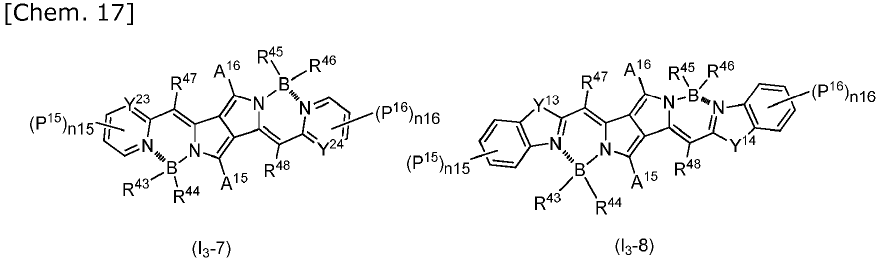 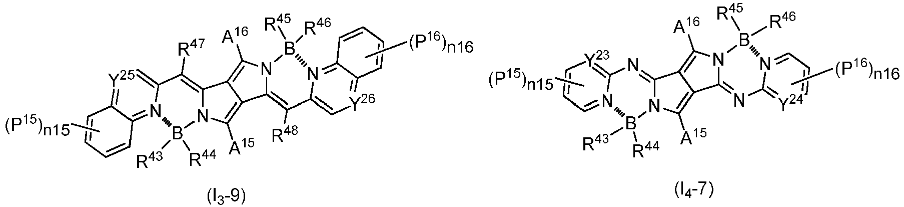 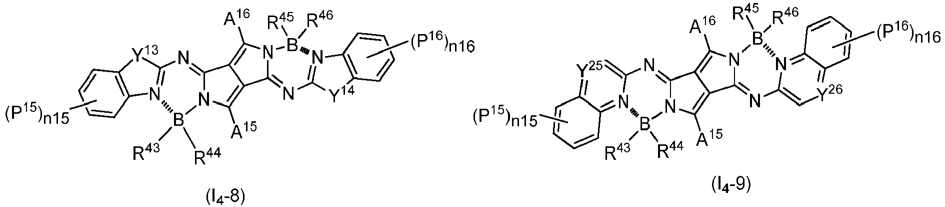
wherein each of Y²³ and Y²⁴ independently represents a carbon atom or a nitrogen atom;
each of Y¹³ and Y¹⁴ independently represents an oxygen atom or a sulfur atom;
each of Y²⁵ and Y²⁶ independently represents a carbon atom or a nitrogen atom;
each of R⁴⁷ and R⁴⁸ independently represents a hydrogen atom or an electron withdrawing group selected from a methyl halide group, a nitro group, a cyano group, an aryl group, a heteroaryl group, an alkynyl group, an alkenyl group, a substituent having a carbonyl group, a sulfoxide group, a sulfonyl group, an alkoxymethyl group, and an aminomethyl group;
each of R⁴³, R⁴⁴, R⁴⁵, and R⁴⁶ represents a halogen atom or an aryl group which may have a substituent;
each of P¹⁵ and P¹⁶ independently represents a halogen atom, a C₁₋₂₀ alkyl group, a C₁₋₂₀ alkoxy group, an amino group, a monoalkylamino group, or a dialkylamino group;
each of n15 and n16 independently represents an integer of 0 to 3; and
each of A¹⁵ and A¹⁶ independently represents a phenyl group which may have one to three substituents selected from the group consisting of a halogen atom, a C₁₋₂₀ alkyl group, a C₁₋₂₀ alkoxy group, an amino group, a monoalkylamino group, or a dialkylamino group.

3. The resin composition according to Claim 1 or 2, containing one or more of compounds selected from the group consisting of compounds represented by any one of the following General Formulas (6-1) to (6-12) and (7-1) to (7-12): 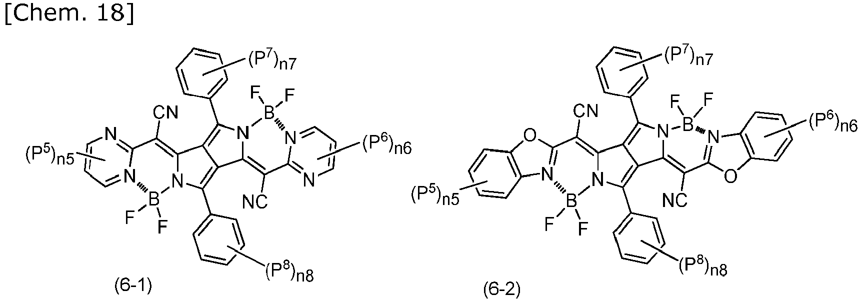 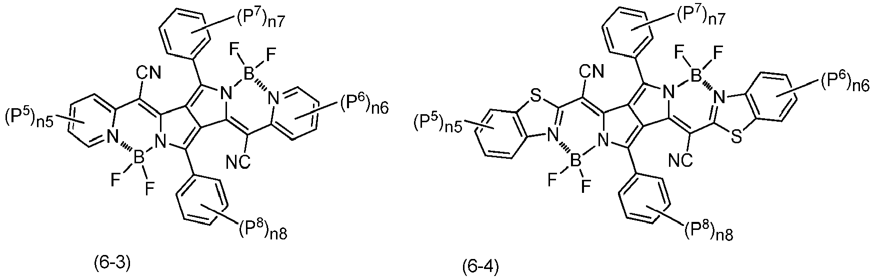 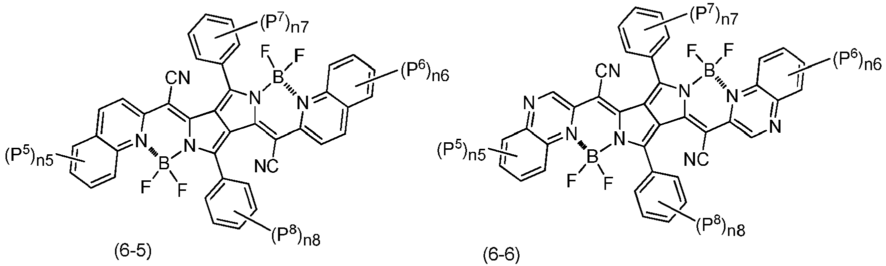 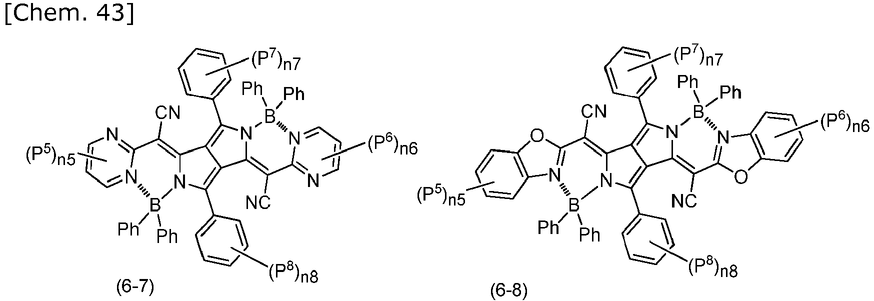 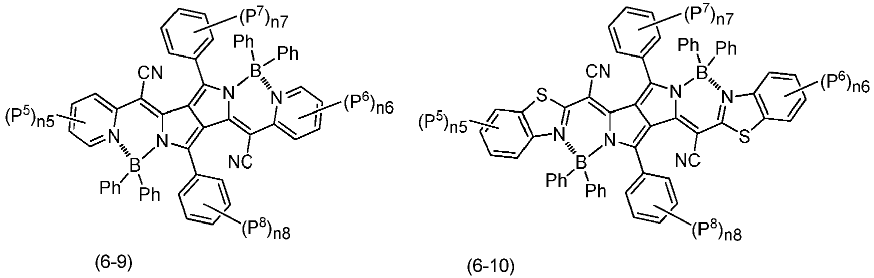 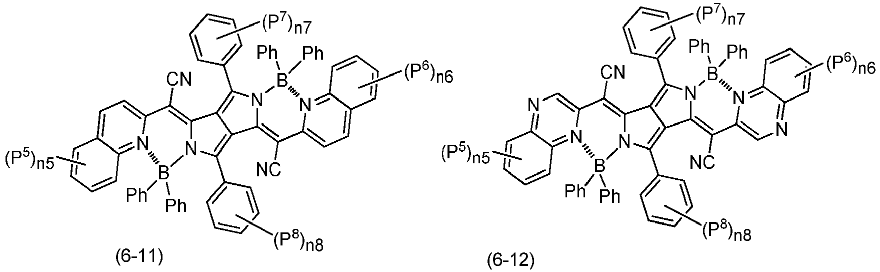 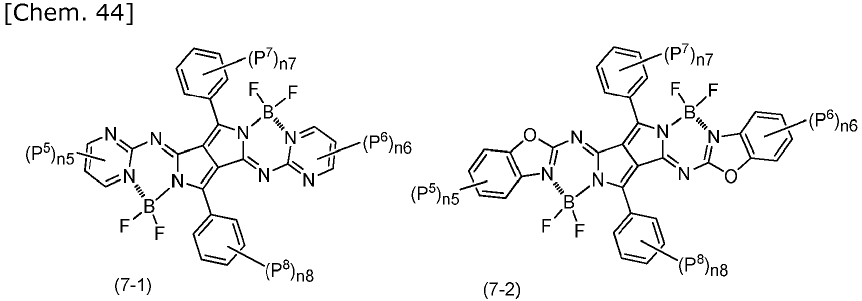 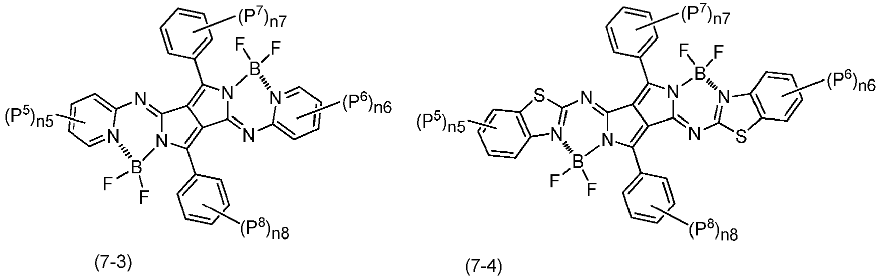 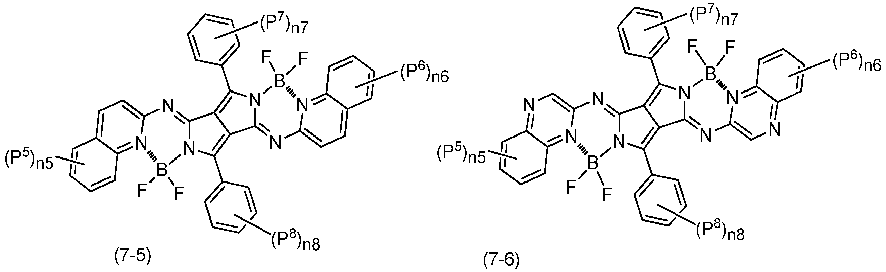 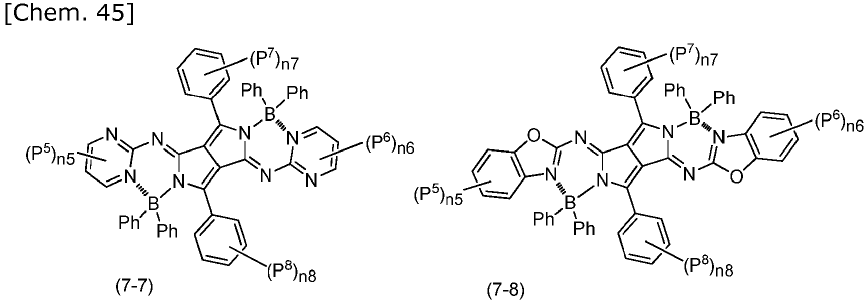 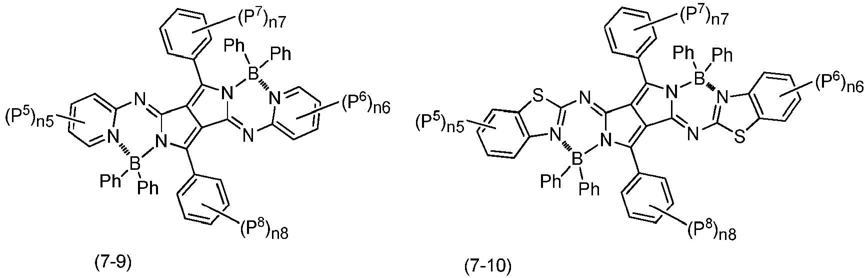 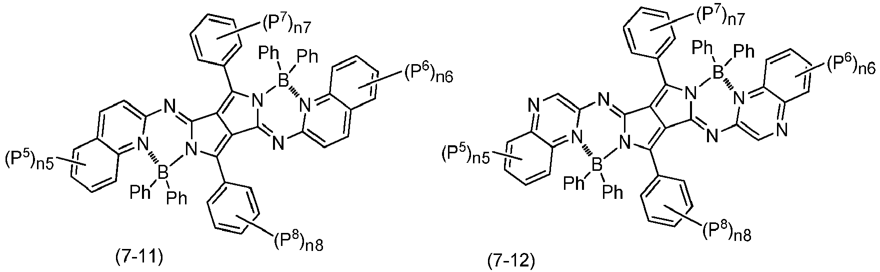
wherein each of P⁵ to P⁸ independently represents a halogen atom, a C₁₋₂₀ alkyl group, a C₁₋₂₀ alkoxy group, an amino group, a monoalkylamino group, or a dialkylamino group;
each of n5 to n8 independently represents an integer of 0 to 3.

4. The resin composition according to any one of Claims 1 to 3,
wherein the resin is a thermoplastic resin.

5. The resin composition according to any one of Claims 1 to 4,
wherein the near infrared fluorescent material and the resin are meltkneaded.

6. The resin composition according to any one of Claims 1 to 5,
wherein the maximum fluorescence wavelength is 700 nm or longer.

7. The resin composition according to any one of Claims 1 to 6, which is used as a medical material.

8. A molded article obtained by processing the resin composition according to any one of Claims 1 to 7.

9. The molded article according to Claim 8,
wherein at least a part of the molded article is a medical tool that is used in the body of a patient.

## Patentansprüche

1. Harzzusammensetzung, die enthält:
ein im nahen Infrarot fluoreszierendes Material; und
ein Harz,
wobei das im nahen Infrarot fluoreszierende Material eine oder mehrere Verbindungen ist, ausgewählt aus der Gruppe bestehend aus Verbindungen, die durch die folgende allgemeine Formel (I₃-1), (I₃-2), (I₃-3), (I₃-4), (I₃-5), (I₃-6), (I₄-1), (I₄-2), (I₄-3), (I₄-4), (I₄-5) und (I₄-6) dargestellt werden,
der Gehalt des im nahen Infrarot fluoreszierenden Materials in der Harzzusammensetzung innerhalb des Bereichs von 0,0001 bis 0,5 Massen-% liegt, und
die Harzzusammensetzung eine maximale Fluoreszenzwellenlänge von 650 nm oder mehr aufweist: 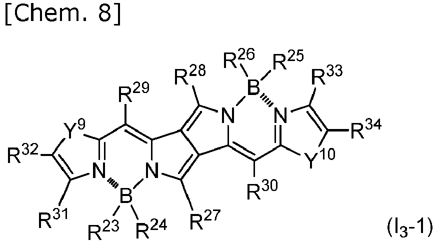
wobei R²³, R²⁴, R²⁵ und R²⁶ jeweils unabhängig ein Halogenatom, eine C₁₋₂₀-Alkylgruppe, eine C₁₋₂₀-Alkoxygruppe, eine Arylgruppe oder eine Heteroarylgruppe darstellt;
R²⁷ und R²⁸ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen;
R²⁹ und R³⁰ jeweils unabhängig ein Wasserstoffatom oder eine elektronenziehende Gruppe ausgewählt aus einer Methylhalogenidgruppe, einer Nitrogruppe, einer Cyanogruppe, einer Arylgruppe, einer Heteroarylgruppe, einer Alkinylgruppe, einer Alkenylgruppe, einem Substituenten mit einer Carbonylgruppe, einer Sulfoxidgruppe, einer Sulfonylgruppe, einer Alkoxymethylgruppe und einer Aminomethylgruppe, darstellen;
Y⁹ und Y¹⁰ jeweils unabhängig ein Schwefelatom, ein Sauerstoffatom, ein Stickstoffatom oder ein Phosphoratom darstellen;
(p4) R³¹ und R³² jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen, oder
(p5) R³¹ und R³² zusammen einen aromatischen 5-gliedrigen Ring, der einen Substituenten aufweisen kann, oder einen aromatischen 6-gliedrigen Ring, der einen Substituenten aufweisen kann, bilden; und
(q4) R³³ und R³⁴ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen, oder
(q5) R³³ und R³⁴ zusammen einen aromatischen 5-gliedrigen Ring, der einen Substituenten aufweisen kann, oder einen aromatischen 6-gliedrigen Ring, der einen Substituenten aufweisen kann, bilden; 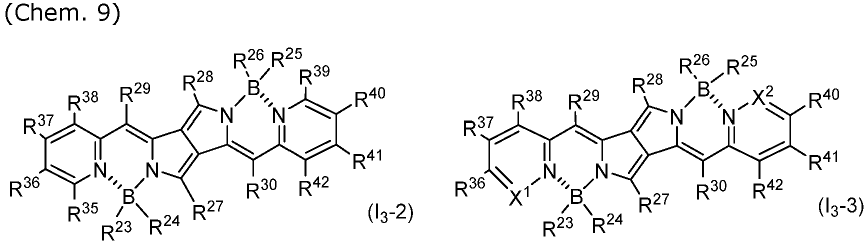 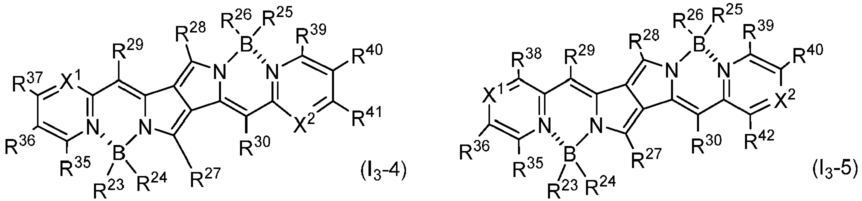 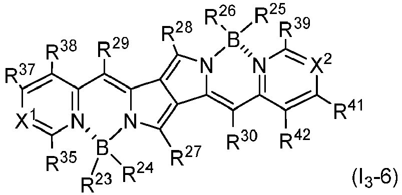
wobei R²³ bis R³⁰ jeweils die gleiche Bedeutung haben wie in Formel (I³-1);
X¹ und X² jeweils unabhängig ein Stickstoffatom oder ein Phosphoratom darstellt;
(p6) R³⁵, R³⁶, R³⁷ und R³⁸ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen,
(p7) R³⁵ und R³⁶ zusammen einen aromatischen 5-gliedrigen Ring, der einen Substituenten aufweisen kann, oder einen aromatischen 6-gliedrigen Ring, der einen Substituenten aufweisen kann, bilden, und R³⁷ und R³⁸ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen,
(p8) R³⁶ und R³⁷ zusammen einen aromatischen 5-gliedrigen Ring, der einen Substituenten aufweisen kann, oder einen aromatischen 6-gliedrigen Ring, der einen Substituenten aufweisen kann, bilden, und R³⁵ und R³⁸ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen, oder
(p9) R³⁷ und R³⁸ zusammen einen aromatischen 5-gliedrigen Ring, der einen Substituenten aufweisen kann, oder einen aromatischen 6-gliedrigen Ring, der einen Substituenten aufweisen kann, bilden, und R³⁵ und R³⁶ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen; und
(q6) R³⁹, R⁴⁰, R⁴¹ und R⁴² jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen,
(q7) R³⁹ und R⁴⁰ zusammen einen aromatischen 5-gliedrigen Ring, der einen Substituenten aufweisen kann, oder einen aromatischen 6-gliedrigen Ring, der einen Substituenten aufweisen kann, bilden, und R⁴¹ und R⁴² jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen,
(q8) R⁴⁰ und R⁴¹ zusammen einen aromatischen 5-gliedrigen Ring, der einen Substituenten aufweisen kann, oder einen aromatischen 6-gliedrigen Ring, der einen Substituenten aufweisen kann, bilden, und R³⁹ und R⁴² jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen, oder
(q9) R⁴¹ und R⁴² zusammen einen aromatischen 5-gliedrigen Ring, der einen Substituenten aufweisen kann, oder einen aromatischen 6-gliedrigen Ring, der einen Substituenten aufweisen kann, bilden, und R³⁹ und R⁴⁰ jeweils unabhängig ein Wasserstoffatom, ein Halogenatom, eine C₁₋₂₀-Alkylgruppe, eine C₁₋₂₀-Alkoxygruppe, eine Arylgruppe oder eine Heteroarylgruppe darstellen; 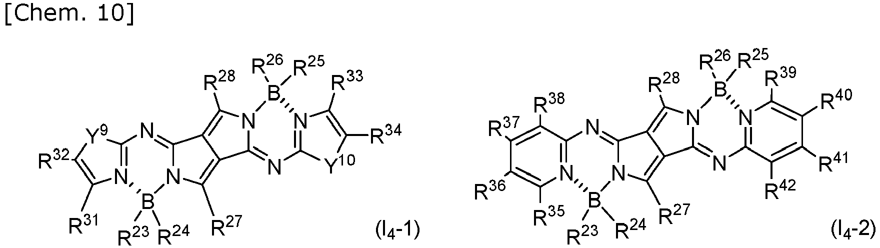 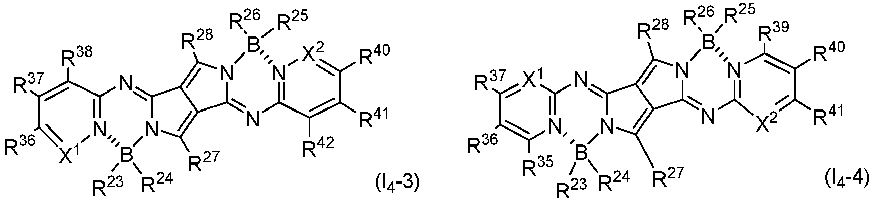 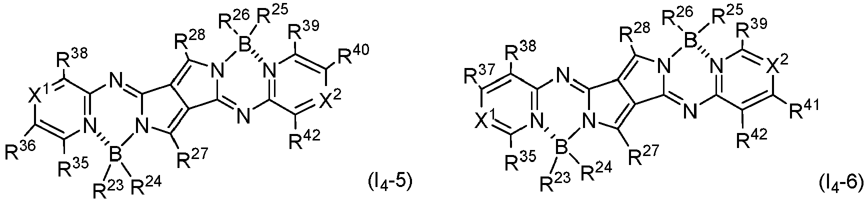
wobei in den Formeln (I₄-1) bis (I₄-6) R²³ bis R²⁸ jeweils die gleiche Bedeutung haben wie in der Formel (I³-1), und in der Formel (I₄-1) R³¹ bis R³⁴, Y⁹ und Y¹⁰ jeweils die gleiche Bedeutung haben wie in der Formel (I³-1), in den Formeln (I4-2) bis (I₄-6) R³⁵ bis R⁴² jeweils die gleiche Bedeutung haben wie in der Formel (I₃-2), und in den Formeln (I₄-3) bis (I₄-6) X¹ und X² jeweils die gleiche Bedeutung haben wie in der Formel (I₃-3).

2. Harzzusammensetzung nach Anspruch 1, die eine oder mehrere Verbindungen enthält, ausgewählt aus der Gruppe bestehend aus Verbindungen, die durch eine der folgenden allgemeinen Formeln (I₃-7) bis (I₃-9) und (I₄-7) bis (I₄-9) dargestellt werden: 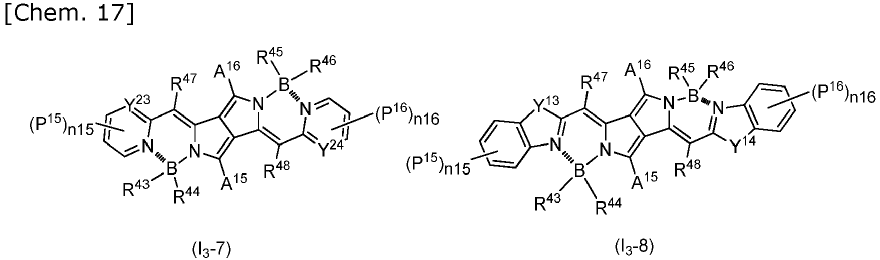 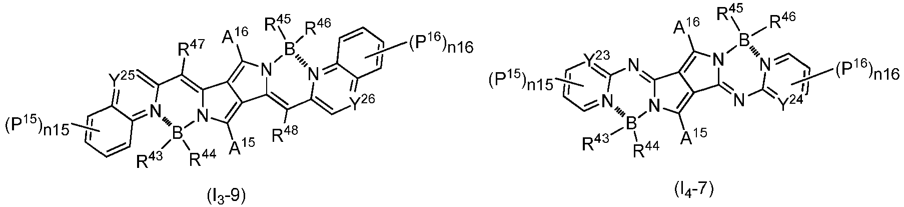 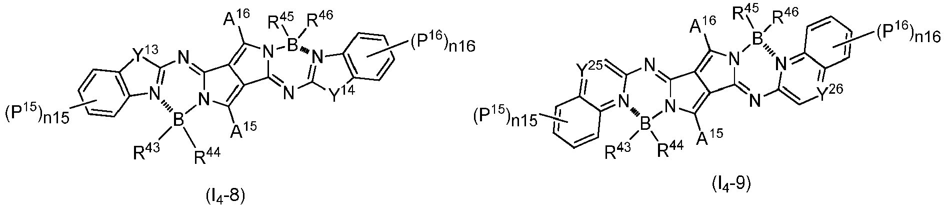
wobei Y²³ und Y²⁴ jeweils unabhängig ein Kohlenstoffatom oder ein Stickstoffatom darstellt;
Y¹³ und Y¹⁴ jeweils unabhängig ein Sauerstoffatom oder ein Schwefelatom darstellt;
Y²⁵ und Y²⁶ jeweils unabhängig ein Kohlenstoffatom oder ein Stickstoffatom darstellen;
R⁴⁷ und R⁴⁸ jeweils unabhängig ein Wasserstoffatom oder eine elektronenziehende Gruppe ausgewählt aus einer Methylhalogenidgruppe, einer Nitrogruppe, einer Cyanogruppe, einer Arylgruppe, einer Heteroarylgruppe, einer Alkinylgruppe, einer Alkenylgruppe, einem Substituenten mit einer Carbonylgruppe, einer Sulfoxidgruppe, einer Sulfonylgruppe, einer Alkoxymethylgruppe und einer Aminomethylgruppe, darstellen;
R⁴³, R⁴⁴, R⁴⁵ und R⁴⁶ jeweils ein Halogenatom oder eine Arylgruppe, die einen Substituenten aufweisen kann, darstellt;
P¹⁵ und P¹⁶ jeweils unabhängig ein Halogenatom, eine C₁₋₂₀-Alkylgruppe, eine C₁₋₂₀-Alkoxygruppe, eine Aminogruppe, eine Monoalkylaminogruppe oder eine Dialkylaminogruppe darstellen;
n15 und n16 jeweils unabhängig eine ganze Zahl von 0 bis 3 darstellen; und
A¹⁵ und A¹⁶ jeweils unabhängig eine Phenylgruppe darstellen, die einen bis drei Substituenten aufweisen kann, die ausgewählt sind aus der Gruppe bestehend aus einem Halogenatom, einer C₁₋₂₀-Alkylgruppe, einer C₁₋₂₀-Alkoxygruppe, einer Aminogruppe, einer Monoalkylaminogruppe oder einer Dialkylaminogruppe.

3. Harzzusammensetzung nach Anspruch 1 oder 2, die eine oder mehrere Verbindungen enthält, ausgewählt aus der Gruppe bestehend aus Verbindungen, die durch eine der folgenden allgemeinen Formeln (6-1) bis (6-12) und (7-1) bis (7-12) dargestellt sind: 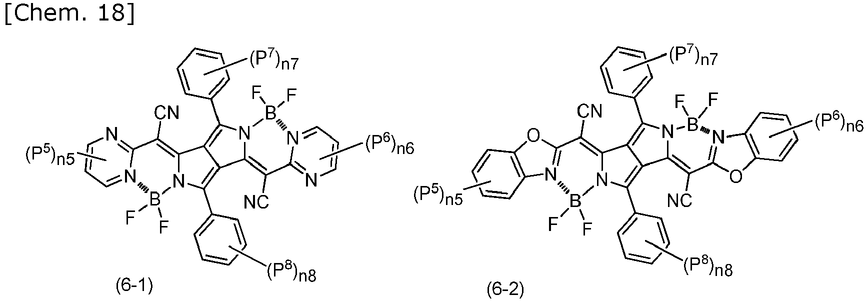 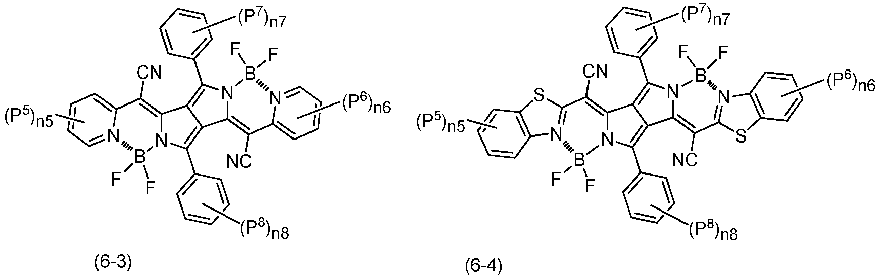 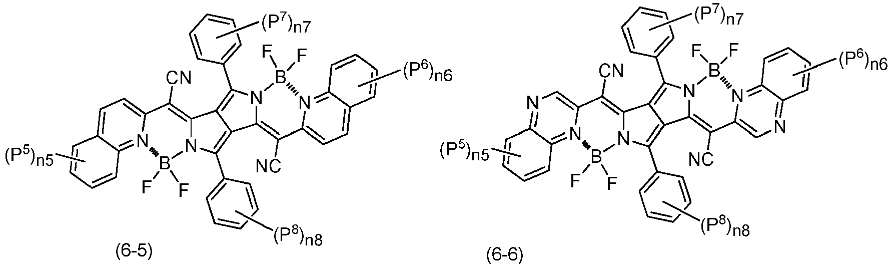 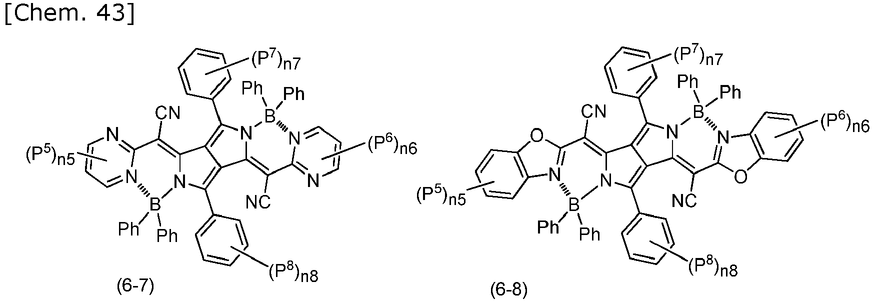 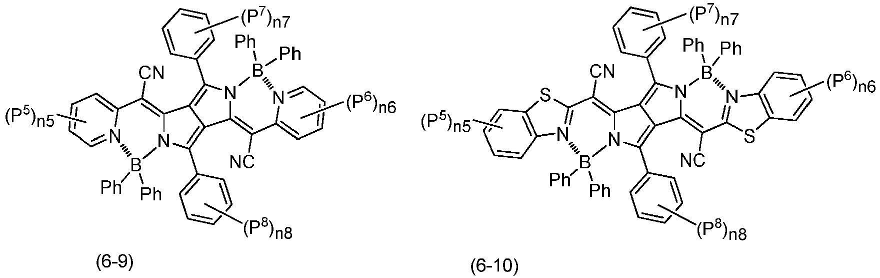 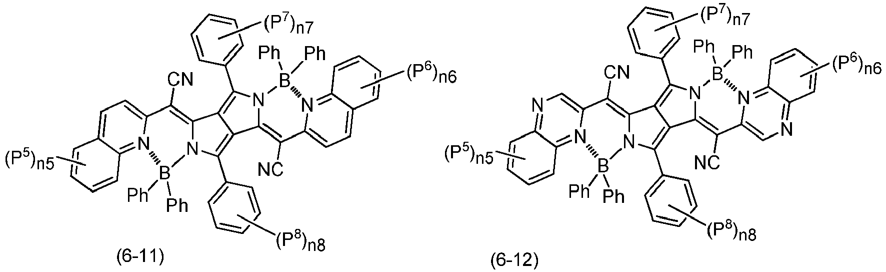 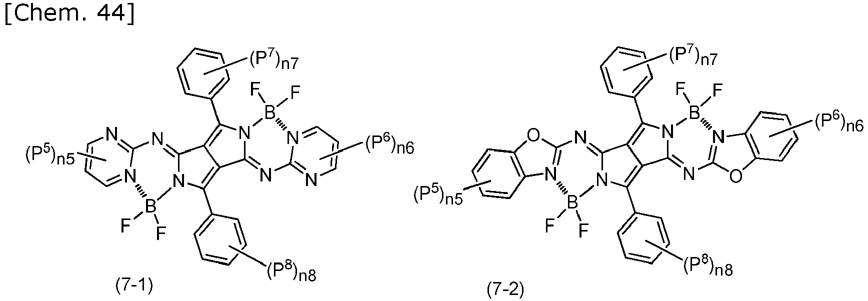 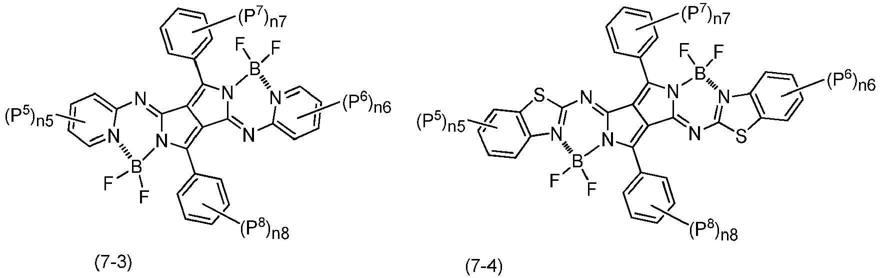 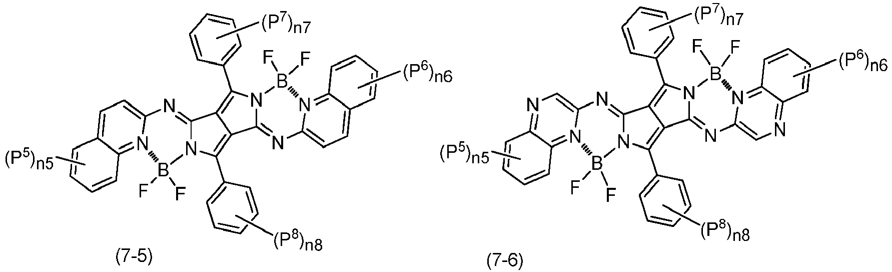 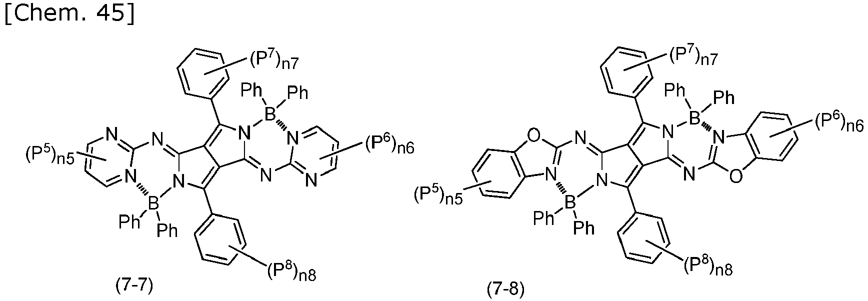 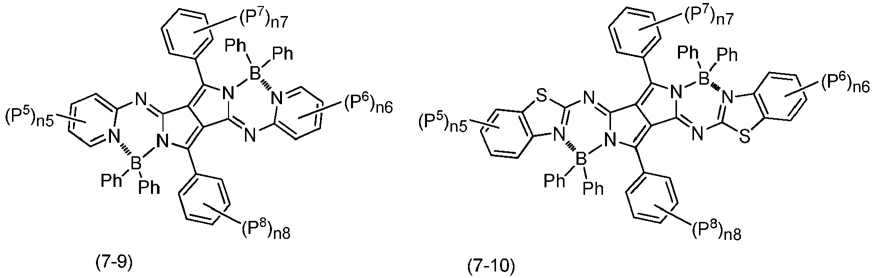 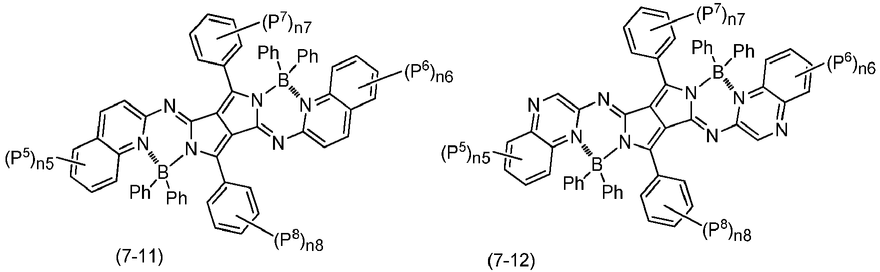
wobei P⁵ bis P⁸ jeweils unabhängig ein Halogenatom, eine C₁₋₂₀-Alkylgruppe, eine C₁₋₂₀-Alkoxygruppe, eine Aminogruppe, eine Monoalkylaminogruppe oder eine Dialkylaminogruppe darstellt;
n5 bis n8 jeweils unabhängig eine ganze Zahl von 0 bis 3 darstellt.

4. Harzzusammensetzung nach einem der Ansprüche 1 bis 3,
wobei das Harz ein thermoplastisches Harz ist.

5. Harzzusammensetzung nach einem der Ansprüche 1 bis 4,
wobei das im nahen Infrarot fluoreszierende Material und das Harz schmelzgeknetet sind.

6. Harzzusammensetzung nach einem der Ansprüche 1 bis 5,
wobei die maximale Fluoreszenzwellenlänge 700 nm oder mehr beträgt.

7. Harzzusammensetzung nach einem der Ansprüche 1 bis 6, die als medizinisches Material verwendet wird.

8. Formkörper, erhalten durch Verarbeitung der Harzzusammensetzung nach einem der Ansprüche 1 bis 7.

9. Formkörper nach Anspruch 8,
wobei zumindest ein Teil des Formkörpers ein medizinisches Werkzeug ist, das im Körper eines Patienten verwendet wird.

## Revendications

1. Composition de résine contenant :
un matériau fluorescent dans le proche infrarouge ; et
une résine,
dans laquelle le matériau fluorescent dans le proche infrarouge est un ou plusieurs composés choisis dans l'ensemble constitué par les composés représentés par les formules générales (I₃-1), (I₃-2), (I₃-3), (I₃-4), (I₃-5), (I₃-6), (I₄-1), (I₄-2), (I₄-3), (I₄-4), (I₄-5) et (I₄-6),
la teneur de la composition de résine en le matériau fluorescent dans le proche infrarouge est située dans la plage allant de 0,0001 % à 0,5 % en masse, et
la composition de résine a une longueur d'onde de fluorescence maximale de 650 nm ou plus : 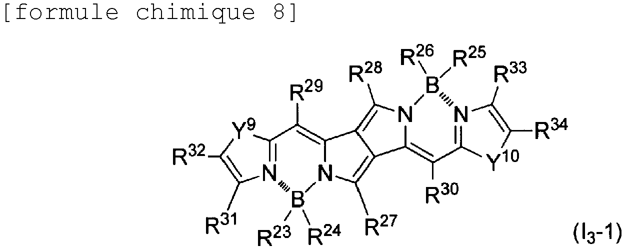
dans laquelle chacun de R²³, R²⁴, R²⁵ et R²⁶ représente indépendamment un atome d'halogène, un groupe alkyle en C₁ à C₂₀, un groupe alcoxy en C₁ à C₂₀, un groupe aryle, ou un groupe hétéroaryle ;
chacun de R²⁷ et R²⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ;
chacun de R²⁹ et R³⁰ représente indépendamment un atome d'hydrogène ou un groupe extracteur d'électrons choisis parmi un groupe halogénométhyle, un groupe nitro, un groupe cyano, un groupe aryle, un groupe hétéroaryle, un groupe alcynyle, un groupe alcényle, un substituant ayant un groupe carbonyle, un groupe sulfoxyde, un groupe sulfonyle, un groupe alcoxyméthyle, et un groupe aminométhyle ;
chacun de Y⁹ et Y¹⁰ représente indépendamment un atome de soufre, un atome d'oxygène, un atome d'azote, ou un atome de phosphore ;
(p4) chacun de R³¹ et R³² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(p5) R³¹ et R³² forment ensemble un cycle aromatique à 5 chaînons qui peut porter un substituant ou un cycle aromatique à 6 chaînons qui peut porter un substituant ; et
(q4) chacun de R³³ et R³⁴ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(q5) R³³ et R³⁴ forment ensemble un cycle aromatique à 5 chaînons qui peut porter un substituant ou un cycle aromatique à 6 chaînons qui peut porter un substituant ; 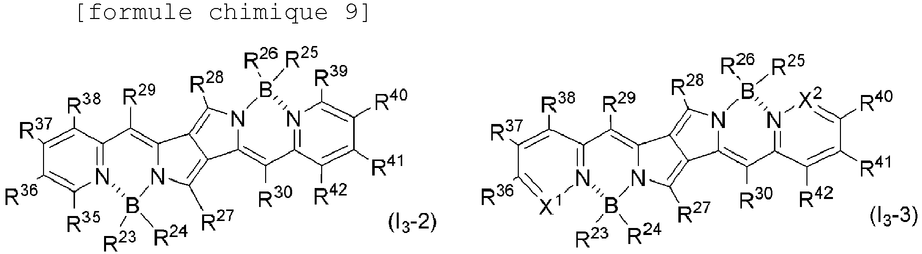 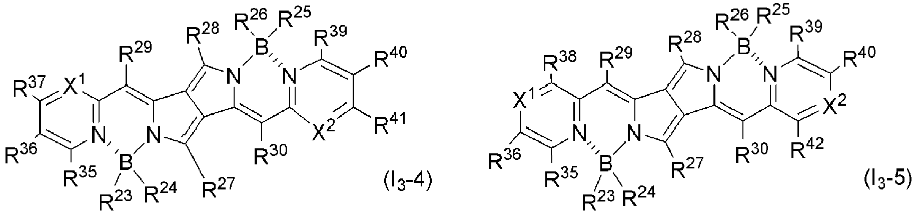 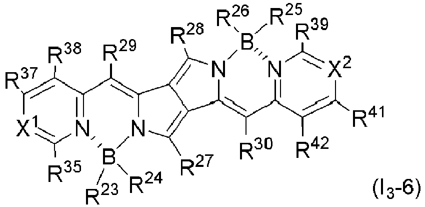
dans laquelle chacun de R²³ à R³⁰ est comme dans la formule (I₃-1) ;
chacun de X¹ et X² représente indépendamment un atome d'azote ou un atome de phosphore ;
(p6) chacun de R³⁵, R³⁶, R³⁷ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(p7) R³⁵ et R³⁶ forment ensemble un cycle aromatique à 5 chaînons qui peut porter un substituant ou un cycle aromatique à 6 chaînons qui peut porter un substituant, et chacun de R³⁷ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(p8) R³⁶ et R³⁷ forment ensemble un cycle aromatique à 5 chaînons qui peut porter un substituant ou un cycle aromatique à 6 chaînons qui peut porter un substituant, et chacun de R³⁵ et R³⁸ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(p9) R³⁷ et R³⁸ forment ensemble un cycle aromatique à 5 chaînons qui peut porter un substituant ou un cycle aromatique à 6 chaînons qui peut porter un substituant, et chacun de R³⁵ et R³⁶ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ; et
(q6) chacun de R³⁹, R⁴⁰, R⁴¹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(q7) R³⁹ et R⁴⁰ forment ensemble un cycle aromatique à 5 chaînons qui peut porter un substituant ou un cycle aromatique à 6 chaînons qui peut porter un substituant, et chacun de R⁴¹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle,
(q8) R⁴⁰ et R⁴¹ forment ensemble un cycle aromatique à 5 chaînons qui peut porter un substituant ou un cycle aromatique à 6 chaînons qui peut porter un substituant, et chacun de R³⁹ et R⁴² représente indépendamment un atome d'hydrogène, un atome d'halogène, un groupe alkyle en C₁ à C₂₀, un groupe alcoxy en C₁ à C₂₀, un groupe aryle, ou un groupe hétéroaryle, ou
(q9) R⁴¹ et R⁴² forment ensemble un cycle aromatique à 5 chaînons qui peut porter un substituant ou un cycle aromatique à 6 chaînons qui peut porter un substituant, et chacun de R³⁹ et R⁴⁰ représente indépendamment un atome d'hydrogène, un atome d'halogène, un groupe alkyle en C₁ à C₂₀, un groupe alcoxy en C₁ à C₂₀, un groupe aryle, ou un groupe hétéroaryle ; 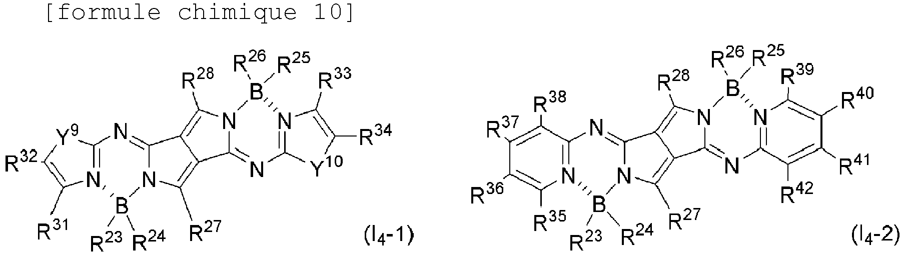 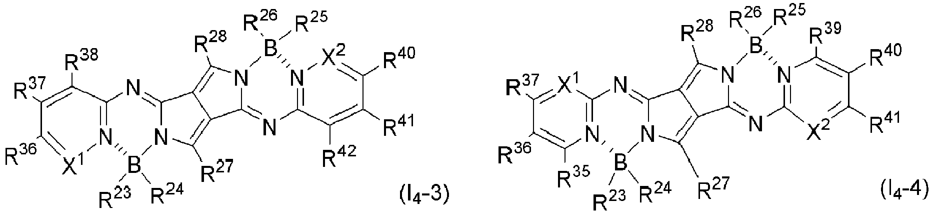 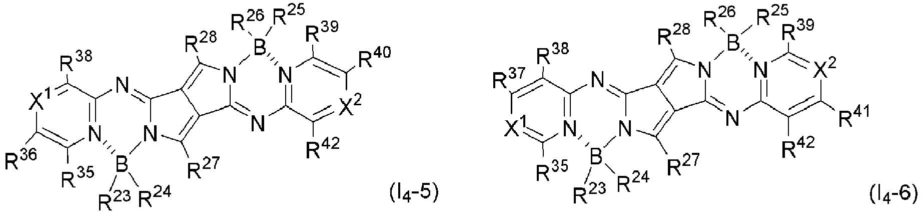
dans laquelle, dans les formules (I₄-1) à (I₄-6),chacun de R²³ à R²⁸ est comme dans la formule (I₃-1) et, dans la formule (I₄-1), chacun de R³¹ à R³⁴, Y⁹ et Y¹⁰ est comme dans la formule (I₃-1), dans les formules (I₄-2) à (I₄-6),chacun de R³⁵ à R⁴² est comme dans la formule (I₃-2) et, dans les formules (I₄-3) à (I₄-6), chacun de X¹ et X² est comme dans la formule (I₃-3).

2. Composition de résine selon la revendication 1, contenant un ou plusieurs composés choisis dans l'ensemble constitué par les composés représentés par n'importe lesquelles des formules générales (I₃-7) à (I₃-9) et (I₄-7) à (I₄-9) qui suivent : 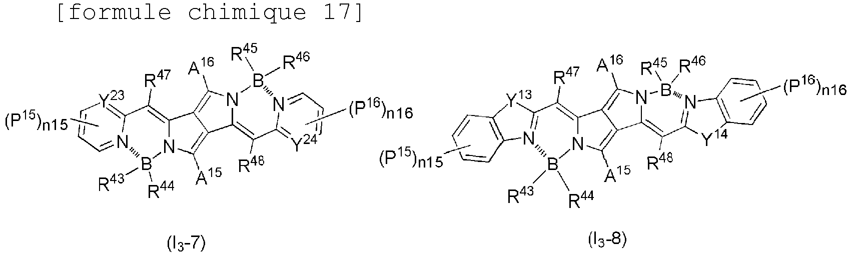 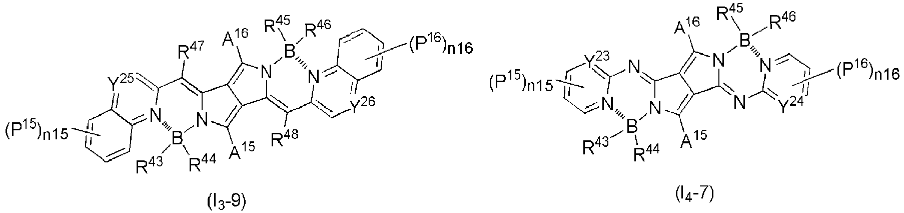 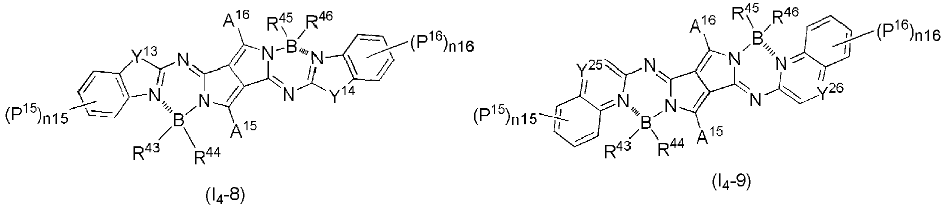
dans laquelle chacun de Y²³ et Y²⁴ représente indépendamment un atome de carbone ou un atome d'azote ;
chacun de Y¹³ et Y¹⁴ représente indépendamment un atome d'oxygène ou un atome de soufre ;
chacun de Y²⁵ et Y²⁶ représente indépendamment un atome de carbone ou un atome d'azote ;
chacun de R⁴⁷ et R⁴⁸ représente indépendamment un atome d'hydrogène ou un groupe extracteur d'électrons choisi parmi un groupe halogénométhyle, un groupe nitro, un groupe cyano, un groupe aryle, un groupe hétéroaryle, un groupe alcynyle, un groupe alcényle, un substituant ayant un groupe carbonyle, un groupe sulfoxyde, un groupe sulfonyle, un groupe alcoxyméthyle, et un groupe aminométhyle ;
chacun de R⁴³, R⁴⁴, R⁴⁵ et R⁴⁶ représente un atome d'halogène ou un groupe aryle qui peut porter un substituant ;
chacun de P¹⁵ et P¹⁶ représente indépendamment un atome d'halogène, un groupe alkyle en C₁ à C₂₀, un groupe alcoxy en C₁ à C₂₀, un groupe amino, un groupe monoalkylamino, ou un groupe dialkylamino ;
chacun de n15 et n16 représente indépendamment un entier de 0 à 3 ; et
chacun de A¹⁵ et A¹⁶ représente indépendamment un groupe phényle qui peut porter un à trois substituants indépendamment choisis dans l'ensemble constitué par un atome d'halogène, un groupe alkyle en C₁ à C₂₀, un groupe alcoxy en C₁ à C₂₀, un groupe amino, un groupe monoalkylamino, ou un groupe dialkylamino.

3. Composition de résine selon la revendication 1 ou 2, contenant un ou plusieurs composés choisis dans l'ensemble constitué par les composés représentés par n'importe lesquelles des formules générales (6-1) à (6-12) et (7-1) à (7-12) qui suivent : 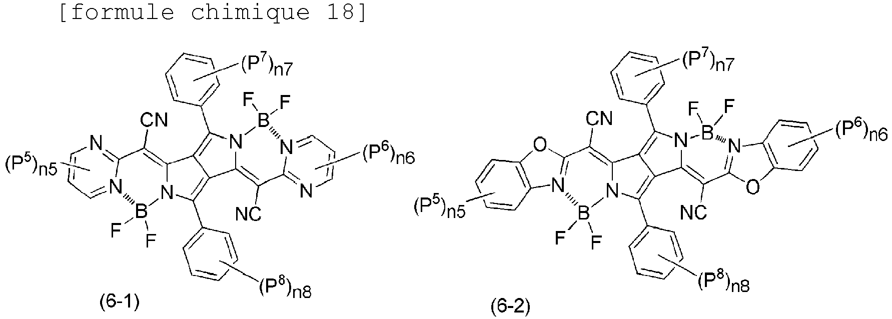 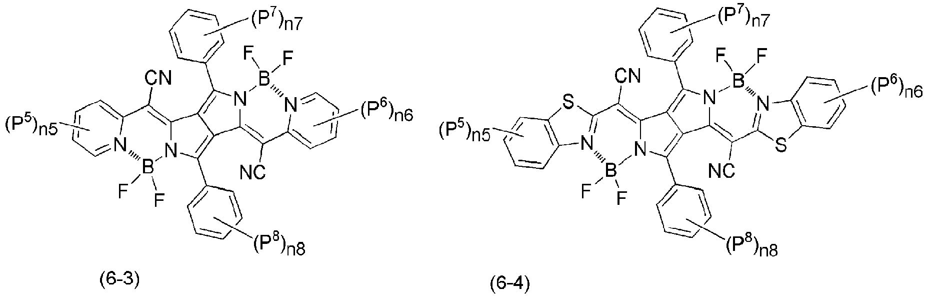 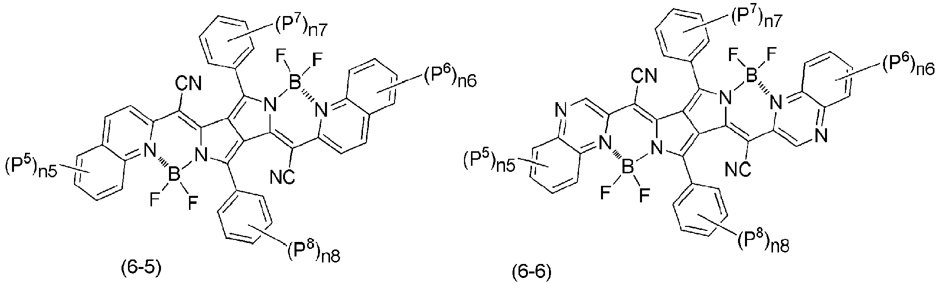 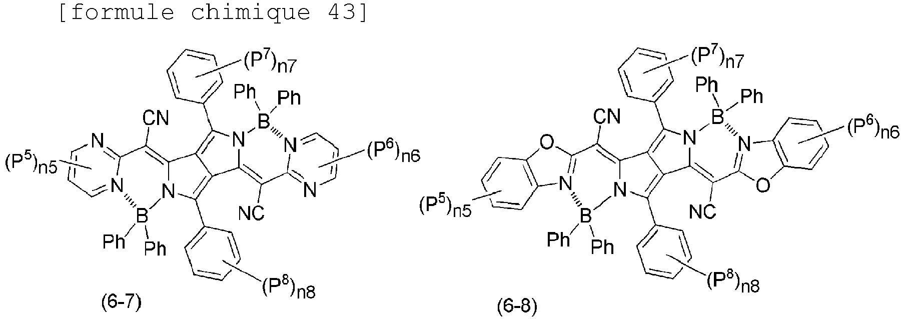 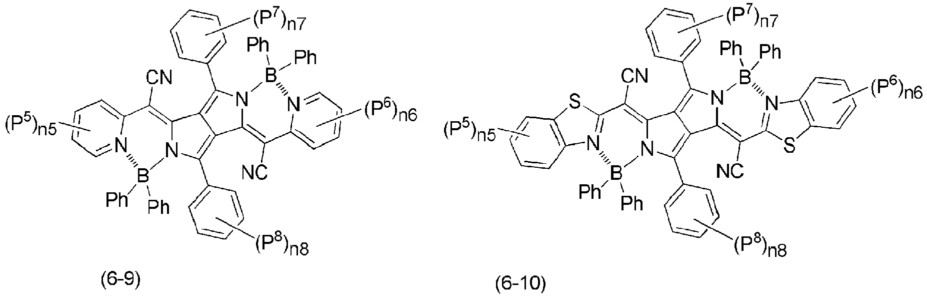 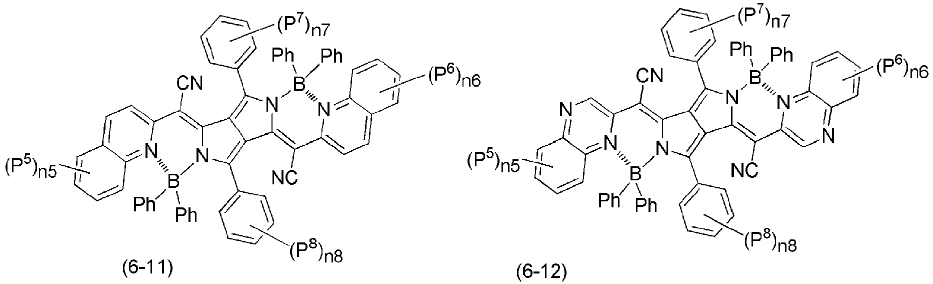 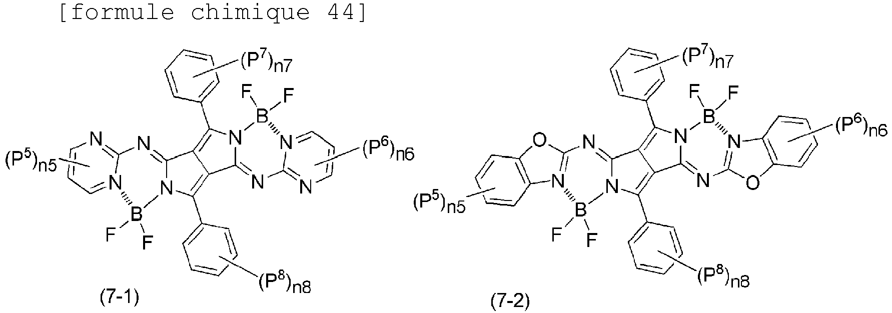 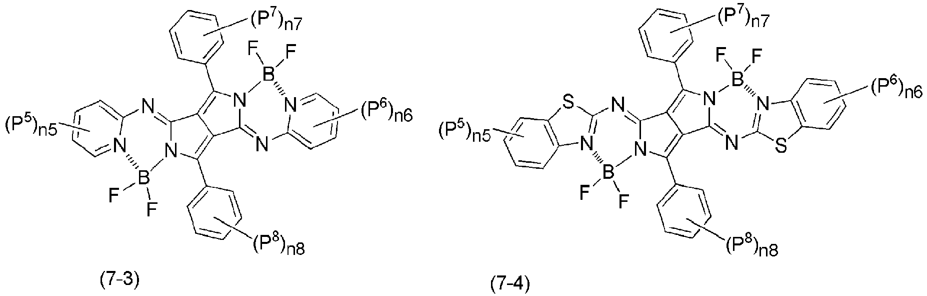 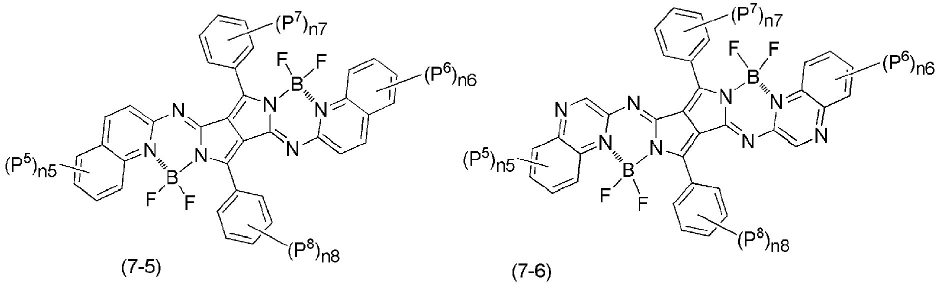 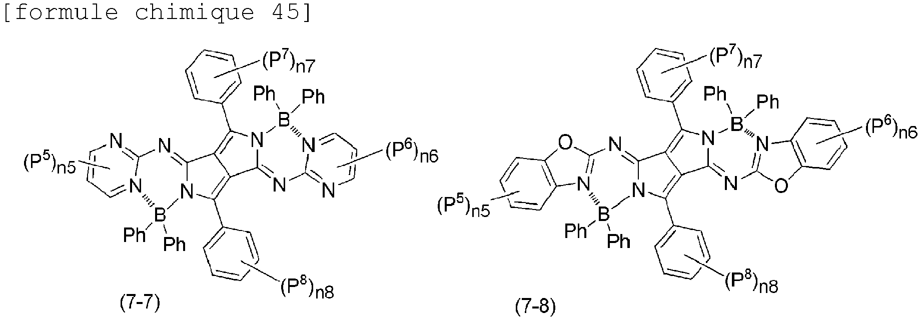 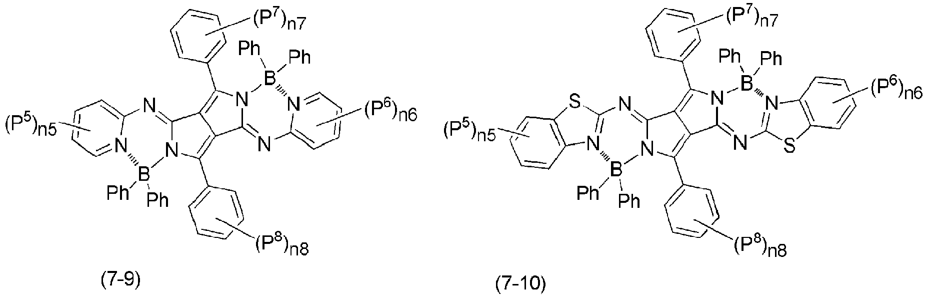 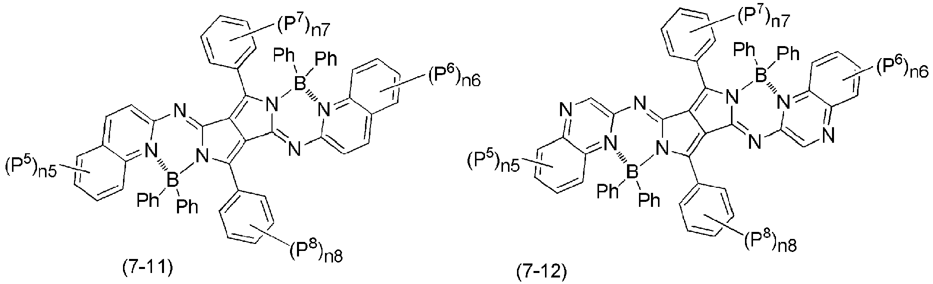
dans laquelle chacun de P⁵ à P⁸ représente indépendamment un atome d'halogène, un groupe alkyle en C₁ à C₂₀, un groupe alcoxy en C₁ à C₂₀, un groupe amino, un groupe monoalkylamino, ou un groupe dialkylamino ;
chacun de n5 à n8 représente indépendamment un entier de 0 à 3.

4. Composition de résine selon l'une quelconque des revendications 1 à 3, dans laquelle la résine est une résine thermoplastique.

5. Composition de résine selon l'une quelconque des revendications 1 à 4, dans laquelle le matériau fluorescent dans le proche infrarouge et la résine sont malaxés à l'état fondu.

6. Composition de résine selon l'une quelconque des revendications 1 à 5, dans laquelle la longueur d'onde de fluorescence maximale est de 700 nm ou plus.

7. Composition de résine selon l'une quelconque des revendications 1 à 6, qui est utilisée en tant que matériel médical.

8. Article moulé obtenu par traitement de la composition de résine selon l'une quelconque des revendications 1 à 7.

9. Article moulé selon la revendication 8, dans lequel au moins une partie de l'article moulé est un outil médical qui est utilisé dans le corps d'un patient.
